# EUROPEAN PATENT SPECIFICATION

(11) **EP 2 007 735 B1**
(45) Date of publication and mention of the grant of the patent: **27.10.2010**
(21) Application number: 07728235.8
(22) Date of filing: 18.04.2007
(51) Int. Cl.: C07D 237/32, C07D 403/06, C07D 403/04, C07D 403/14, A61K 31/502, A61P 29/00, A61P 37/08

(54) **2-SUBSTITUTED 4-BENZYLPHTHALAZINONE DERIVATIVES AS HISTAMINE H1 AND H3 ANTAGONISTS**
2-SUBSTITUIERTE 4-BENZYLPHTHALAZINONDERIVATE ALS HISTAMIN-H1- UND H3-ANTAGONISTEN
DERIVES DE 4-BENZYLPHTALAZINONE SUBSTITUES EN 2 EN TANT QU'ANTAGONISTES DES HISTAMINES H1 ET H3

(30) Priority: 20.04.2006 GB 0607839; 29.03.2007 GB 0706160; 29.03.2007 GB 0706176
(43) Date of publication of application: 31.12.2008
(73) Proprietor: Glaxo Group Limited, Greenford Middlesex UB6 0NN (GB)
(72) Inventor: GORE, Paul, Martin, Stevenage Hertfordshire SG1 2NY (GB); HANCOCK, Ashley, Paul, Stevenage Hertfordshire SG1 2NY (GB); HODGSON, Simon, Teanby, Stevenage Hertfordshire SG1 2NY (GB); KINDON, Leanda, Jane, Stevenage Hertfordshire SG1 2NY (GB); PROCOPIOU, Panayiotis, Alexandrou, Stevenage Hertfordshire SG1 2NY (GB)
(74) Representative: Priestley, Ruth Elizabeth
(86) International application number: PCT/EP2007/053773
(87) International publication number: WO 2007/122156

(56) References cited:
- EP-A2- 0 174 464
- WO-A-2004/035556
- DE-A1- 2 164 058

## Description

The present invention relates to compounds, processes for their preparation, pharmaceutical compositions containing them and to their use in the treatment of various diseases, in particular inflammatory and/or allergic diseases of the respiratory tract.

Allergic rhinitis, pulmonary inflammation and congestion are medical conditions that are often associated with other conditions such as asthma, chronic obstructive pulmonary disease (COPD), seasonal allergic rhinitis and perennial allergic rhinitis. In general these conditions are mediated, at least in part, by inflammation associated with the release of histamine from various cells, in particular mast cells.

Allergic rhinitis, also known as 'hay fever', affects a large proportion of the population worldwide. There are two types of allergic rhinitis; seasonal and perennial. The clinical symptoms of seasonal allergic rhinitis typically include nasal itching and irritation, sneezing and watery rhinorrhea which is often accompanied by nasal congestion. The clinical symptoms of perennial allergic rhinitis are similar except that nasal blockage may be more pronounced. Either type of allergic rhinitis may also cause other symptoms such as itching of the throat and/or eyes, epiphora and oedema around the eyes. The symptoms of allergic rhinitis may vary in intensity from the nuisance level to debilitating.

Allergic rhinitis and other allergic conditions are associated with the release of histamine from various cell types, but particularly mast cells. The physiological effects of histamine are classically mediated by three receptor subtypes, termed H1, H2 and H3. H1 receptors are widely distributed throughout the CNS and periphery, and are involved in wakefulness and acute inflammation. H2 receptors mediate gastric acid secretion in response to histamine. H3 receptors are present on the nerve endings in both the CNS and periphery and mediate inhibition of neurotransmitter release [Hill et al., Pharmacol. Rev., 49:253-278, (1997)]. Recently, a fourth member of the histamine receptor family has been identified, termed the H4 receptor [Hough, Mol. Pharmacol., 59:415-419, (2001)]. Whilst the distribution of the H4 receptor appears to be restricted to cells of the immune and inflammatory systems, a physiological role for this receptor remains to be clarified.

The activation of H1 receptors in blood vessels and nerve endings is responsible for many of the symptoms of allergic rhinitis, which include itching, sneezing, and the production of watery rhinorrhea. Oral antihistamine compounds (such as chlorphenyramine, cetirizine, desloratidine and fexofenadine) and intranasal antihistamines (such as azelastine and levocabastine) which are selective H1 receptor antagonists are effective in treating the itching, sneezing and rhinorrhea associated with allergic rhinitis, but are not effective against the nasal congestion symptoms [Aaronson, Ann. Allergy, 67:541-547, (1991)]. Thus H1 receptor antagonists have been administered in combination with sympathomimetic agents such as pseudoephedrine or oxymetazoline to treat the nasal congestion symptoms of allergic rhinitis. These drugs are thought to produce a decongestant action by activating α-adrenergic receptors and increasing the vascular tone of blood vessels in the nasal mucosa. The use of sympathomimetic drugs for the treatment of nasal congestion is frequently limited by the CNS stimulant properties and their effects on blood pressure and heart rate. A treatment which decreases nasal congestion without having effects on the CNS and cardiovascular system may therefore offer advantages over existing therapies.

Histamine H3 receptors are expressed widely on both CNS and peripheral nerve endings and mediate the inhibition of neurotransmitter release. *In vitro* electrical stimulation of peripheral sympathetic nerves in isolated human saphenous vein results in an increase in noradrenaline release and smooth muscle contraction, which can be inhibited by histamine H3 receptor agonists [Molderings et al., Naunyn-Schmiedeberg's Arch. Pharmacol., 346:46-50, (1992); Valentine et al., Eur. J. Pharmacol., 366:73-78, (1999)]. H3 receptor agonists also inhibit the effect of sympathetic nerve activation on vascular tone in porcine nasal mucosa [Varty & Hey., Eur. J. Pharmacol., 452:339-345, (2002)]. *In vivo*, H3 receptor agonists inhibit the decrease in nasal airway resistance produced by sympathetic nerve activation [Hey et al., Arzneim-Forsch Drug Res., 48:881-888, (1998)]. Activation of histamine H3 receptors in human nasal mucosa inhibits sympathetic vasoconstriction [Varty et al., Eur. J. Pharmacol., 484:83-89, (2004)]. Furthermore, H3 receptor antagonists, in combination with histamine H1 receptor antagonists, have been shown to reverse the effects of mast cell activation on nasal airway resistance and nasal cavity volume, an index of nasal congestion [Mcleod et al., Am. J. Rhinol., 13:391-399, (1999)], and further evidence for the contribution of H3 receptors to histamine-induced nasal blockage is provided by histamine nasal challenge studies performed on normal human subjects [Taylor-Clark et a/., Br. J. Pharmacol., 144, 867-874, (2005)], although the H3 mechanism in this regard would appear to be novel and unprecedented and may ultimately prove to be clinically silent.

WO2004/035556 discloses substituted piperazines, (1,4) diazepines and 2,5-diazabicyclo [2.2.1] heptanes as histamine H3 or histamine H1/H3 dual antagonists or reverse agonists.

A novel class of compounds has been found that are dual histamine H1 and H3 receptor antagonists. By 'dual' histamine H1 and H3 receptor antagonists it is meant that compounds have activity at both receptor subtypes. For example, the activity at the H1 receptor may be within approximately 100 fold of the activity at the H3 receptor, such as within approximately 10 fold or less.

Thus the present invention provides a compound of formula (I) 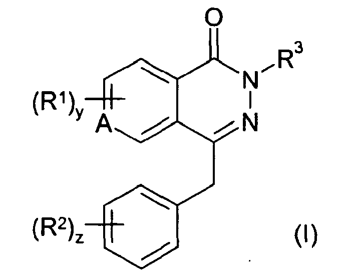 wherein
A represents N or CH;
R¹ and R² each independently represent halogen, C₁₋₆alkyl, C₁₋₆alkoxy, hydroxyl or trifluoromethyl;
y and z each independently represent 0, 1 or 2;
R³ represents the group -(CH₂)ₐN⁴R⁵ or a group of formula (i) 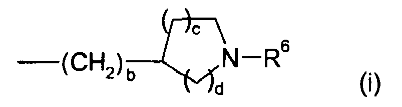
in which
a represents 1, 2 or 3;
b represents 0 or 1;
c represents 0, 1 or 2 and d represents 0,1, 2 or 3, such that c and d cannot both be 0;
R⁴ represents hydrogen or C₁₋₆alkyl;
R⁵ and R⁶ each independently represent a group selected from the formulae (a), (b) or (c) 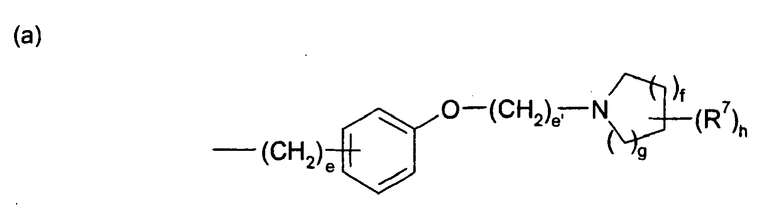 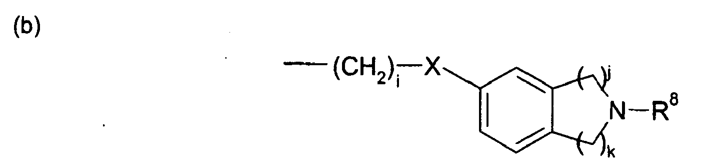 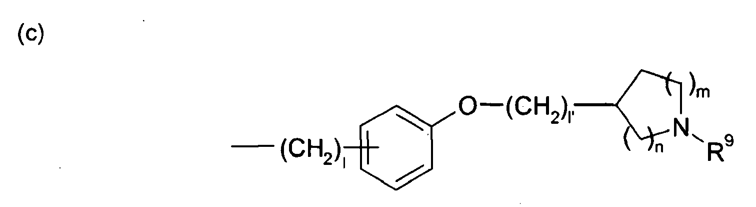
in which, for formula (a)
e represents 1 to 6;
e' represents 2 to 4;
f represents 0, 1 or 2 and g represents 0, 1, 2 or 3, such that f and g cannot both be 0;
h represents 0, 1 or 2;
R⁷ represents C₁₋₃alkyl;
in which, for formula (b)
i represents 1 to 6;
X represents either a bond, O or -N(R¹⁰)C(O)-, in which R¹⁰ represents hydrogen or C₁₋₆alkyl;
j and k each represent 1 or each represent 2;
R⁸ represents hydrogen, C₃₋₆cycloalkyl or C₁₋₆alkyl;
in which, for formula (c)
I represents 1 to 6;
I' represents 0 to 3;
m represents 0, 1 or 2 and n represents 0, 1, 2 or 3, such that m and n cannot both be 0,
and such that I' plus n must represent 1, 2 or 3;
R⁹ represents hydrogen, C₃₋₆cycloalkyl or C₁₋₆alkyl;
or a salt thereof.

The compounds of formula (I) may be expected to be useful in the treatment of various diseases in particular inflammatory and/or allergic diseases, such as inflammatory and/or allergic diseases of the respiratory tract, for example allergic rhinitis, that are associated with the release of histamine from cells such as mast cells. Further, the compounds of formula (I) may show an improved profile in that they may possess one or more of the following properties:
(i) H3 antagonist activity with a pKi of greater than about 7, for example greater than about 8;
(ii) H1 receptor antagonist activity with a pKi of greater than 7, for example greater than about 8;
(iii) lower mucocilliary clearance/prolonged duration of action;
(iv) lower CNS penetration.

Compounds having such a profile may be suitable for intranasal delivery, and/or capable of once daily administration and/or further may have an improved side effect profile compared with other existing therapies.

In one embodiment, there is provided a compound of formula (I) in which A represents CH, thus there is provided a compound of formula (I): 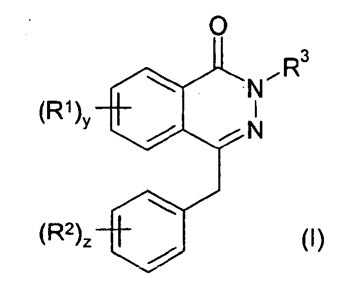 wherein the substituents R¹, R², R³, y and z are as defined above.

In another embodiment, there is provided a compound of formula (I) in which A represents N.

In a further embodiment, there is provided a compound of formula (I) wherein
A represents CH and R¹ and R² each independently represent halogen e.g. fluorine or chlorine, C₁-₃alkyl e.g. methyl, C₁₋₃alkoxy e.g. methoxy, hydroxyl or trifluoromethyl;
y and z each independently represent 0, 1 or 2, e.g. 0 or 1;
R³ represents the group -(CH₂)ₐNR⁴R⁵ or a group of formula (i) 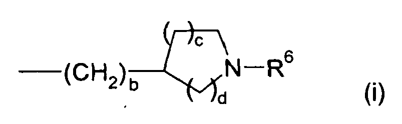 in which
a represents 1, 2 or 3, e.g. 2;
b represents 0 or 1, e.g. 0;
c represents 0, 1 or 2 and d represents 0, 1, 2 or 3, such that c and d cannot both be 0, e.g. c and d both independently represent 1 or 2;
R⁴ represents hydrogen or C₁₋₃alkyl, e.g. methyl;
R⁵ and R⁶ each independently represent a group selected from the formulae (a), (b) or (c) 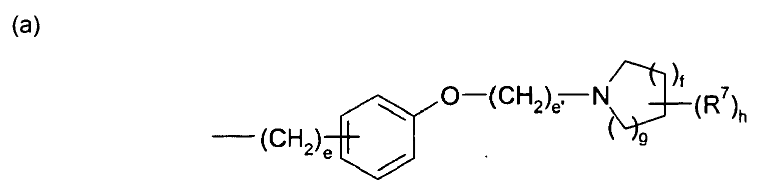 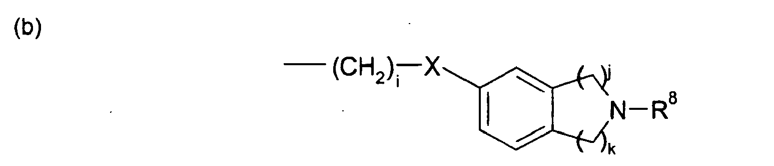 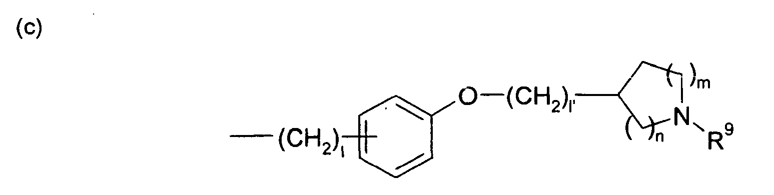
in which, for formula (a)
e represents 1 to 6, such as 1 to 5, e.g. 3 or 4;
e' represents 2 to 4, e.g. 3;
f and g each independently represent 1 or 2;
h represents 0 or 1, e.g. 0;
R⁷ represents C₁₋₃alkyl, e.g. methyl or isopropyl;
in which, for formula (b)
i represents 1 to 6, such as 1 to 3, e.g. 3;
X represents either a bond, O or -N(R¹⁰)C(O)-, in which R¹⁰ represents either hydrogen or C₁₋₃alkyl such as methyl, e.g. hydrogen;
j and k each represent 1 or each represent 2, e.g. each represent 2;
R⁸ represents hydrogen, C₃₋₅cycloalkyl, e.g. cyclobutyl or C₁₋₃alkyl, e.g. isopropyl;
in which, for formula (c)
I represents 1 to 5, e.g. 2 or 4
I' represents 0 to 2, e.g. 0;
m and n each independently represent 1 or 2, such that m and n cannot both be 0, e.g. 1 and 2 respectively, such that I' plus n must represent 1, 2 or 3;
R⁹ represents hydrogen, C₃₋₅cycloalkyl, e.g. cyclobutyl or C₁₋₃alkyl, e.g. isopropyl. Alternatively, in this further embodiment, A may represent N.

In another embodiment, R³ represents a group of formula (i).

In another embodiment, A represents CH and R³ represents a group of formula (i).

In another embodiment, R³ represents a group of formula (i) and R⁶ represents a group of formula (a).

In another embodiment, A represents CH, R³ represents a group of formula (i) and R⁶ represents a group of formula (a).

In another embodiment, R³ represents the group -(CH₂)ₐNR⁴R⁵ and R⁵ represents a group selected from the formulae (a), (b) or (c), especially a group of formula (b).

In another embodiment, A represents CH, R³ represents the group -(CH₂)ₐNR⁴R⁵ and R⁵ represents a group selected from the formulae (a), (b) or (c), especially a group of formula (b).

In another embodiment, y represents 0.

In another embodiment, A represents CH and y represents 0.

In another embodiment, z represents 1.

In another embodiment, A represents CH and z represents 1.

In another embodiment, z represents 1, and R² is substituted in the 4-position, i.e. para.

In another embodiment, A represents CH, z represents 1, and R² is substituted in the 4-position, i.e. para.

In another embodiment, R² represents chlorine, fluorine, C₁₋₃alkoxy e.g. methoxy, or hydroxyl.

In another embodiment, A represents CH and R² represents chlorine, fluorine, C₁₋₃alkoxy e.g. methoxy, or hydroxyl.

In another embodiment, R⁵ and R⁶ represent a group of formula (a) or (c) in which the substitution pattern on the phenyl ring is para.

In another embodiment, A represents CH and R⁵ and R⁶ represent a group of formula (a) or (c) in which the substitution pattern on the phenyl ring is para.

In another embodiment, R⁵ and R⁶ represent a group of formula (a) in which f and g both represent 2 and h represents 0.

In another embodiment, R⁵ and R⁶ represent a group of formula (b) in which R⁸ represents C₃₋₅cycloalkyl, particularly cyclobutyl.

In another embodiment, R⁵ and R⁶ represent a group of formula (c) in which R⁹ represents C₃₋₅cycloalkyl, particularly cyclobutyl.

In another embodiment, R⁵ and R⁶ represent a group of formula (b) in which j and k each represent 2.

In another embodiment,
b represents 0 or 1;
c represents 0, 1 or 2 and d represents 0, 1, 2 or 3, such that c and d cannot both be 0, with the proviso that b and d both cannot be 0;
i represents 1 to 6; and
X represents either a bond, O or -N(R¹⁰)C(O)-, in which R¹⁰ represents hydrogen or C₁₋₆alkyl, with the proviso that when X represents O, i represents 2 to 6.

When A represents CH, representative examples of R⁵ and R⁶ include:
4-(4-{[3-(hexahydro-1*H*-azepin-1-yl)propyl]oxy}phenyl)butyl,
2-(4-{[3-(hexahydro-1*H*-azepin-1-yl)propyl]oxy}phenyl)ethyl,
4-(4-{[3-(hexahydro-1*H-*azepin-1-yl)propyl]oxy}phenyl)butyl,
5-(4-{[3-(hexahydro-1*H*-azepin-1-yl)propyl]oxy}phenyl)pentyl,
(4-{[3-(hexahydro-1*H*-azepin-1-yl)propyl]oxy}phenyl)methyl,
2-(4-{[3-(hexahydro-1*H*-azepin-1-yl)propyl]oxy}phenyl)ethyl,
3-(4-{[3-(hexahydro-1*H*-azepin-1-yl)propyl]oxy}phenyl)propyl,
3-[(3-cyclobutyl-2,3,4,5-tetrahydro-1*H*-3-benzazepin-7-yl)oxy]propyl,
(3-cyclobutyl-2,3,4,5-tetrahydro-1*H-*3-benzazepin-7-yl)methyl,
*N*-(2-ethyl)-3-cyclobutyl-2,3,4,5-tetrahydro-1*H-*3-benzazepine-7-carboxamide,
*N*-(3-propyl)-3-cyclobutyl-2,3,4,5-tetrahydro-1*H*-3-benzazepine-7-carboxamide,
*N*-(4-butyl)-3-cyclobutyl-2,3,4,5-tetrahydro-1*H-*3-benzazepine-7-carboxamide,
2-{4-[(1-cyclobutyl-4-piperidinyl)oxy]phenyl}ethyl or
4-{4-[(1-cydobutyl-4-piperidinyl)oxy]phenyl}butyl.

in another embodiment, there is provided a compound of formula (I) as defined above with the proviso that the compound is not 4-[(4-chlorophenyl)methyl]-2-({1-[4-(4-{[3-(hexahydro-1*H*-azepin-1-yl)propyl]oxy}phenyl)butyl]-2-pyrrolidinyl}methyl)-1-(2*H*)-phthalazinone or a salt thereof, or an individual isomer thereof or mixtures thereof.

Representative compounds of formula (I) include the compounds of Examples 1 to 24, or salts thereof.

In another embodiment, there is provided 4-[(4-chlorophenyl)methyl]-2-({1-[4-(4-{[3-(hexahydro-1*H*-azepin-1-yl)propyl]oxy}phenyl)butyl]-2-pyrrolidinyl}methyl)-1 (2H)-phthalazinone (including its R and S isomers, and mixtures thereof) and salts thereof, particularly pharmaceutically acceptable salts thereof.

In another embodiment, there is provided 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone and salts thereof, particularly pharmaceutically acceptable salts thereof.

In another embodiment, there is provided 4-[(4-chlorophenyl)methyl]-2-({(2*S*)-1-[4-(4-{[3-(hexahydro-1*H*-azepin-1-yl)propyl]oxy}phenyl)butyl]-2-pyrrolidinyl}methyl)-1(2*H*)-phthalazinone and salts thereof, particularly pharmaceutically acceptable salts thereof.

In another embodiment, there is provided 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)pyrido[3,4-*d*] pyridazin-1(2*H*)-one and salts thereof, particularly pharmaceutically acceptable salts thereof.

It is to be understood that the invention includes all possible combinations of embodiments, groups, representative examples and substituents described herein.

C₁₋₆alkyl, whether alone or as part of another group, may be straight chain or branched and C₁₋₆alkoxy shall be interpreted similarly. Representative examples include methyl, ethyl, *n*-propyl, *iso*-propyl, *n*-butyl, *sec*-butyl, *iso*-butyl, *t*-butyl, *n*-pentyl, *neo*-pentyl and *n-*hexyl. Particular alkyl and alkoxy groups are C₁₋₃ alkyl and C₁-₃ alkoxy.

C₃₋₆cycloalkyl refers to a non-aromatic cyclic hydrocarbon ring having from three to six carbon atoms. Representative examples include cyclopropyl, cyclobutyl, cyclopentyl and cyclohexyl.

The term "halogen" is used herein to describe, unless otherwise stated, fluorine, chlorine, bromine or iodine.

It is to be understood that the references herein to compounds of formula (I) covers the compounds of formula (I) as the free base or as salts thereof, for example as a pharmaceutically acceptable salt thereof, or as a solvate.

The compounds of formula (I) may be in the form of and/or may be administered as a pharmaceutically acceptable salt. Pharmaceutically acceptable salts include acid and base addition salts. For a review on suitable salts see Berge et al., J. Pharm. Sci., 66:1-19, (1977).

Typically, a pharmaceutically acceptable salt may be readily prepared by using a desired acid as appropriate. The salt may precipitate from solution and be collected by filtration or may be recovered by evaporation of the solvent.

A pharmaceutically acceptable acid addition salt can be formed by reaction of a compound of formula (I) with a suitable inorganic or organic acid (such as hydrobromic, hydrochloric, formic, sulfuric, nitric, phosphoric, succinic, maleic, acetic, fumaric, citric, tartaric, benzoic, p-toluenesulfonic, methanesulfonic, naphthalene disulfonic acid, biphenyl sulfonic acid or naphthalenesulfonic acid), optionally in a suitable solvent such as an organic solvent, to give the salt which is usually isolated for example by crystallisation and filtration. Thus, a pharmaceutically acceptable acid addition salt of a compound of formula (I) can be for example a hydrobromide, hydrochloride, formate, sulfate, nitrate, phosphate, succinate, maleate, acetate, fumarate, citrate, tartrate, benzoate, p-toluenesulfonate, methanesulfonate, naphthalene disulfonate, biphenyl disulfonate or naphthalenesulfonate salt.

Other non-pharmaceutically acceptable salts, e.g. oxalates or trifluoroacetates, may be used, for example in the isolation of the compounds of formula (I), and are included within the scope of the present invention. Included within its scope are all possible stoichiometric and non-stoichiometric forms of the salts of the compounds of formula (I).

Particular salts include naphthalene disulfonate salts, such as a 2,6- or a 1,5- naphthalene disulfonate salt, e.g. a 1,5-naphthalene disulfonate salt.

In one embodiment there is provided a naphthalene disulfonate salt of 4-[(4-chlorophenyl)methyl]-2-({(2*R*)-1-[4-(4-{[3-(hexahydro-1*H*-azepin-1-yl)propyl]oxy}phenyl) butyl]-2-pyrrolidinyl}methyl)-1(2*H*)-phthalazinone.

In another embodiment there is provided a 1,5-naphthalene disulfonate salt of 4-[(4-chlorophenyl)methyl]-2-({(2*R*)-1-[4-(4-{[3-(hexahydro-1*H*-azepin-1-yl)propyl]oxy}phenyl) butyl]-2-pyrrolidinyl}methyl)-1(2*H*)-phthalazinone.

In another embodiment there is provided a 1,5-naphthalene disulfonate monohydrate salt of 4-[(4-chlorophenyl)methyl]-2-({(2*R*)-1-[4-(4-{[3-(hexahydro-1*H*-azepin-1-yl)propyl]oxy} phenyl)butyl]-2-pyrrolidinyl}methyl)-1(2*H*)-phthalazinone.

It will be appreciated that many organic compounds can form complexes with solvents in which they are reacted or from which they are precipitated or crystallized. These complexes are known as "solvates". For example, a complex with water is known as a "hydrate". Solvents with high boiling points and/or solvents with a high propensity to form hydrogen bonds such as water, xylene, *N*-methyl pyrrolidinone or methanol may be used to form solvates. Methods for identification of solvates include, but are not limited to, NMR and microanalysis. Solvates of the compounds of formula (I) are within the scope of the invention.

The compounds of formula (I) may be in crystalline or amorphous form. Furthermore, some of the crystalline forms of the compounds of formula (I) may exist as polymorphs, which are included within the scope of the present invention. The most thermodynamically stable polymorphic forms of the compounds of formula (I) are of particular interest.

Polymorphic forms of the compounds of formula (I) may be characterized and differentiated using a number of conventional analytical techniques, including, but not limited to, X-ray powder diffraction (XRPD) patterns, infrared (IR) spectra, Raman spectra, differential scanning calorimetry (DSC), thermogravimetric analysis (TGA) and solid state nuclear magnetic resonance (NMR).

It will be appreciated that the compounds of formula (I) may possess one or more asymmetric carbon atoms so that optical isomers e.g. enantiomers or diastereoisomers may be formed. The present invention encompasses all optical isomers of the compounds of formula (I) whether as individual isomers isolated such as to be substantially free of the other isomer (i.e. pure) or as mixtures thereof (e.g. racemates and racemic mixtures). An individual isomer isolated such as to be substantially free of the other isomer (i.e. pure) may be isolated such that less than about 10%, particularly less than about 1%, for example less than about 0.1 % of the other isomer is present.

Further, it will be appreciated that the R and S enantiomers may be isolated from the racemate by conventional resolution methods such as preparative HPLC involving a chiral stationary phase, by resolution using fractional crystallisation of a salt of the free base with a chiral acid, by chemical conversion to a diastereoisomer using a chiral auxiliary followed by chromatographic separation of the isomers and then removal of the chiral auxiliary and regeneration of the pure enantiomer, or by total asymmetric synthesis.

It will be appreciated from the foregoing that included within the scope of the invention are all solvates, hydrates, complexes, isomers and polymorphic forms of the compounds of formula (I) and salts thereof.

There is also provided processes for the preparation of compounds of formula (I) or salts thereof.

According to a first process, A, a compound of formula (I) may be prepared by reacting a compound of formula (IIa) or (IIb) 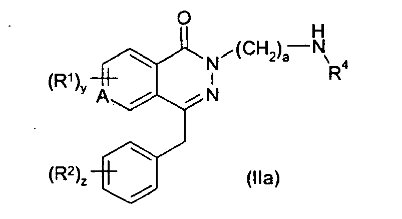 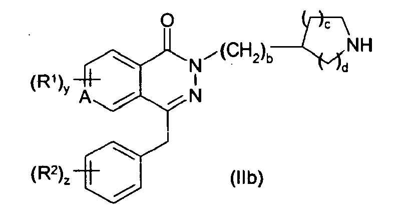 wherein A, R¹, y, R², z, a, b, c, d and R⁴ are as defined hereinabove for formula (I),
with a compound of formula (IIIa), formula (IIIb) or formula (IIIc) 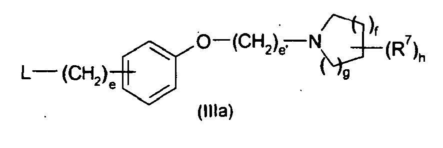 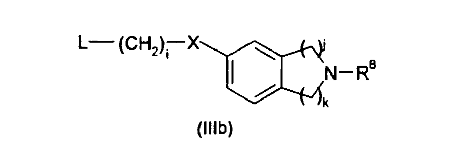 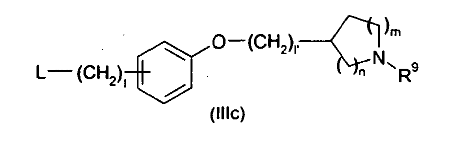 wherein e, e', f, g, h, R⁷, i, X, j, k, R⁸, I, I', m, n, and R⁹ are as defined hereinabove for formula (I) and L represents a leaving group such as chlorine, bromine, iodine, an activated hydroxyl such as mesylate or tosylate, or L represents an aldehyde such that L-(CH₂)_{e,i or I} is HC(O)- (CH₂)_{(e,i or I)-1}.

The alkylation reaction may typically be carried out in the presence of a suitable base such as triethylamine (NEt₃), diisopropylethylamine (DIPEA) or sodium hydrogen carbonate (NaHCO₃), in an appropriate solvent such as acetonitrile (MeCN) or *N,N-*dimethylformamide (DMF) optionally at an appropriate elevated temperature such as about 80 °C, optionally using microwave irradiation and optionally with the addition of an activating agent such as potassium iodide (KI) or sodium iodide (Nal).

When L represents an aldehyde, the reductive amination reaction may be carried out using a suitable reducing agent such as sodium triacetoxyborohydride (NaBH(OAc)₃), optionally in the presence of an appropriate acid catalyst such as acetic acid, in a solvent such as dichloromethane (DCM) or tetrahydrofuran (THF).

In one embodiment of process A, A represents CH.

Compounds of formula (IIa) may be prepared according to the methods described herein (see Schemes 1 and 2).

Compounds of formula (IIb) are disclosed in German patent application DE 3634942A1. Alternatively, compounds of formula (IIb) may be prepared according to the methods described herein (see Scheme 2). 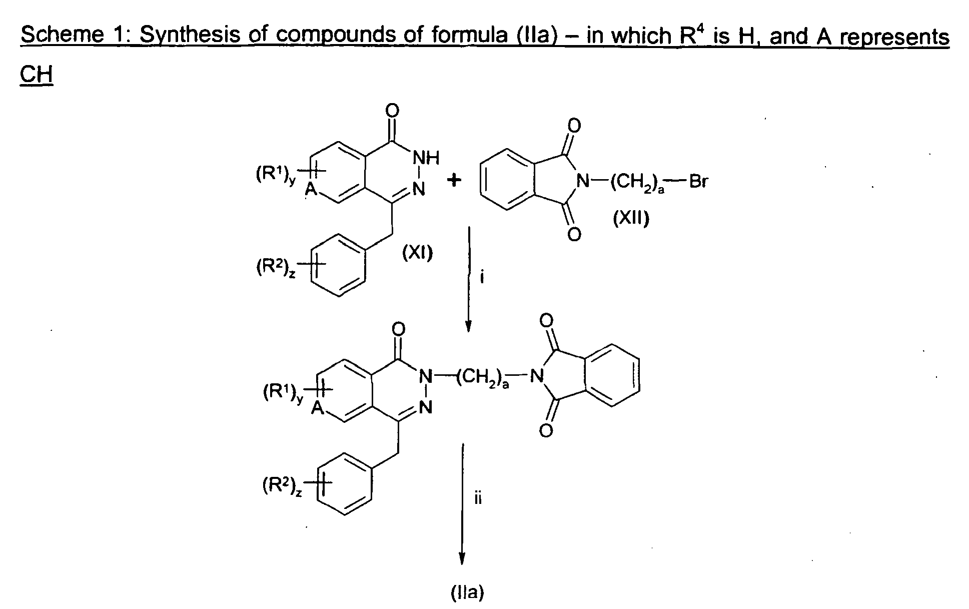 wherein A represents CH, and R¹, y, R², z and a are as defined hereinabove for formula (I).

**Reagents and Conditions**: i) suitable base e.g. sodium hydride (NaH), solvent e.g. DMF; ii) hydrazine (NH₂NH₂), solvent e.g. ethanol (EtOH), at an elevated temperature such as under reflux.

For compounds of formula (IIa) in which R⁴ is C₁₋₆alkyl, an optional alkylation reaction may be carried out. Typically, the alkylation reaction takes place using an alkylating agent, R⁴-L (wherein, R⁴ is as defined hereinabove and L represents a leaving group such as chlorine, bromine, iodine, or an activated hydroxyl such as mesylate or tosylate), with a suitable base e.g. potassium carbonate (K₂CO₃), in a solvent such as 2-butanone, usually at an elevated temperature e.g. about 80 °C, optionally using microwave irradiation and optionally with the addition of an activating agent such as KI or Nal.

Syntheses of compounds of formula (XI), in which A represents CH, are disclosed in US patent US 4,841,047, US patent 1,377,231 and by G Scheffer et al. in Arch. Pharm., 321:205-208 (1988), (see compound 4). Alternatively, compounds of formula (XI) may be prepared according to the methods described herein (see Scheme 2).

Compounds of formula (XII) are commercially available, for example, from Sigma-Aldrich, such as *N*-(bromomethyl)phthalimide, *N*-(2-bromoethyl)phthalirnide and *N*-(3-bromopropyl)phthalimide. 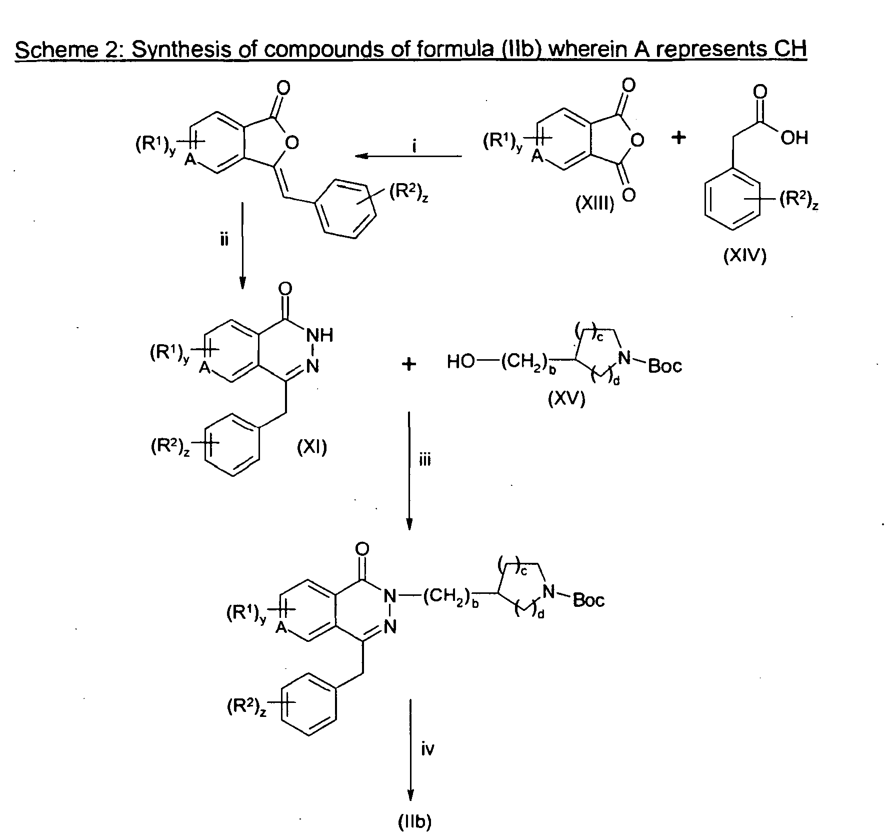 wherein A represents CH, and R¹, y, R², z, b, c and d are as defined hereinabove for formula (I).

**Reagents and Conditions**: i) elevated temperature e.g. such as between about 180 °C and about 250 °C, suitable base e.g. sodium acetate (NaOAc), suitable solvent such as N-methyl-2-pyrrolidinone (NMP); ii) NH₂NH₂, or hydrazine sulfate and sodium hydroxide (NaOH), in a suitable solvent such as ethanol; iii) suitable solvent e.g. tetrahydrofran (THF), appropriate azodicarboxylate e.g. diisopropylazodicarboxylate (DIAD) or other reagent such as tetrabutylammonium bromide (TBAD), suitable phosphine e.g. triphenylphosphine (PPh₃), optionally at a lowered temperature such as at about -15 °C; iv) deprotection using an acid e.g. hydrogen chloride (HCl) or trifluoroacetic acid (TFA), solvent e.g. dioxane or DCM.

In a modification of the synthesis described above, steps iii and iv may be performed sequentially, without isolation of the Boc-protected intermediate.

In another modification of the above reaction scheme, the alcohol group in compounds of formula (XV) may be activated to increase reactivity, to give a group such as either a mesylate or tosylate. Typically, the coupling reaction between an activated compound of formula (XV) and a compound of formula (XI) takes place using a suitable base, such as cesium carbonate or potassium carbonate optionally at an elevated temperature, such as approximately 100 °C, in a suitable solvent such as methyl isobutyl ketone (MIBK).

Compounds of formula (XIII) in which A represents CH are commercially available, for example, from Sigma-Aldrich, Apollo, Fluorochem, Apin, Davos or Merck, such as phthalic anhydride, 3-chlorophthalic anhydride, 4-chlorophthalic anhydride, 4-bromophthalic anhydride, 5-bromo-isobenzofuran-1,3-dione, 3-fluorophthalic anhydride, 4-fluorophthalic anhydride, 3,6-dichlorophthalic anhydride, 4,5-dichlorophthalic anhydride, 4,5-difluorophthalic anhydride, 3,6-difluorophthalic anhydride, 3-hydroxyphthalic anhydride and 4-methylphthalic anhydride and/or may be prepared using methods well known to those skilled in the art, for example 3,6-dihydroxyphthalic anhydride may be prepared from 3,6-diacetoxyphthalic anhydride, which is commercially available, for example, from Wako. C₁₋₆alkyl substituted phthalic anhydrides may be prepared using methods well known to those skilled in the art from the commercially available bromide compounds. Such a reaction may typically be carried out using the appropriate trialkylborane (for example, triethylborane and tributylborane are available for example from Sigma-Aldrich), with an appropriate palladium catalyst, such as [1,1'-bis(diphenylphosphino)ferrocene] dichloropalladium at an appropriate elevated temperature e.g. 70 - 100 °C, with a suitable base such as K₂CO₃, in a suitable solvent e.g. DMF.

Compounds of formula (XIV) are commercially available, for example, from Sigma-Aldrich, Wako, Fluka or Apollo, such as phenylacetic acid, 3-iodophenylacetic acid, 2-bromophenylacetic acid, 4-bromophenylacetic acid, 3-chlorophenylacetic acid, 4-chlorophenylacetic acid, 3-fluorophenylacetic acid, 4-methylphenylacetic acid, 4-isopropylphenylacetic acid, 4-*tert-*butylphenylacetic acid, 4-methoxyphenylacetic acid, 4-ethoxyphenylacetic acid, 4-*N*-butoxyphenylacetic acid, 4-hydroxyphenylacetic acid, 3-(trifluoromethyl)phenylacetic acid, 4-(trifluoromethyl)phenylacetic acid, 4-(bromomethyl)phenylacetic acid, 2,4-dichlorophenylacetic acid, 2-fluoro-3-(trifluoromethyl)phenylacetic acid, 4-hydroxy-3-methoxyphenylacetic acid and 2,4-dimethoxyphenylacetic acid.

Compounds of formula (XV) are commercially available, for example, from Sigma-Aldrich, Magical Scientific, Fluka, SynChem Inc. or Apollo, such as (R)-(-)-*N*-Boc-3-pyrrolidinol, (*S*)-(+)-*N*-Boc-3-pyrrolidinol, (*R*)-1-Boc-2-pyrrolidinemethanol, (*S*)-1-Boc-2-pyrrolidinemethanol, *tert*-butyl 3-hydroxy-1-piperidinecarboxylate, 1-Boc-4-hydroxypiperidine, *N*-Boc-4-piperidinemethanol and 1,1-dimethylethyl 4-hydroxyhexahydro-1*H*-azepine-1-carboxylate. 1,1-dimethylethyl 3-hydroxyhexahydro-1*H-*azepine-1-carboxylate is disclosed in Israeli J. Chem., 37:47-67 (1997).

Compounds of formula (XV) in which the alcohol is activated may be prepared by methods well known to those skilled in the art, for example by mesylation or tosylation of the corresponding commercially available alcohol. Additionally, they may be prepared by methods described herein (see above, and Example 24 C, Stage 3A). The activation reaction may typically be carried out using an appropriate activating agent, such as mesyl chloride (MsCl), with a suitable base e.g. triethylamine (NEt₃) in an appropriate solvent such as *tert*-butyl methyl ether (TBME), usually at a lowered temperature, such as from 0 to 20 °C. 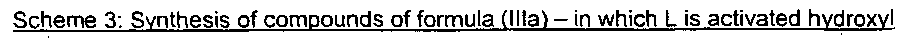 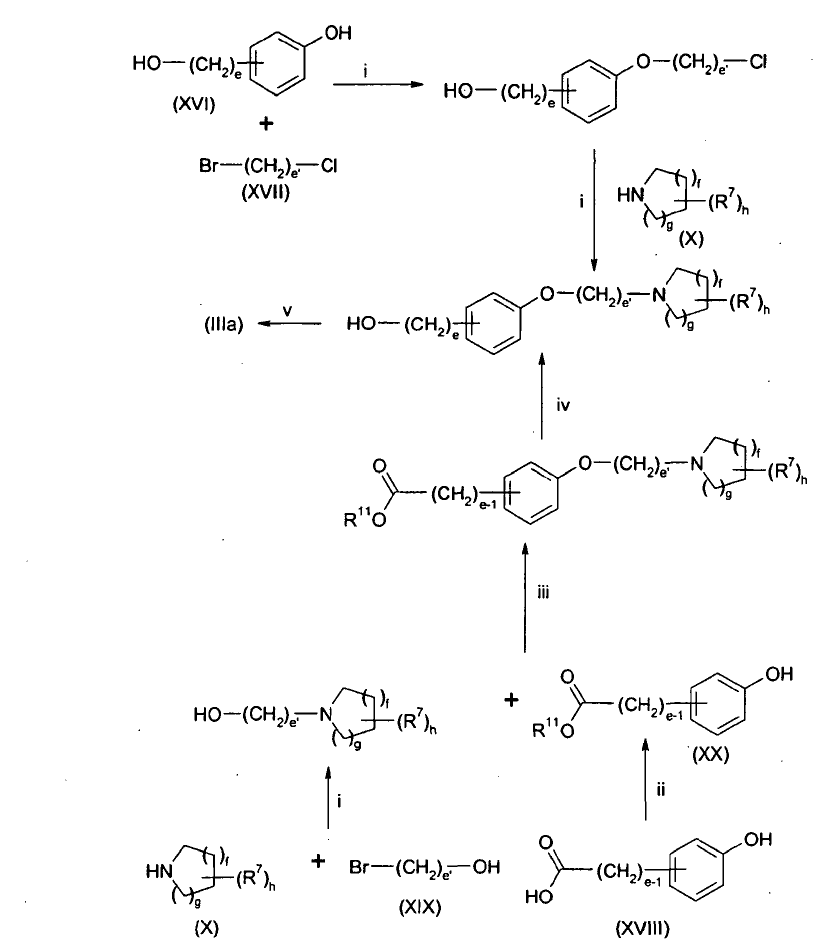 wherein e, e', f, g, h and R⁷ are as defined hereinabove for formula (I) and R¹¹ represents C₁₋₆alkyl.

**Reagents and Conditions:** i) suitable base e.g. K₂CO₃, solvent e.g. 2-butanone, usually an elevated temperature e.g. about 80 °C, optionally using microwave irradiation and optionally with the addition of an activating agent such as KI; ii) R¹¹OH (in which R¹¹ represents C₁₋₆alkyl e.g. methanol [MeOH]), acid such as HCl; iii) solvent e.g. THF, suitable catalysts e.g. DIAD, PPh₃; iv) suitable reducing agent e.g. lithium aluminium hydride (LiAlH₄), solvent e.g. THF and/or diethyl ether; v) suitable activating agent e.g. tosyl chloride (TsCl) or MsCl, an appropriate base e.g. DIPEA in a suitable solvent such as DCM.

Compounds of formula (X) are commercially available, for example, from Sigma-Aldrich or Alfa Aesar, such as azacyclooctane, hexahydro-1*H*-azepine, piperidine, 2-methylpiperidine, 3-methylpiperidine, 4-methylpiperidine, 2,6-dimethylpiperidine, 3,3-dimethylpiperidine, pyrrolidine, 2-methyl pyrrolidine, 2,5-dimethylpyrrolidine and azetidine.

Compounds of formula (XVI) are commercially available, for example, from Sigma-Aldrich, such as 3-hydroxybenzyl alcohol, 4-hydroxybenzyl alcohol, 4-hydroxyphenethyl alcohol and 3-(4-hydroxyphenyl)-1-propanol.

Compounds of formula (XVII) are commercially available, for example, from Sigma-Aldrich, for example 1-bromo-2-chloroethane, 1-bromo-3-chloropropane and 1-bromo-4-chlorobutane.

Compounds of formula (XVIII) are commercially available, for example, from Sigma-Aldrich, such as 3-hydroxybenzoic acid, 4-hydroxybenzoic acid, 2-hydroxyphenylacteic acid, 4-hydroxyphenylacteic acid, 3-(4-hydroxyphenyl)propionic acid and 4-(2-hydroxyphenyl)-butyric acid.

Other compounds of formula (XVIII) may be prepared by methods well known to those skilled in the art, for example by demethylation of the corresponding methoxy compound, which is commercially available. Such a reaction may be carried out for example by using hydrogen bromide in acetic acid (approximately 48%). 4-(4-methoxyphenyl)butyric acid is commercially available, for example, from Sigma-Aldrich.

Compounds of formula (XIX) are available commercially, for example, from Sigma-Aldrich, such as 2-bromoethanol, 3-bromo-1-propanol and 4-bromo-1-butanol.

Compounds of formula (XX) are commercially available, for example, from Apollo or Maybridge, such as ethyl 3-(4-hydroxyphenyl)propanoate. Alternatively, compounds of formula (XX) may be prepared according to the methods described herein (see Scheme 3, above). 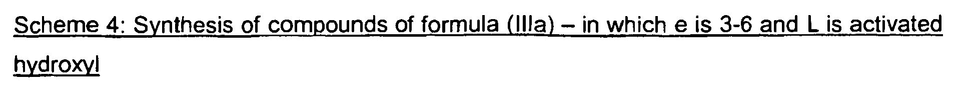 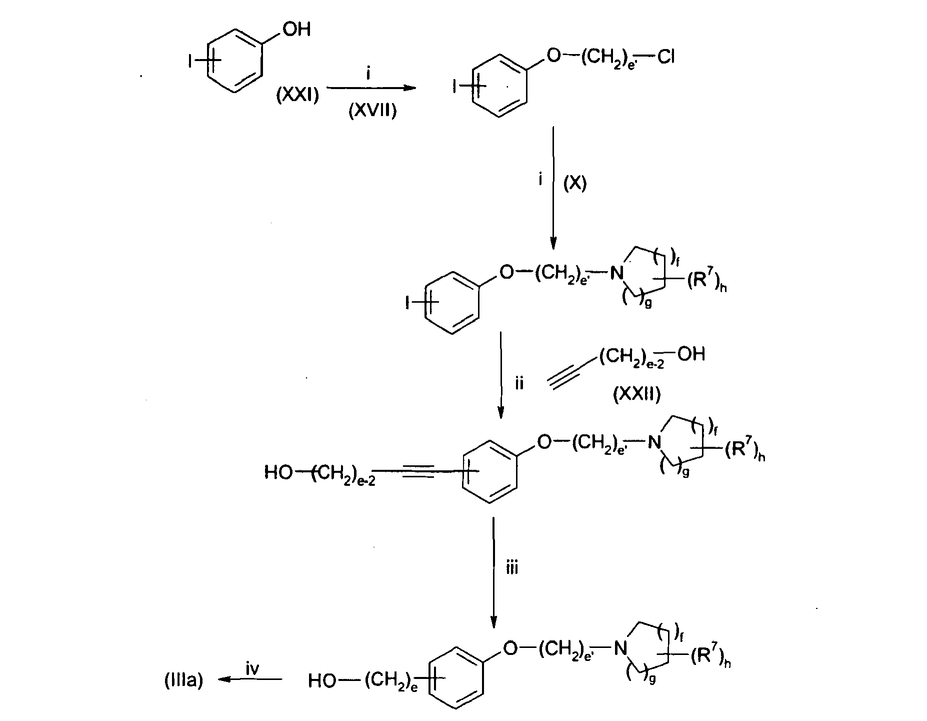 wherein e is 3-6 and e', f, g, h and R⁷ are as defined hereinabove for formula (I).

**Reagents and Conditions:** i) base e.g. K₂CO₃, solvent e.g. 2-butanone, usually at an elevated temperature e.g. about 80 °C, optionally using microwave irradiation and optionally with the addition of an activating agent such as KI or Nal; ii) copper iodide (Cul), appropriate catalyst e.g. bis[triphenylphosphine]palladium (II) chloride (Pd(PPh₃)₂Cl₂), base e.g. NEt₃, solvent e.g. THF; iii) hydrogen, suitable catalyst e.g. palladium on carbon (Pd/C), solvent e.g. EtOH; iv) suitable activating agent e.g. TsCl or MsCl, base e.g. NEt₃ or pyridine, optionally in a suitable solvent e.g. DCM.

Compounds of formula (XXI) are available commercially, for example, from Sigma-Aldrich, for example 2-iodophenol, 3-iodophenol and 4-iodophenol.

Compounds of formula (XXII) are available commercially, for example, from Sigma-Aldrich, such as propargyl alcohol, 3-butyn-1-ol, 4-pentyn-1-ol and 5-hexyn-1-ol. 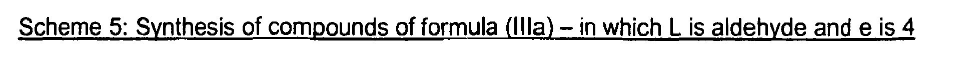 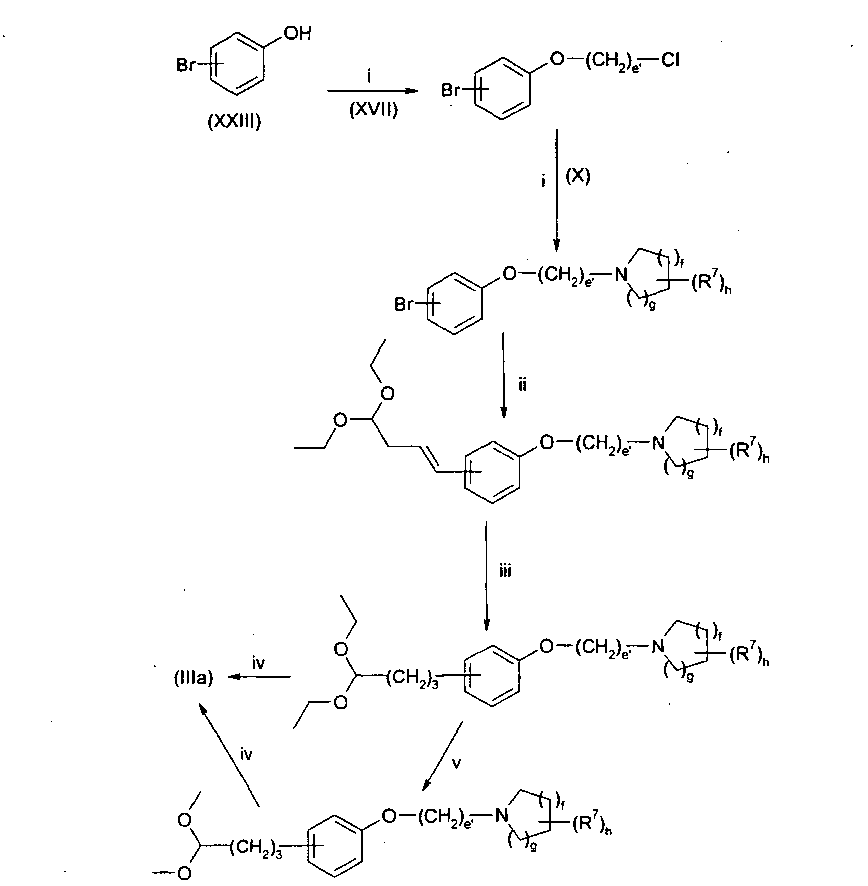 wherein e represents 4 and e', f, g, h and R⁷ are as defined hereinabove for formula (I).

**Reagents and Conditions:** i) base e.g. K₂CO₃, solvent e.g. 2-butanone, usually at an elevated temperature e.g. about 80 °C, optionally using microwave irradiation and optionally with the addition of an activating agent such as KI or Nal; ii) 3-butenal diethylacetal, suitable borane e.g. 9-borabicyclononane (9-BBN), appropriate catalyst e.g. [1,1'-bis(diphenylphosphino)ferrocene]dichloropalladium, base e.g. K₂CO₃, solvent e.g. water/DMF, usually at an elevated temperature e.g. about 70 °C; iii) H₂, catalyst e.g. Pd/C, solvent e.g. EtOH; iv) suitable acid e.g. acetic acid (AcOH); v) MeOH. SCX-2 cartridge.

Compounds of formula (XXIII), such as 2-bromophenol, 3-bromophenol and 4-bromophenol are available commercially, for example, from Sigma-Aldrich. 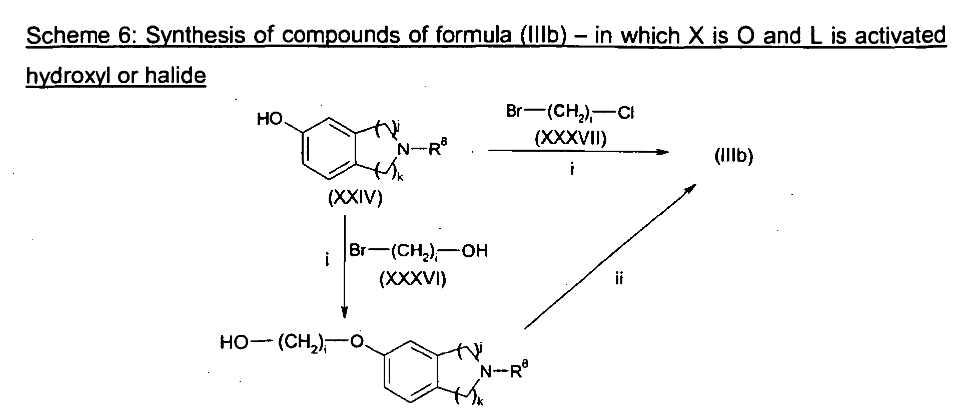 wherein i, j, k, and R⁸ are as defined hereinabove for formula (I).

**Reagents and Conditions:** i) base e.g. K₂CO₃, solvent e.g. 2-butanone, usually at an elevated temperature e.g. about 80 °C, optionally using microwave irradiation and optionally with the addition of an activating agent such as KI; ii) suitable activating agent e.g. TsCl or MsCl, base e.g. NEt₃, solvent e.g. DCM.

Compounds of formula (XXIV) are disclosed in International patent applications WO2004/056369 (see Examples 3 and4) and WO2005/123723 (see Description 17), and are also described in Bioorg. Med. Chem. Lett., 11:685-688 (2001).

Compounds of formula (XXXVI) are available commercially, for example, from Sigma-Aldrich or TCI, such as 2-bromoethanol, 3-bromo-1-propanol, 4-bromo-1-butanol, 5-bromo-1-pentanol and 6-bromo-1-hexanol

Compounds of formula (XXXVII) are commercially available, for example, from Sigma-Aldrich, for example bromochloromethane, 1-bromo-2-chloroethane, 1-bromo-3-chloropropane and 1-bromo-4-chlorobutane, 1-bromo-5-chloropentane and 1-bromo-6-chlorohexane. 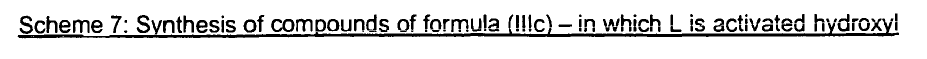 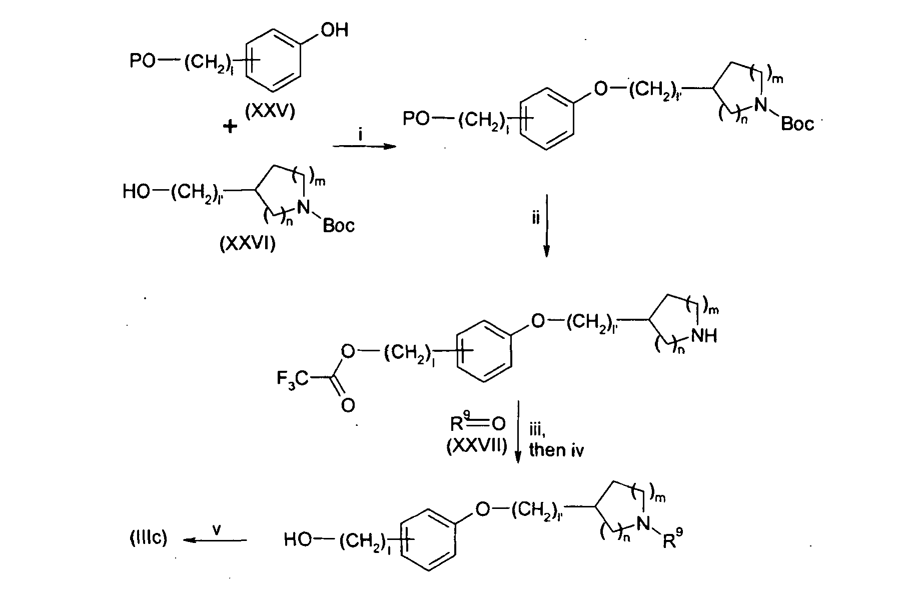 wherein I, I', m, n and R⁹ are as defined hereinabove for formula (I) and P represents a silicon-based protecting group.

**Reagents and Conditions**: i) solvent e.g. THF, suitable azodicarboxylate e.g. DIAD or di-*tert*-butylazodicarboxylate (DTBAD), suitable phosphine e.g. PPh₃; ii) TFA, solvent e.g. DCM; iii) appropriate reducing agent e.g. NaBH(OAc)₃, solvent e.g. MeOH, acid catalyst e.g. AcOH; iv) 2N sodium hydroxide; v) activating agent e.g. TsCl or MsCl, suitable base e.g. NEt₃, solvent e.g. DCM.

Compounds of formula (XXV) may be prepared by methods well known to a person skilled in the art, by protection of the corresponding commercially available alcohol (for example 4-(2-hydroxyethyl)phenol, which is available, for example, from Sigma-Aldrich) or by using methods described herein (see, for example, Intermediate 52).

Compounds of formula (XXVI) are commercially available, for example, from Sigma-Aldrich, Magical Scientific, Fluka, SynChem Inc. or Apollo, such as (*R*)-(-)-*N*-Boc-3-pyrroiidinoi, (*S*)-(+)-*N*-Boc-3-pyrrolidinol, (*R*)-1-Boc-2-pyrrolidinemethanol, (*S*)-1-Boc-2-pyrrolidinemethanol, *tert*-butyl 3-hydroxy-1-piperidinecarboxylate, 1-Boc-4-hydroxypiperidine, *N*-Boc-4-piperidinemethanol and 1,1-dimethylethyl 4-hydroxyhexahydro-1*H*-azepine-1-carboxylate. 1,1-dimethylethyl 3-hydroxyhexahydro-1*H-*azepine-1-carboxylate is disclosed in Israeli J. Chem., 37:47-67 (1997).

Compounds of formula (XXVI) may also be prepared by methods well known to a person skilled in the art, such as Boc-protection of the commercially available amines, for example 3-(hydroxymethyl)pyrrolidine, 2-(2-hydroxyethyl)piperidine, 4(2-hydroxyethyl)piperidine which are commmercially available, for example, from Sigma-Aldrich or Albernate Corporation.

Compounds of formula (XXVII) are commercially available, for example, from Sigma-Aldrich, such as cyclobutanone, cyclopentanone, cyclohexanone, formaldehyde, acetaldehyde, propionaldehyde, 2-propanone, 2-butanone, butyraldehyde, valeraldehyde and hexanal. 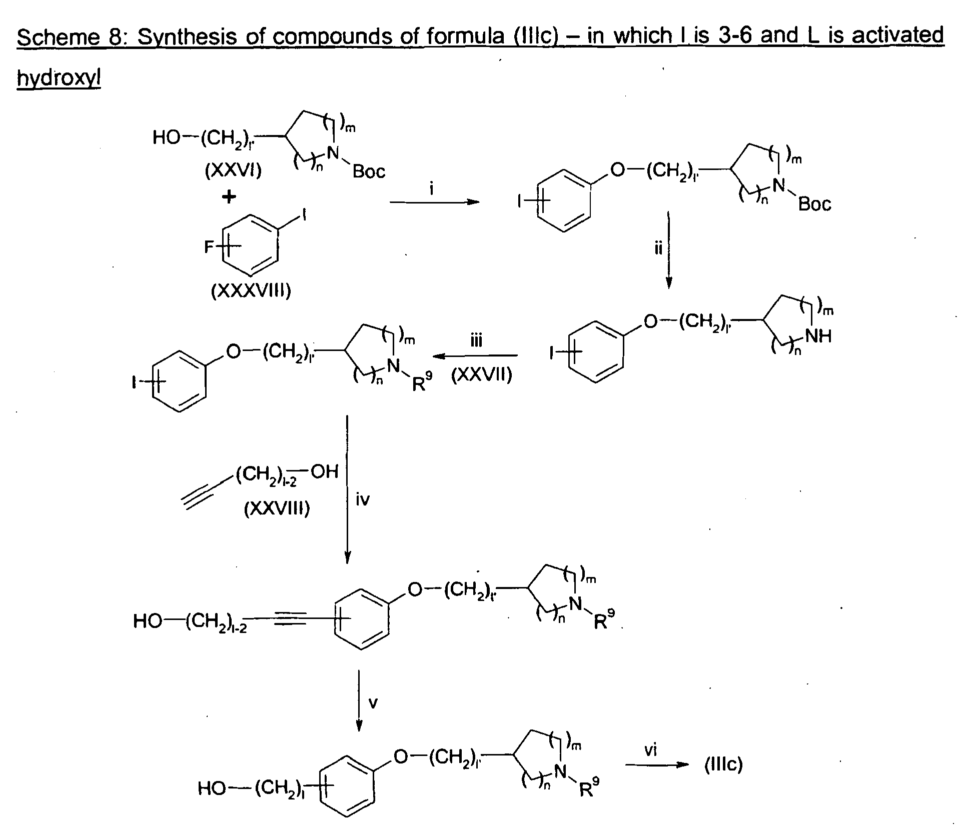 wherein I is 3-6 and I', m, n and R⁹ are as defined hereinabove for formula (I).

**Reagents and Conditions**: i) Suitable base e.g. sodium hydride, suitable solvent e.g. N-methyl-2-pyrrolidinone, elevated temperature such as about 80 °C; ii) suitable acid e.g. TFA, solvent e.g. DCM; iii) appropriate reducing agent e.g. NaBH(OAc)₃, solvent e.g. DCM, optional acid catalyst e.g. AcOH; iv) Cul, suitable catalyst e.g. Pd(PPh₃)₂Cl₂. base e.g. NEt₃, solvent such as THF; v) H₂, catalyst e.g. Pd/C, solvent e.g. EtOH; vi) appropriate activating agent e.g. TsCl or MsCl, base such as NEt₃, solvent e.g. DCM.

Compounds of formula (XXXVIII), such as 1-fluoro-2-iodobenzene, 1-fluoro-3-iodobenzene and 1-fluoro-4-iodobenzene are available commercially, for example, from Sigma-Aldrich.

Compounds of formula (XXVIII), such as propargyl alcohol, 3-butyn-1-ol, 4-pentyn-1-ol and 5-hexyn-1-ol are available commercially, for example, from Sigma-Aldrich.

According to a second process, B, compounds of formula (I) wherein R³ is a group of formula (i) and wherein A represents CH, may be prepared by reacting a compound of formula (IV) 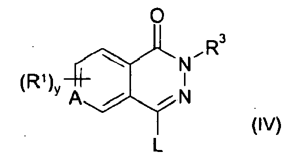 wherein A represents CH, R¹ and y are as defined hereinabove for formula (I), R³ is a group of formula (i) and L represents a leaving group, such as chlorine or bromine,
with a compound of formula (V) 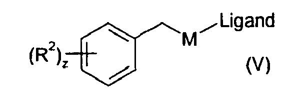 wherein R² and z are as defined hereinabove for formula (I) and M-Ligand represents one of the following groups: Zn-X (in which X is chlorine or bromine), B(OH)₂, SnR₃ (in which R represents C₁₋₆atky), such as butyl) or SiR₃ (in which R represents C₁₋₆alkoxy, such as methoxy or ethoxy).

When M-Ligand is Zn-X, the Negishi coupling reaction may be carried out in the presence of a suitable catalyst, such as tetrakis(triphenylphosphine) palladium (0), (Pd(PPh₃)₄) in an appropriate solvent, such as THF at a suitable temperature.

When M-Ligand is B(OH)₂, the Suzuki coupling reaction may be carried out in the presence of a suitable base, such as K₂CO₃, in the presence of a suitable catalyst, for example Pd(PPh₃)₄, or (diphenylphosphinylferrocene)palladium (II) chloride, (Pd(dppf)Cl₂) in a suitable solvent, e.g. THF or benzene, usually at an elevated temperature, such as about 60 °C.

When M-Ligand is SnR₃, the Stille coupling reaction is typically carried out using a suitable catalyst, such as Pd(PPh₃)₄, in a suitable solvent, such as MeCN or DMF, optionally with an activating agent, such as lithium chloride or Cul, optionally at an elevated temperature, e.g. about 60 °C.

When M-Ligand is SiR₃, the Hiyama coupling reaction may be carried out using a suitable base, such as sodium hydroxide, in an appropriate solvent, e.g. THF with a suitable source of fluoride ions, for example *tert*-butylammonium fluoride, with an appropriate catalyst, such as palladium (II) acetate.

Compounds of formula (IV) may be prepared according to Scheme 9 below.

Compounds of formula (V), such as 3-chlorobenzylzinc chloride, 4-chlorobenzylzinc chloride, 4-bromobenzylzinc chloride, 4-fluorobenzylzinc chloride, 3,4-difluorobenzylzinc chloride, 4-methoxybenzylzinc chloride, 4-ethoxybenzylzinc chloride, 3-methylbenzylzinc chloride, 4-methylbenzylzinc chloride and 3-(trifluoromethyl)benzylzinc chloride are commercially available, for example, from Sigma-Aldrich or Reike Metals.

Compounds of formula (V) may also be prepared by methods well known to a person skilled in the art, such as reaction of the corresponding commercially available benzyl chloride (such as 2-methoxybenzyl chloride, 4-isopropylbenzyl chloride and 4*-tert-*butylbenzyl chloride, which are commercially available, for example, from Sigma-Aldrich) with zinc dust under standard conditions. 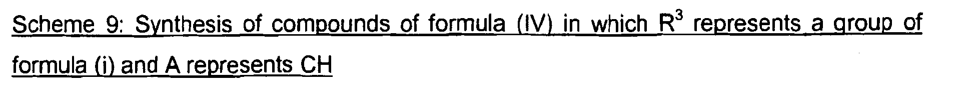 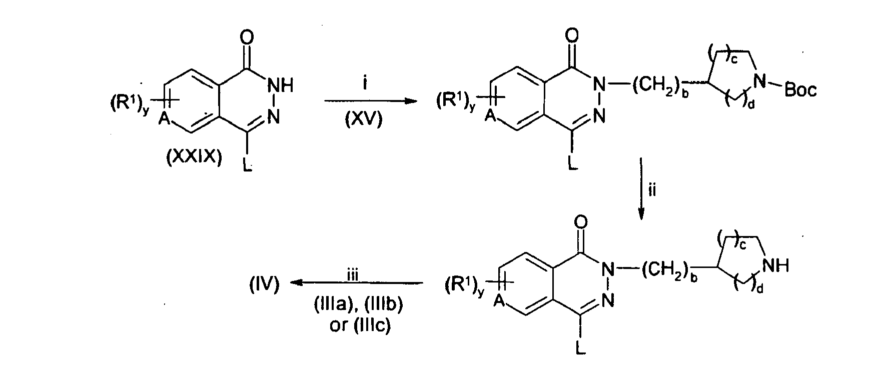 wherein A represents CH, and R¹, y, b, c and d are as described hereinabove for formula (I) and L represents a leaving group, such as chlorine or bromine.

**Reagents and conditions**: i) suitable solvent e.g. THF, appropriate azodicarboxylate e.g. DIAD, suitable phosphine such as PPh₃; ii) acid e.g. HCl, solvent e.g. dioxane; iii) base e.g. NEt₃, solvent e.g. DMF, at an appropriate temperature e.g. about 80 °C.

Compounds of formula (XXIX) are commercially available, for example, from Maybridge or Apollo, for example 4-chloro-1,2-dihydrophthatazin-1-one, and/or may be prepared by methods known to a person skilled in the art, such as that described in Acta Chim. Acad. Sci. Hungaricae, 88:129-136 (1976) from commercially available compounds of formula (XIII) (See scheme 2).

According to a third process, C, a compound of formula (I), wherein R⁵ or R⁶ represent a group of formula (b) in which X represents -N(R¹⁰)C(O)-, may be prepared by reacting a compound of formula (VI) 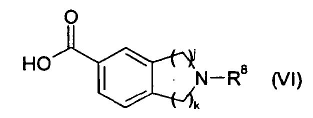 wherein j, k and R⁸ are as defined hereinabove for formula (I),
with a compound of formula (VIIa) or (VIIb) 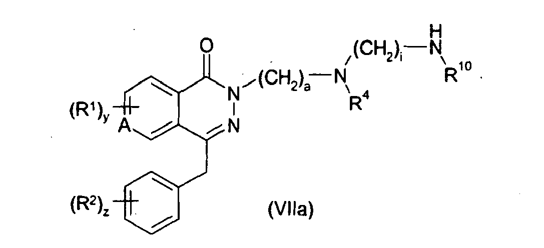 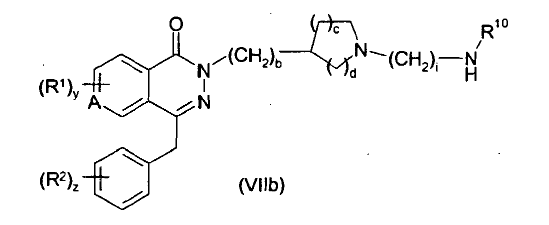 wherein A, R¹, y, R², z, a, b, c, d, I, R⁴ and R¹⁰ are as defined hereinabove for formula (I).

The amide coupling reaction may typically be carried out in the presence of a suitable activating agent, such as O-(1*H*-benzotriazol-1-yl)-*N,N,N',N'-*tetramethyluronium tetrafluoroborate (TBTU) in the presence of a suitable base, such as NEt₃, in an appropriate solvent, such as DMF.

In one embodiment of process C, A represents CH.

Compounds of formula (VI) may be prepared according to Scheme 10: 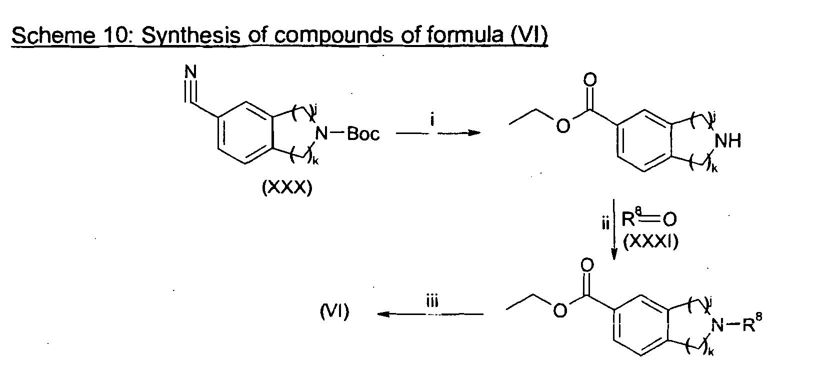 wherein j, k and R⁸ are as defined hereinabove for formula (I).

**Reagents and Conditions**: i) trimethylsilylchloride and EtOH followed by sodium hydroxide (NaOH) and water; ii) appropriate reducing agent e.g. NaBH(OAc)₃, solvent e.g. DCM, optional acid catalyst e.g. AcOH; iii) NaOH, water, EtOH.

Compounds of formula (XXX) are disclosed in European patent application EP 0528369A2 (see Example 17). *tert*-Butyl-5-cyanoisoindoline-2-carboxylate is commercially available, for example, from Milestone Pharm. Tech.

Compounds of formula (XXXI) are commercially available, for example, from Sigma-Aldrich, for example cyclobutanone, cyclopentanone, cyclohexanone, formaldehyde, acetaldehyde, propionaldehyde, 2-propanone, 2-butanone, butyraldehyde, valeraldehyde and hexanal.

Compounds of formula (VIIa) and (VIIb) may be prepared according to the following reaction scheme: 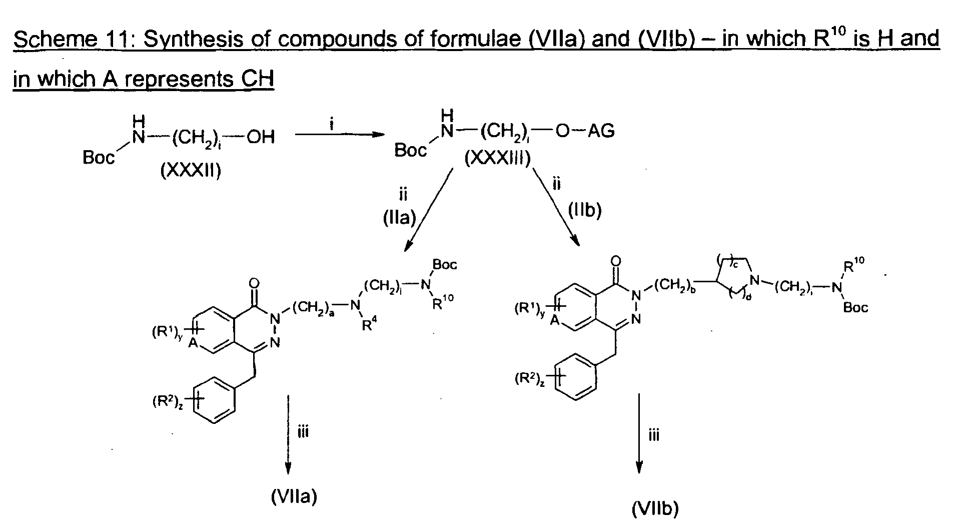 wherein R¹⁰ is H, A represents CH, and i, R¹, R², y, z, a, b, c, d and R⁴ are as defined hereinabove for formula (I) and AG is an activating group, e.g. mesylate or tosylate.

**Reagents and Conditions**: i) suitable activating agent e.g. TsCl or MsCl, base e.g. NEt₃, solvent e.g. DCM; ii) appropriate base e.g. K₂CO₃, solvent such as 2-butanone, usually at an elevated temperature e.g. about 80 °C, optionally using microwave irradiation and optionally with the addition of an activating agent such as KI; iii) appropriate acid such as HCl, solvent e.g. dioxane.

For compounds of formulae (VIIa) and (VIIb) in which R¹⁰ represents C₁₋₆alkyl, an optional alkylation reaction may be carried out. Typically, the alkylation reaction takes place using an alkylating agent R¹⁰-L (wherein, L is a leaving group as defined hereinabove), with a suitable base e.g. K₂CO₃, in a solvent such as 2-butanone, usually at an elevated temperature e.g. about 80 °C. Optionally, there may be included an activating agent such as KI or Nal.

Compounds of formula (XXXII) are available commercially, for example, from Sigma-Aldrich, such as N-Boc-aminoethanol, 3-(Boc-amino)-1-propanol, 4-(Boc-amino)-1-butanol 5-(Boc-amino)-1-pentanol and 6-(Boc-amino)-1-hexanol.

Compounds of formula (XXXIII) may be prepared according to methods described in L.E. Canne, R.L. Winston, S.B.H. Kent, Tet. Lett., 38:3361-4 (1997), see compound 2, S. Kondo et al., J. Antibiotics, 34:1625-7 (1981) and W. Hu, E. Reder, M. Hesse, Helv. Chim. Acta, 79:2137-51 (1996), see compound 6.

According to a fourth process, D, a compound of formula (I), wherein R⁵ or R⁶ represent a group of formula (b) in which X represents a bond, may be prepared by reacting a compound of formula (VIII) 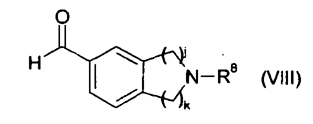 wherein j, k and R⁸ are as defined hereinabove for formula (I),
with a compound of formula (IIa) or (IIb).

The reductive amination reaction may typically be carried out using a suitable reducing agent such as NaBH(OAc)₃, optionally in the presence of a suitable acid catalyst such as acetic acid, in an appropriate solvent such as DCM or THF.

In one embodiment of process D, A represents CH.

Compounds of formula (VIII) may be prepared according to scheme 12: 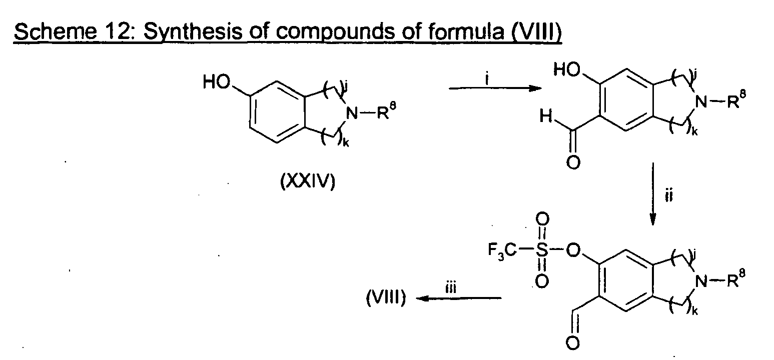 wherein j, k, and R⁸ are as defined hereinabove for formula (I).

**Reagents and Conditions**: i) methyl magnesium bromide, suitable solvent such as THF, then paraformaldehyde, in a solvent such as toluene, suitable base e.g. NEt₃, elevated temperature e.g. about 80 °C; ii) suitable base such as NEt₃, solvent such as DMF, 1,1,1-trifluoro-*N*-phenyl-*N*-[(trifluoromethyl)suffonyl]methanesulfonamide; iii) palladium (II) acetate, 1,3-bis(diphenylphosphino)propane, trioctylsilane, elevated temperature e.g. about 75 °C.

The intermediate triflate compound may or may not be isolated as desired.

Compounds of formula (XXIV) are disclosed in International patent applications WO2004/056369 (see Examples 3 and 4) and WO2005/123723 (see Description 17), and are also described in Bioorg. Med. Chem. Lett., 11:685-688 (2001).

According to a fifth process E, a compound of formula (I) wherein R⁵ or R⁶ represent a group of formula (a), may be prepared by reacting a compound of formula (IXa) or (IXb) 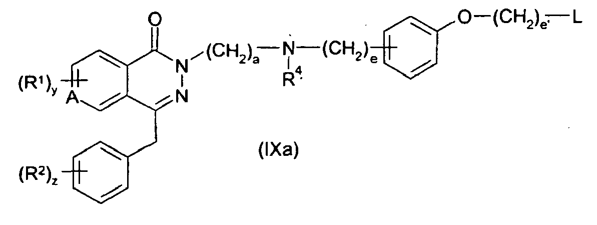 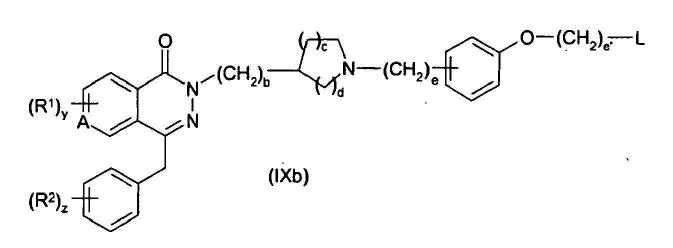 wherein A, R¹, y, R², z, a, b, c, d, e, e' and R⁴ are as defined hereinabove for formula (I), and L represents a leaving group such as chlorine, bromine, iodine,
with a compound of formula (X) 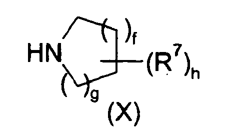 wherein f, g, h and R⁷ are as defined hereinabove for formula (I).

The alkylation reaction may typically be carried out in a suitable solvent such as 2-butanone, MIBK or acetone, optionally in the presence of an activating agent, such as sodium or potassium iodide and an appropriate base such as DIPEA or potassium carbonate, at an appropriate temperature such as at reflux.

In one embodiment of process E, A represents CH.

Compounds of formula (IXa) and (IXb) may be prepared according to the following reaction scheme: 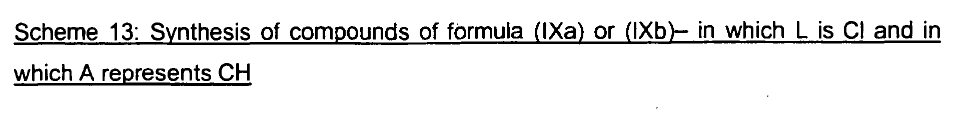 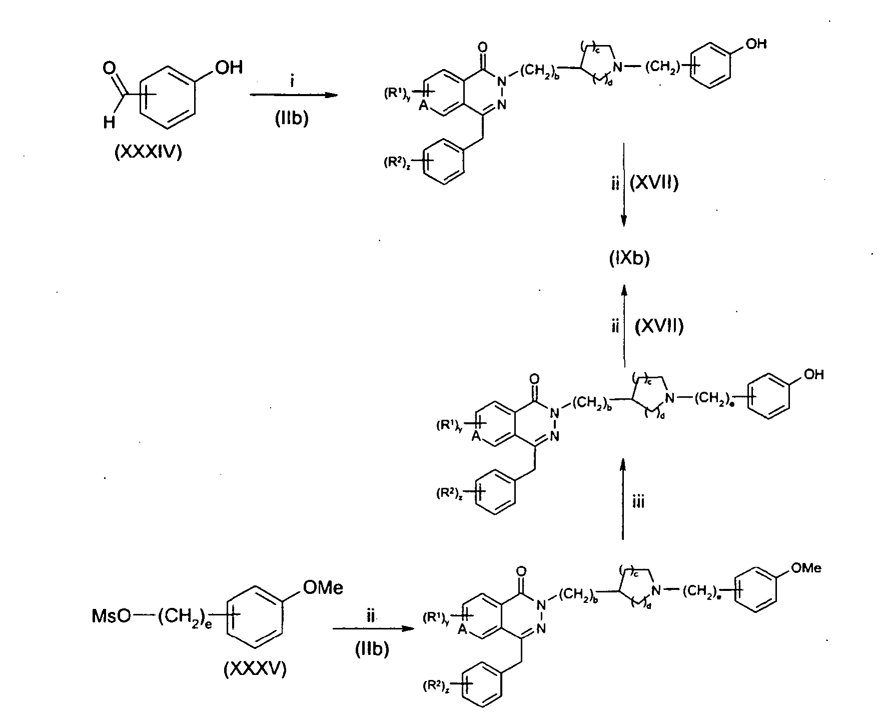 wherein R¹, y, R², z, b, c, d, e and e' are as described hereinabove for formula (I) and Ms represents mesylate. It will be appreciated that replacement of a compound of formula (IIb) with a compound of formula (IIa) will give compounds of formula (IXa) employing the same sequence of reactions and reagents.

**Reagents and conditions:** i) appropriate reducing agent e.g. NaBH(OAc)₃, solvent e.g. DCM, optional addition of an acid catalyst such as AcOH; ii) suitable base e.g. K₂CO₃, solvent e.g. 2-butanone or MIBK, usually at an elevated temperature e.g. about 80 °C or at reflux, optionally using microwave irradiation and optionally with the addition of an activating agent such as KI or Nal; iii) BBr₃, solvent such as DCM at a suitable temperature such as at -60 °C to room temperature e.g. 0°C to room temperature.

If desired, compounds of formula (IXa) and (IXb) need not be isolated before use in process E.

Compounds of formula (XXXIV) are available commercially, for example, from Sigma-Aldrich, such as 2- hydroxybenzaldehyde, 3-hydroxybenzaldehyde and 4-hydroxybenzaldehyde.

Compounds of formula (XXXV) may be prepared by methods well known to a person skilled in the art and/or by the methods described herein, such as activation of the corresponding alcohols. 2-methoxybenzyl alcohol, 3-methoxybenzyl alcohol, 4-methoxybenzyl alcohol, 2-(3-methoxyphenyl)ethanol, 2-(4-methoxyphenyl)ethanol, 3-(4-methoxyphenyl)-1-propanol and 4-[4-(methyloxy)phenyl]-l-butanol are available from Sigma-Aldrich, for example. Such an activation reaction may, for example, use an appropriate activating agent, such as MsCl, in a suitable solvent e.g. TBME with an appropriate base such as NEt₃.

### Compounds of formula (I) in which A represents N

The compounds of formula (I) in which A represents N may be prepared by the methods described herein.

More particularly, compounds of formula (I) in which A represents N may be prepared from compounds of formula (IIa) and (IIb) in which A represents N generally in accordance with the reactions described in process A, process C (scheme 11), process D and process E (scheme 13). Further compounds of formula (I) wherein A represents N may be prepared according to processes F and G.

Compounds of formula (IIa) and (IIb) in which A represents N may be prepared from compounds of formula (XI) in which A represents N generally in accordance with the reactions described in schemes 1 and 2 above.

Compounds of formula (XI) in which A represents N may be prepared according to Scheme 14, below. 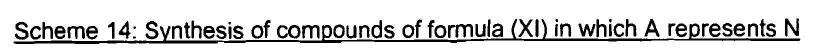 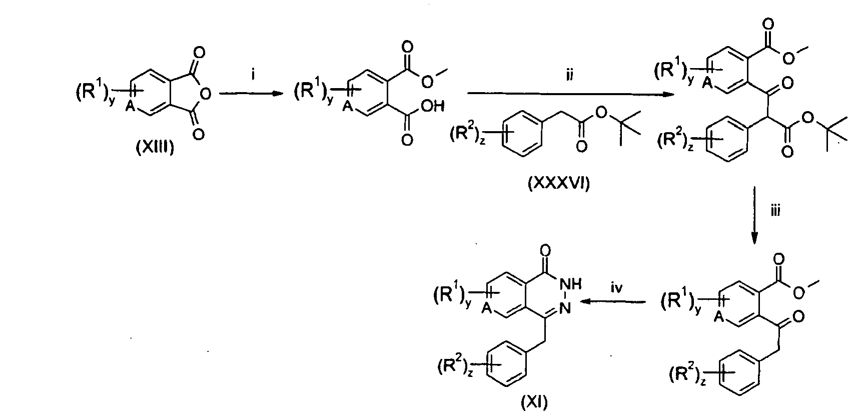 wherein R¹, R², y and z are as described hereinabove for formula (I).

**Reagents and conditions:** i) Sodium methoxide, THF/MeOH; ii) a) suitable activating agent such as carbonyl diimidazole or oxalyl chloride, suitable solvent such as DMF, appropriate elevated temperature such as at approximately 50 °C, b) appropriate base for example NaH, c); iii) suitable acid catalyst for example TFA, appropriate solvent such as DCM; iv) H₂NNH₂.H₂O, in an appropriate solvent for example EtOH, catalytic amount of acid such as AcOH.

Compounds of formula (XIII) in which A represents N are commercially available, for example, from Sigma-Aldrich, such as pyridine-3,4-dicarboxylic anhydride. 2-methylpyridine-4,5-dicarboxylic anhydride may be prepared according to the methods described by Werner, W. Graefe, U., Ihn, W., Tresselt, D., Winter, S., Paulus, E., Tetrahedron, 53(1):109-118 (1997), see compound 4. 3-Methoxypyridine-4,5-dicarboxylic anhydride may be prepared according to the methods disclosed by Krapcho, A. P., Maresch, M. J., Gallagher, C. E., Hacker, M. P., J. Het. Chem., 32(6):1693-702, (1995), see compound 10. 2-Methyl-3,4-pyridinedicarboxylic anhydride may be prepared according to the methods described by Moriconi, E. J. and Spano, F. A., J. Amer. Chem. Soc., 86(1):38-46, (1964), see compound 14.

Compounds of formula (XXXVI) may be prepared by the methods disclosed in scheme 15, below, or by the methods described in WO 2002/079143 (see Preparation 149). 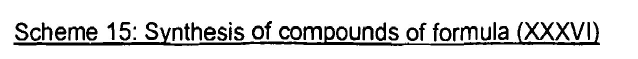 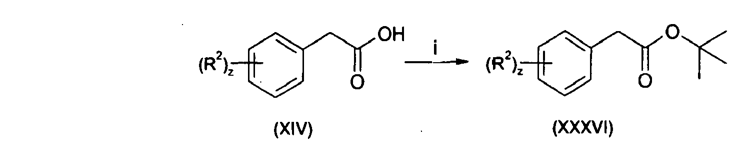 wherein R² and z are as described hereinabove for formula (I).

**Reagents and conditions:** i) dimethylformamide di-*tert*-butyl acetal, suitable solvent such as toluene, elevated temperatue, e.g. 80 °C, for approximately 18 h.

Dimethylformamide di-*tert*-butyl acetate is commercially available, for example, from Sigma-Aldrich.

Availability of compounds of formula (XIV) are described above (see Scheme 2).

According to a sixth process F, a compound of formula (I), may be prepared by interconversion from other compounds of formula (I).

Interconversions include, but are not limited to alkylation and deprotection, under standard conditions well known to those skilled in the art.

Thus, typically, an alkylation reaction may be carried out between a compound of formula (I) and a C₁₋₆alkyl, activated to substitution by means of a leaving group such as halogen or an activated hydroxyl group. The reaction takes place in the presence of a suitable base such as NEt₃ or DIPEA, in an appropriate solvent such as 2-butanone or DMF at an appropriate temperature such as at about 80 °C.

Examples of protecting groups that may be employed in the synthetic routes described and the means for their removal can be found in T. W. Greene 'Protective Groups in Organic Synthesis' (3rd edition, J. Wiley and Sons, 1999). Suitable amine protecting groups include sulfonyl (e.g. tosyl), acyl (e.g. acetyl, 2',2',2'-trichloroethoxycarbonyl, benzyloxycarbonyl or *t*-butoxycarbonyl) and arylalkyl (e.g. benzyl), which may be removed by hydrolysis (e.g. using an acid such as hydrogen chloride in dioxane or trifluoroacetic acid in dichloromethane) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃), which may be removed by base catalysed hydrolysis or a solid phase resin bound benzyl group, such as a Merrifield resin bound 2,6-dimethoxybenzyl group (Ellman linker), which may be removed by acid cleavage, for example with trifluoroacetic acid.

In one embodiment of process F, A represents CH.

According to a seventh process, G, a salt of a compound of formula (I) may be prepared by exchange of counterions, or precipitation of said salt from the free base.

In one embodiment of process G, A represents CH.

It will be appreciated that all novel intermediates used to prepare compounds of formula (I) form a further embodiment of the present invention.

In one embodiment there is provided a compound of formula (IIb) which is 4-[(4-chlorophenyl)methyl]-2-[(2*R*)-2-pyrrolidinylmethyl]-1(2*H*)-phthalazinone (Intermediate 4) or a salt thereof.

Examples of disease states in which a compound of formula (I), or a pharmaceutically acceptable salt thereof may have potentially beneficial anti-inflammatory and/or anti-allergic effects include inflammatory and/or allergic diseases of the respiratory tract, such as allergic rhinitis or other diseases such as bronchitis (including chronic bronchitis), asthma (including allergen-induced asthmatic reactions), chronic obstructive pulmonary disease (COPD), sinusitis and allergic rhinitis (seasonal and perennial).

Furthermore, the compounds of formula (I) may be of use in the treatment of nephritis, skin diseases such as psoriasis, eczema, allergic dermatitis and hypersensitivity reactions. Also, the compounds of formula (I) may be useful in the treatment of insect bites and stings.

The compounds of formula (I) may also be of use in the treatment of nasal polyposis, conjunctivitis or pruritis.

A disease of particular interest is allergic rhinitis.

Other diseases in which histamine may have may have a pathophysiological role include non-allegic rhinitis, and also diseases of the gastrointestinal tract such as intestinal inflammatory diseases including inflammatory bowel disease (e.g.Crohn's disease or ulcerative colitis) and intestinal inflammatory diseases secondary to radiation exposure or allergen exposure.

It will be appreciated by those skilled in the art that references herein to treatment or therapy extend to prophylaxis as well as the treatment of established conditions.

As mentioned above, compounds of formula (I) may be useful as therapeutic agents. There is thus provided a compound of formula (I) or a pharmaceutically acceptable salt thereof for use in therapy.

In another embodiment, there is provided a compound which is 4-[(4-chlorophenyl)methyl]-2-({(2R)-1-[4-(4-{[3-(hexahydro-1*H*-azepin-1-yl)propyl]oxy} phenyl)butyl] -2-pyrrolidinyl}methyl)-1(2*H*)-phthalazinone or a pharmaceutically acceptable salt thereof for use in therapy.

In one embodiment, there is provided the use of a compound of formula (I) or a pharmaceutically acceptable salt thereof for the manufacture of a medicament for the treatment of any of the above diseases.

In another embodiment, there is provided the use of a compound which is 4-[(4-chlorophenyl)methyl]-2-({(2*R*)-1-[4-(4-{[3-(hexahydro-1*H*-azepin-1-yl)propyl]oxy} phenyl)butyl]-2-pyrrolidinyl}methyl)-1(2*H*)-phthalazinone or a pharmaceutically acceptable salt thereof for the manufacture of a medicament for the treatment of any of the above diseases.

In another embodiment, there is provided a method for the treatment of any of the above diseases, in a human or animal subject in need thereof, which method comprises administering an effective amount of a compound of formula (I) or a pharmaceutically acceptable salt thereof.

In another embodiment, there is provided a method for the treatment of any of the above diseases, in a human or animal subject in need thereof, which method comprises administering an effective amount of a compound which is 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl} methyl)-1(2*H*)-phthalazinone or a pharmaceutically acceptable salt thereof.

When used in therapy, the compounds of formula (I) or pharmaceutically acceptable salts thereof may typically be formulated in a suitable composition. Such compositions may be prepared using standard procedures.

Thus, there is provided a composition which comprises a compound of formula (I) or a pharmaceutically acceptable salt thereof optionally with one or more pharmaceutically acceptable carriers and/or excipients.

There is further provided a composition which comprises a compound which is 4-[(4-chlorophenyl)methyl]-2-({(2*R*)-1-[4-(4-{[3-(hexahydro-1*H*-azepin-1-yl)propyl]oxy}phenyl) butyl]-2-pyrrolidinyl}methyl)-1(2*H*)-phthalazinone or a pharmaceutically acceptable salt thereof optionally with one or more pharmaceutically acceptable carriers and/or excipients.

A composition comprising a compound of formula (I) or a pharmaceutically acceptable salt thereof, which may be prepared by admixture, suitably at ambient temperature and atmospheric pressure, may be suitable for topical administration (which includes epicutaneous, inhaled, intranasal or ocular administration), enteral administration (which includes oral or rectal administration) or parenteral administration (such as by injection or infusion). Of interest are compositions comprising a compound of formula (I) or a pharmaceutically acceptable salt thereof, suitable for topical administration, particularly suitable for intranasal administration.

Generally, compositions may be in the form of solutions or suspensions (aqueous or non-aqueous), tablets, capsules, oral liquid preparations, powders, granules, lozenges, lotions, creams, ointments, gels, foams, reconstitutable powders or suppositories as required by the route of administration.

Generally, the compositions comprising a compound of formula (I) or a pharmaceutically acceptable salt thereof may contain from about 0.1 % to 99% (w/w) , such as from about 10 to 60% (w/w) (based on the total weight of the composition), of the compound of formula (I) or the pharmaceutically acceptable salt thereof, depending on the route of administration. The dose of the compound used in the treatment of the aforementioned diseases will vary in the usual way with the seriousness of the diseases, the weight of the sufferer, and other similar factors. However, as a general guide, suitable unit doses may be about 0.05 to 1000 mg, for example about 0.05 to 200 mg, and such unit doses may be administered more than once a day, for example two or three times a day or as desired. Such therapy may extend for a number of weeks or months.

The proportion of the compound of formula (1) or a pharmaceutically acceptable salt thereof in a topical composition will depend on the precise type of composition to be prepared and the particular route of administration, but will generally be within the range of from about 0.001 to 10% (w/w), based on the total weight of the composition. Generally, however for most types of preparations the proportion used will be within the range of from about 0.005 to 1% (w/w), such as about 0.01 to 1% (w/w), for example about 0.01 to 0.5% (w/w), based on the total weight of the composition. However, in powders for inhalation the proportion used will generally be within the range of from about 0.1 to 5% (w/w), based on the total weight of the composition.

Generally, compositions suitable for intranasal or inhaled administration may conveniently be formulated as aerosols, solutions, suspensions, drops, gels or dry powders, optionally with one or more pharmaceutically acceptable carriers and/or excipients such as aqueous or non-aqueous vehicles, thickening agents, isotonicity adjusting agents, antioxidants and/or preservatives.

For compositions suitable for intranasal or inhaled administration, the compound of formula (I) or a pharmaceutically acceptable salt thereof may typically be in a particle-size-reduced form, which may be prepared by conventional techniques, for example, micronisation and milling. Generally, the size-reduced (e.g. micronised) compound of formula (I) or a pharmaceutically acceptable salt thereof can be defined by a D₅₀ value of about 0.5 to 10 microns, such as of about 2 to 4 microns (for example as measured using laser diffraction).

In one embodiment, compositions comprising a compound of formula (I) or a pharmaceutically acceptable salt thereof are suitable for intranasal administration. Intranasal compositions comprising a compound of formula (I) or a pharmaceutically acceptable salt thereof may permit the compound(s) to be delivered to all areas of the nasal cavities (the target tissue) and further, may permit the compound(s) to remain in contact with the target tissue for longer periods of time. A suitable dosing regime for intranasal compositions would be for the patient to inhale slowly through the nose subsequent to the nasal cavity being cleared. During inhalation the composition would be administered to one nostril while the other is manually compressed. This procedure would then be repeated for the other nostril. Typically, one or two sprays per nostril would be administered by the above procedure up to two or three times each day, ideally once daily. Of particular interest are intranasal compositions suitable for once daily administration.

Intranasal compositions may optionally contain one or more suspending agents, one or more preservatives, one or more wetting agents and/or one or more isotonicity adjusting agents as desired. Compositions suitable for intranasal administration may optionally further contain other excipients, such as antioxidants (for example sodium metabisulphite), taste-masking agents (such as menthol) and sweetening agents (for example dextrose, glycerol, saccharin and/or sorbitol).

The suspending agent, if included, will typically be present in the intranasal composition in an amount of between about 0.1 and 5% (w/w), such as between about 1.5% and 2.4% (w/w), based on the total weight of the composition. Examples of suspending agents include Avicel®, carboxymethylcellulose, veegum, tragacanth, bentonite, methylcellulose and polyethylene glycols, e.g. microcrystalline cellulose or carboxy methylcellulose sodium. Suspending agents may also be included in compositions suitable for inhaled, ocular and oral administration as appropriate.

For stability purposes, intranasal compositions comprising a compound of formula (I) or a pharmaceutically acceptable salt thereof may be protected from microbial or fungal contamination and growth by inclusion of a preservative. Examples of pharmaceutically acceptable anti-microbial agents or preservatives may include quaternary ammonium compounds (e.g. benzalkonium chlorine, benzethonium chloride, cetrimide and cetylpyridinium chloride), mercurial agents (e.g. phenylmercuric nitrate, phenylmercuric acetate and thimerosal), alcoholic agents (e.g. chlorobutanol, phenylethyl alcohol and benzyl alcohol), antibacterial esters (e.g. esters of para-hydroxybenzoic acid), chelating agents such as disodium ethylenediaminetetraacetate (EDTA) and other anti-microbial agents such as chlorhexidine, chlorocresol, sorbic acid and its salts (such as potassium sorbate) and polymyxin. Examples of pharmaceutically acceptable anti-fungal agents or preservatives may include sodium benzoate. The preservative, if included, may be present in an amount of between about 0.001 and 1% (w/w), such as about 0.015% (w/w), based on the total weight of the composition. Preservatives may be included in compositions suitable for other routes of administration as appropriate.

Compositions which contain a suspended medicament may include a pharmaceutically acceptable wetting agent which functions to wet the particles of medicament to facilitate dispersion thereof in the aqueous phase of the composition. Typically, the amount of wetting agent used will not cause foaming of the dispersion during mixing. Examples of wetting agents include fatty alcohols, esters and ethers, such as polyoxyethylene (20) sorbitan monooleate (Polysorbate 80). The wetting agent may be present in intranasal compositions in an amount of between about 0.001 and 0.05% (w/w), for example about 0.025% (w/w), based on the total weight of the composition. Wetting agents may be included in compositions suitable for other routes of administration, e.g. for inhaled and/or ocular administration, as appropriate.

An isotonicity adjusting agent may be included to achieve isotonicity with body fluids e.g. fluids of the nasal cavity, resulting in reduced levels of irritancy. Examples of isotonicity adjusting agents include sodium chloride, dextrose, xylitol and calcium chloride. An isotonicity adjusting agent may be included in intranasal compositions in an amount of between about 0.1 and 10% (w/w), such as about 5.0% (w/w), based on the total weight of the composition. Isotonicity adjusting agents may also be included in compositions suitable for other routes of administration, for example in compositions suitable for inhaled, ocular, oral liquid and parenteral administration, as appropriate.

Further, the intranasal compositions comprising a compound of formula (I) or a pharmaceutically acceptable salt thereof may be buffered by the addition of suitable buffering agents such as sodium citrate, citric acid, phosphates such as disodium phosphate (for example the dodecahydrate, heptahydrate, dihydrate and anhydrous forms) or sodium phosphate and mixtures thereof. Buffering agents may also be included in compositions suitable for other routes of administration as appropriate.

Compositions for administration topically to the nose or lung for example, for the treatment of rhinitis, include pressurised aerosol compositions and aqueous compositions delivered to the nasal cavities by pressurised pump. Compositions which are non-pressurised and adapted to be administered topically to the nasal cavity are of particular interest. Suitable compositions contain water as the diluent or carrier for this purpose. Aqueous compositions for administration to the lung or nose may be provided with conventional excipients such as buffering agents, tonicity modifying agents and the like. Aqueous compositions may also be administered to the nose by nebulisation.

A fluid dispenser may typically be used to deliver a fluid composition to the nasal cavities. The fluid composition may be aqueous or non-aqueous, but typically aqueous. Such a fluid dispenser may have a dispensing nozzle or dispensing orifice through which a metered dose of the fluid composition is dispensed upon the application of a user-applied force to a pump mechanism of the fluid dispenser. Such fluid dispensers are generally provided with a reservoir of multiple metered doses of the fluid composition, the doses being dispensable upon sequential pump actuations. The dispensing nozzle or orifice may be configured for insertion into the nostrils of the user for spray dispensing of the fluid composition into the nasal cavity. A fluid dispenser of the aforementioned type is described and illustrated in WO05/044354 the entire content of which is hereby incorporated herein by reference. The dispenser has a housing which houses a fluid discharge device having a compression pump mounted on a container for containing a fluid composition. The housing has at least one finger-operable side lever which is movable inwardly with respect to the housing to cam the container upwardly in the housing to cause the pump to compress and pump a metered dose of the composition out of a pump stem through a nasal nozzle of the housing. In one embodiment, the fluid dispenser is of the general type illustrated in Figures 30-40 of WO05/044354.

In one embodiment, there is provided an intranasal composition comprising a compound of formula (I) or a pharmaceutically acceptable salt thereof, particularly a compound which is 4-[(4-chlorophenyl)methyl]-2-({(2*R*)-1-[4-(4-{[3-(hexahydro-1*H*-azepin-1-yl)propyl]oxy} phenyl)butyl]-2-pyrrolidinyl}methyl)-1(2*H*)-phthalazinone or a pharmaceutically acceptable salt thereof. In another embodiment such an intranasal composition is benzalkonium chloride-free.

Inhaled administration involves topical administration to the lung, such as by aerosol or dry powder composition.

Aerosol compositions suitable for inhaled administration may comprise a solution or fine suspension of the compound in a pharmaceutically acceptable aqueous or non-aqueous solvent. Aerosol compositions suitable for inhalation can be either a suspension or a solution and generally contain a compound of formula (I) or a pharmaceutically acceptable salt thereof and a suitable propellant such as a fluorocarbon or hydrogen-containing chlorofluorocarbon or mixtures thereof, such as hydrofluoroalkanes, e.g. 1,1,1,2-tetrafluoroethane, 1,1,1,2,3,3,3-heptafluoro-*n*-propane or a mixture thereof. The aerosol composition may optionally contain additional excipients well known in the art such as surfactants or cosolvents. Examples of surfactants include, but are not limited to oleic acid, lecithin, an oligolactic acid or derivative e.g. as described in WO94/21229 and WO98/34596 An example of a cosolvent includes, but is not limited to ethanol. Aerosol compositions may be presented in single or multidose quantities in sterile form in a sealed container, which may take the form of a cartridge or refill for use with an atomising device or inhaler. Alternatively, the sealed container may be a unitary dispensing device such as a single dose nasal inhaler or an aerosol dispenser fitted with a metering valve (metered dose inhaler), which is intended for disposal once the contents of the container have been exhausted.

Dry powder inhalable compositions may take the form of capsules and cartridges of, for example, gelatine, or blisters of, for example, laminated aluminium foil, for use in an inhaler or insufflator. Such compositions may be formulated comprising a powder mix of a compound of formula (I) or a pharmaceutically acceptable salt thereof and a suitable powder base such as lactose or starch.

Optionally, for dry powder inhalable compositions, a composition suitable for inhaled administration may be incorporated into a plurality of sealed dose containers (e.g. comprising the dry powder composition) mounted longitudinally in a strip or ribbon inside a suitable inhalation device. The container is rupturable or peel-openable on demand and the dose of e.g. the dry powder composition may be administered by inhalation via the device such us the DISKUS^{™} device, marketed by GlaxoSmithKline. The DISKUS TM inhalation device is for example described in GB 2242134 A, and in such a device, at least one container for the composition in powder form (the container or containers may, for example, be a plurality of sealed dose containers mounted longitudinally in a strip or ribbon) is defined between two members peelably secured to one another; the device comprises: a means of defining an opening station for the said container or containers; a means for peeling the members apart at the opening station to open the container; and an outlet, communicating with the opened container, through which a user can inhale the composition in powder form from the opened container.

Aerosol compositions are typically arranged so that each metered dose or "puff" of aerosol contains about 20 µg - 2000 µg, particularly about 20 µg - 500 µg of a compound of formula (I) or a pharmaceutically acceptable salt thereof. Administration may be once daily or several times daily, for example 2, 3, 4 or 8 times, giving for example 1, 2 or 3 doses each time. The overall daily dose with an aerosol will be within the range of about 100 µg - 10 mg, such as between about 200 µg - 2000 µg. The overall daily dose and the metered dose delivered by capsules and cartridges in an inhaler or insufflator will generally be double those with aerosol compositions.

In another embodiment, there is provided a composition comprising a compound of formula (I) or a pharmaceutically acceptable salt thereof which is suitable for epicutaneous administration. An epicutaneous composition to be applied to the affected area e.g. the skin, by one or more application per day, may be in the form of, for example, an ointment, a cream, an emulsion, a lotion, a foam, a spray, an aqueous gel, or a microemulsion. Such compositions may optionally contain one or more solubilising agents, skin-penetration-enhancing agents, surfactants, fragrances, preservatives or emulsifying agents.

Ointments, creams and gels, may, for example, be formulated with an aqueous or oily base with the addition of suitable thickening and/or gelling agent and/or solvents. Such bases may thus, for example, include water and/or an oil such as liquid paraffin or a vegetable oil such as arachis oil or castor oil, or a solvent such as polyethylene glycol. Thickening agents and gelling agents which may be used according to the nature of the base include soft paraffin, aluminium stearate, cetostearyl alcohol, polyethylene glycols, woolfat, beeswax, carboxypolymethylene and cellulose derivatives, and/or glyceryl monostearate and/or non-ionic emulsifying agents. Lotions may be formulated with an aqueous or oily base and will in general also contain one or more emulsifying agents, stabilising agents, dispersing agents, suspending agents or thickening agents.

In another embodiment, there is provided a composition comprising a compound of formula (I) or a pharmaceutically acceptable salt thereof which is suitable for ocular administration. Such compositions may optionally contain one or more suspending agents, one or more preservatives, one or more wetting/lubricating agents and/or one or more isotonicity adjusting agents. Examples of ophthalmic wetting/lubricating agents may include cellulose derivatives, dextran 70, gelatin, liquid polyols, polyvinyl alcohol and povidone such as cellulose derivatives and polyols.

In another embodiment, there is provided a composition comprising a compound of formula (I) or a pharmaceutically acceptable salt thereof which is suitable for oral administration. Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colorants.

In another embodiment, there is provided a composition comprising a compound of formula (I) or a pharmaceutically acceptable salt thereof which is suitable for parenteral administration. Fluid unit dosage forms suitable for parenteral administration may be prepared utilising a compound of formula (I) or pharmaceutically acceptable salt thereof and a sterile vehicle which may be aqueous or oil based. The compound, depending on the vehicle and concentration used, may be either suspended or dissolved in the vehicle. In preparing solutions, the compound may be dissolved for injection and filter sterilised before filling into a suitable vial or ampoule and sealing. Optionally, adjuvants such as a local anaesthetic, preservatives and buffering agents may be dissolved in the vehicle. To enhance the stability, the composition may be frozen after filling into the vial and the water removed under vacuum. The lyophilised parenteral composition may be reconstituted with a suitable solvent just prior to administration. Parenteral suspensions may be prepared in substantially the same manner, except that the compound is suspended in the vehicle instead of being dissolved, and sterilisation cannot be accomplished by filtration. The compound may be sterilised by exposure to ethylene oxide before suspension in a sterile vehicle. A surfactant or wetting agent may be included in the composition to facilitate uniform distribution of the compound.

The compounds and pharmaceutical compositions containing a compound of formula (I) may also be used in combination with or include one or more other therapeutic agents, for example other antihistaminic agents for example H4 receptor antagonists, anticholinergic agents, anti-inflammatory agents such as corticosteroids (e.g. fluticasone propionate, beclomethasone dipropionate, mometasone furoate, triamcinolone acetonide, budesonide and fluticasone furoate); or non-steroidal anti-inflammatory drugs (NSAIDs) (e.g. sodium cromoglycate, nedocromil sodium), PDE-4 inhibitors, leukotriene antagonists, lipoxygenase inhibitors, chemokine antagonists (e.g. CCR3, CCR1, CCR2, CCR4, CCR8, CXCR1, CXCR2), IKK antagonists, iNOS inhibitors, tryptase and elastase inhibitors, beta-2 integrin antagonists and adenosine 2a agonists; or beta adrenergic agents (e.g. salmeterol, salbutamol, formoterol, fenoterol, terbutaline, and the beta agonists described in WO 02/66422, WO 02/270490, WO02/076933 WO03/024439 and WO03/072539 and salts thereof); or antiinfective agents e.g. antibiotic agents (such as retapamulin) and antiviral agents.

It will be clear to a person skilled in the art that, where appropriate, the other therapeutic agent(s) may be used in the form of salts, (e.g. as alkali metal or amine salts or as acid addition salts), or prodrugs, or as esters (e.g. lower alkyl esters), or as solvates (e.g. hydrates) to optimise the activity and/or stability and/or physical characteristics (e.g. solubility) of the therapeutic agent. It will be clear also that where appropriate, the therapeutic agents may be used in optically pure form.

There is provided, in another embodiment, a combination comprising a compound of formula (I) or a pharmaceutically acceptable salt thereof together with one or more (such as one or two, e.g. one) other therapeutically active agents, optionally with one or more pharmaceutically acceptable carriers and/or excipients.

In another embodiment, there is provided a combination comprising a compound which is 4-[(4-chlorophenyl)methyl]-2-({(2*R*)-1-[4-(4-{[3-(hexahydro-1*H*-azepin-1-yl)propyl]oxy} phenyl)butyl]-2-pyrrolidinyl}methyl)-1(2*H*)-phthalazinone or a pharmaceutically acceptable salt thereof together with one or more (such as one or two, e.g. one) other therapeutically active agents, optionally with one or more pharmaceutically acceptable carriers and/or excipients.

Other histamine receptor antagonists which may be used alone, or in combination with a dual H1/H3 receptor antagonist include antagonists (and/or inverse agonists) of the H4 receptor, for example, the compounds disclosed in Jablonowski et al., J. Med. Chem. 46:3957-3960 (2003).

In one embodiment, there is provided a combination comprising a compound of formula (I) or a pharmaceutically acceptable salt thereof and a β₂-adrenoreceptor agonist.

Examples of β₂-adrenoreceptor agonists include salmeterol (which may be a racemate or a single enantiomer, such as the *R*-enantiomer), salbutamol (which may be a racemate or a single enantiomer such as the *R*-enantiomer), formoterol (which may be a racemate or a single diastereomer such as the *R,R*-diastereomer), salmefamol, fenoterol, carmoterol, etanterol, naminterol, clenbuterol, pirbuterol, flerbuterol, reproterol, bambuterol, indacaterol, terbutaline and salts thereof, for example the xinafoate (1-hydroxy-2-naphthalenecarboxylate) salt of salmeterol, the sulfate salt or free base of salbutamol or the fumarate salt of formoterol. In one embodiment, combinations containing a compound of formula (I) may include longer-acting β₂-adrenoreceptor agonists, for example, compounds which provide effective bronchodilation for about 12 h or longer.

Other β₂-adrenoreceptor agonists include those described in WO 02/066422, WO 02/070490, WO 02/076933, WO 03/024439, WO 03/072539, WO 03/091204, WO 04/016578, WO 2004/022547, WO 2004/037807, WO 2004/037773, WO 2004/037768, WO 2004/039762, WO 2004/039766, WO01/4219 and WO03/042160.

Examples of β₂-adrenoreceptor agonists include:
3-(4-{[6-({(2*R*)-2-hydroxy-2-[4-hydroxy-3-(hydroxymethyl)phenyl]ethyl}amino)hexyl]oxy} butyl)benzenesulfonamide;
3-(3-{[7-({(2*R*)-2-hydroxy-2-[4-hydroxy-3-hydroxymethyl) phenyl]ethyl}-amino)heptyl]oxy} propyl)benzenesulfonamide;
4-{(1*R*)-2-[(6-{2-[(2,6-dichlorobenzyl)oxy]ethoxy}hexyl)amino]-1-hydroxyethyl}-2-(hydroxyl methyl) phenol;
4-{(1*R*)-2-[(6-{4-[3-(cyclopentylsulfonyl)phenyl]butoxy}hexyl)amino]-1-hydroxyethyl}-2-(hydroxylmethyl)phenol;
N-[2-hydroxyl-5-[(1*R*)-1-hydroxy-2-[[2-4-[[*(2R)*-2-hydroxy-2-phenylethyl]amino]phenyl] ethyl]amino]ethyl]phenyl]formamide;
N-2{2-[4-(3-phenyl-4-methoxyphenyl)aminophenyl]ethyl}-2-hydroxy-2-(8-hydroxy-2(1*H*)-quinolinon-5-yl)ethylamine; and
5-[(*R*)-2-(2-{4-[4-(2-amino-2-methyl-propoxy)-phenylamino]-phenyl}-ethylamino)-1-hydroxy -ethyl]-8-hydroxy-1H-quinolin-2-one.

The β₂-adrenoreceptor agonist may be in the form of a salt formed with a pharmaceutically acceptable acid selected from sulfuric, hydrochloric, fumaric, hydroxynaphthoic (for example 1- or 3-hydroxy-2-naphthoic), cinnamic, substituted cinnamic, triphenylacetic, sulfamic, sulfanilic, naphthaleneacrylic, benzoic, 4-methoxybenzoic, 2- or 4-hydroxybenzoic, 4-chlorobenzoic and 4-phenylbenzoic acid.

In another embodiment, there is provided a combination comprising a compound of formula (I) or a pharmaceutically acceptable salt thereof and an adenosine 2a agonist.

Adenosine 2a agonists include those disclosed in international patent application no. PCT/EP/2005/005651, such as (2*R*,3*R*,4*S*,5*R*,2'*R*,3'*R*,4'*S*,5'*R*)-2,2'-{*trans*-1,4-cyclo hexanediylbis[imino(2-{[2-(1-methyl-1*H*-imidazol-4-yl)ethyl]amino}-9*H*-purine-6,9-diyl)]}bis [5-(2-ethyl-2*H*-tetrazol-5-yl)tetrahydro-3,4-furandiol].

In another embodiment, there is provided a combination comprising a compound of formula (I) or a pharmaceutically acceptable salt thereof and an anti-inflammatory agent.

Anti-inflammatory agents include corticosteroids. Suitable corticosteroids which may be used in combination with the compounds of formula (I) are those oral and inhaled corticosteroids and their pro-drugs which have anti-inflammatory activity. Examples include methyl prednisolone, prednisolone, dexamethasone, fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid *S*-fluoromethyl ester, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate), 6α,9α-difluoro-11β-hydroxy-16α-methyl-3-oxo-17α-propionyloxy- androsta-1,4-diene-17β-carbothioic acid *S*-(2-oxo-tetrahydro-furan-3S-yl) ester, 6α,9α-difluoro-11β-hydroxy-16α-methyl-3-oxo-17α-(2,2,3,3- tetramethycyclo propylcarbonyl)oxy-androsta-1,4-diene-17β-carbothioic acid S-cyanomethyl ester and 6α,9α-difluoro-11β-hydroxy-16α-methyl-17α-(1-methycyclopropylcarbonyl)oxy-3-oxo-androsta-1,4-diene-17β-carbothioic acid *S*-fluoromethyl ester, beclomethasone esters (for example the 17-propionate ester or the 17,21-dipropionate ester), budesonide, flunisolide, mometasone esters (for example mometasone furoate), triamcinolone acetonide, rofleponide, ciclesonide (16α,17-[[(*R*)-cyclohexylmethylene]bis(oxy)]-11β,21-dihydroxy-pregna-1,4-diene-3,20-dione), butixocort propionate, RPR-106541, and ST-126. Corticosteroids of particular interest may include fluticasone propionate, 6α,9α-difluoro-11β-hydroxy-16α-methyl-17α-[(4-methyl-1,3-thiazole-5-carbonyl)oxy]-3-oxo-androsta-1,4-diene-17β-carbothioic acid S-fluoromethyl ester, 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester, 6α,9α-difluoro-11β-hydroxy-16α-methyl-3-oxo-17α-(2,2,3,3-tetramethycyclopropylcarbonyl)oxy-androsta-1,4-diene-17β-carbothioic acid S-cyano methylester, 6α,9α-difluoro-11β-hydroxy-16α-methyl-17α-(1-methycyclopropylcarbonyl) oxy-3-oxo-androsta-1,4-diene-17β-carbothioic acid *S*-fluoromethyl ester and mometasone furoate. In one embodiment the corticosteroid is 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate) or mometasone furoate.

There is provided, in a further embodiment, a combination comprising a compound of formula (I) or a pharmaceutically acceptable salt thereof, together with a corticosteroid, such as fluticasone propionate or 6α,9α-difluoro-17α-[(2-furanylcarbonyl)oxy]-11β-hydroxy-16α-methyl-3-oxo-androsta-1,4-diene-17β-carbothioic acid *S*-fluoromethyl ester (fluticasone furoate) or mometasone furoate. Such combinations may be of particular interest for intranasal administration.

Non-steroidal compounds having glucocorticoid agonism that may possess selectivity for transrepression over transactivation and that may be useful in combination therapy include those covered in the following patent application and patents: WO03/082827, WO98/54159, WO04/005229, WO04/009017, WO04/018429, WO03/104195, WO03/082787, WO03/082280, WO03/059899, WO3/101932, WO2/02565, WO01/16128, WO00/66590, WO03/086294, WO04/026248, WO03/061651, WO03/08277, WO06/000401, WO06/000398 and WO06/015870.

In one embodiment, there is provided a combination comprising a compound of formula (I) or a pharmaceutically acceptable salt thereof and a glucocorticoid agonist.

Anti-inflammatory agents include non-steroidal anti-inflammatory drugs (NSAID's).

NSAID's include sodium cromoglycate, nedocromil sodium, phosphodiesterase (PDE) inhibitors (e.g. theophylline, PDE4 inhibitors or mixed PDE3/PDE4 inhibitors), leukotriene antagonists, inhibitors of leukotriene synthesis (eg. montelukast), iNOS (inducible nitric oxide synthase) inhibitors (e.g. oral iNOS inhibitors), IKK antagonists, tryptase and elastase inhibitors, beta-2 integrin antagonists and adenosine receptor agonists or antagonists (e.g. adenosine 2a agonists), cytokine antagonists (e.g. chemokine antagonists, such as a CCR1, CCR2, CCR3, CCR4, or CCR8 antagonists) or inhibitors of cytokine synthesis, or 5-lipoxygenase inhibitors. iNOS inhibitors include those disclosed in WO93/13055, WO98/30537, WO02/50021, WO95/34534 and WO99/62875.

In one embodiment there is provided the use of the compounds of formula (I) or a pharmaceutically acceptable salt thereof in combination with a phosphodiesterase 4 (PDE4) inhibitor. The PDE4-specific inhibitor useful in this embodiment may be any compound that is known to inhibit the PDE4 enzyme or which is discovered to act as a PDE4 inhibitor, and which are only PDE4 inhibitors, not compounds which inhibit other members of the PDE family, such as PDE3 and PDE5, as well as PDE4.

Compounds which may be of interest include cis-4-cyano-4-(3-cyclopentyloxy-4-methoxyphenyl)cyclohexan-1-carboxylic acid, 2-carbomethoxy-4-cyano-4-(3-cyclopropylmethoxy-4-difluoromethoxyphenyl)cyclohexan-1-one and *cis*-[4-cyano-4-(3-cyclopropylmethoxy-4-difluoromethoxyphenyl)cyclohexan-l-ol]. Also, *cis*-4-cyano-4-[3-(cyclopentyloxy)-4-methoxyphenyl]cyclohexane-1-carboxylic acid (also known as cilomilast) and its salts, esters, pro-drugs or physical forms, which is described in U.S. patent 5,552,438 issued 03 September, 1996.

Other PDE4 inhibitors include AWD-12-281 from Elbion (Hofgen, N. et al., 15th EFMC Int. Symp. Med. Chem., (Sept 6-10, Edinburgh) 1998, Abst. P. 98; CAS reference No. 247584020-9); a 9-benzyladenine derivative nominated NCS-613 (INSERM); D-4418 from Chiroscience and Schering-Plough; a benzodiazepine PDE4 inhibitor identified as CI-1018 (PD-168787) and attributed to Pfizer; a benzodioxole derivative disclosed by Kyowa Hakko in WO99/16766; K-34 from Kyowa Hakko; V-11294A from Napp (Landells, L.J. et al., Eur. Resp. J. [Ann. Cong. Eur. Resp. Soc. (Sept 19-23, Geneva) 1998] 1998, 12 (Suppl. 28): Abst P2393); roflumilast (CAS reference No 162401-32-3) and a pthalazinone (WO99/47505) from Byk-Gulden; Pumafentrine, (-)-p-[(4*a*R*,10*b*S*)-9-ethoxy-1,2,3,4,4a,10b-hexahydro-8-methoxy-2-methylbenzo[*c*][1,6]naphthyridin-6-yl]-*N,N-*diisopropylbenzamide which is a mixed PDE3/PDE4 inhibitor which has been prepared and published on by Byk-Gulden, now Altana; arofylline under development by Almirall-Prodesfarma; VM554/UM565 from Vernalis; or T-440 (Tanabe Seiyaku; Fuji, K. et al., J. Pharmacol. Exp. Ther., 284(1):162, (1998)), and T2585.

Further compounds which may be of interest are disclosed in the published international patent applications WO04/024728 (Glaxo Group Ltd), WO04/056823 (Glaxo Group Ltd) and WO04/103998(Glaxo Group Ltd).

In another embodiment, there is provided a combination comprising a compound of formula (I) or a pharmaceutically acceptable salt thereof and an anticholinergic agent.

Anticholinergic agents are those compounds that act as antagonists at the muscarinic receptors, in particular those compounds which are antagonists of the M₁ or M₃ receptors, dual antagonists of the M₁/M₃ or M₂/M₃. receptors or pan-antagonists of the M₁/M₂/M₃ receptors. Exemplary compounds for administration via inhalation include ipratropium (for example, as the bromide, CAS 22254-24-6, sold under the name Atrovent), oxitropium (for example, as the bromide, CAS 30286-75-0) and tiotropium (for example, as the bromide, CAS 136310-93-5, sold under the name Spiriva). Also of interest are revatropate (for example, as the hydrobromide, CAS 262586-79-8) and LAS-34273 which is disclosed in WO01/04118. Exemplary compounds for oral administration include pirenzepine (for example, CAS 28797-61-7), darifenacin (for example, CAS 133099-04-4, or CAS 133099-07-7 for the hydrobromide sold under the name Enablex), oxybutynin (for example, CAS 5633-20-5, sold under the name Ditropan), terodiline (for example, CAS 15793-40-5), tolterodine (for example, CAS 124937-51-5, or CAS 124937-52-6 for the tartrate, sold under the name Detrol), otilonium (for example, as the bromide, CAS 26095-59-0, sold under the name Spasmomen), trospium chloride (for example, CAS 10405-02-4) and solifenacin (for example, CAS 242478-37-1, or CAS 242478-38-2, or the succinate also known as YM-905 and sold under the name Vesicare).

Other anticholinergic agents include compounds of formula (XXI), which are disclosed in US patent application 60/487981: 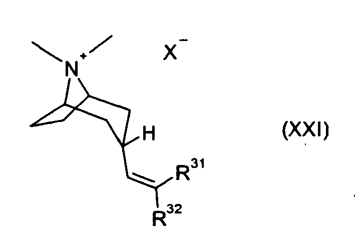
in which a particular orientation of the alkyl chain attached to the tropane ring is endo;
R³¹ and R³² are, independently, selected from the group consisting of straight or branched chain lower alkyl groups having e.g. from 1 to 6 carbon atoms, cycloalkyl groups having from 5 to 6 carbon atoms, cycloalkyl-alkyl having 6 to 10 carbon atoms, 2-thienyl, 2-pyridyl, phenyl, phenyl substituted with an alkyl group having not in excess of 4 carbon atoms and phenyl substituted with an alkoxy group having not in excess of 4 carbon atoms;
X⁻ represents an anion associated with the positive charge of the N atom. X- may be, but is not limited to chloride, bromide, iodide, sulfate, benzene sulfonate, and toluene sulfonate, including, for example:
   (3-*endo*)-3-(2,2-di-2-thienylethenyl)-8,8-dimethyl-8-azoniabicyclo[3.2.1]octane bromide;
   (3-*endo*)-3-(2,2-diphenylethenyl)-8,8-dimethyl-8-azoniabicyclo[3.2.1]octane bromide;
   (3-*endo*)-3-(2,2-diphenylethenyl)-8,8-dimethyl-8-azoniabicyclo[3.2.1]octane 4-methyl benzenesulfonate;
   (3-*endo*)-8,8-dimethyl-3-[2-phenyl-2-(2-thienyl)ethenyl]-8-azoniabicyclo[3.2.1]octane bromide; and/or
   (3-*endo*)-8,8-dimethyl-3-[2-phenyl-2-(2-pyridinyl)etheny]-8-azoniabicyclo[3.2.1]octane bromide.

Further anticholinergic agents include compounds of formula (XXII) or (XXIII), which are disclosed in US patent application 60/511009: 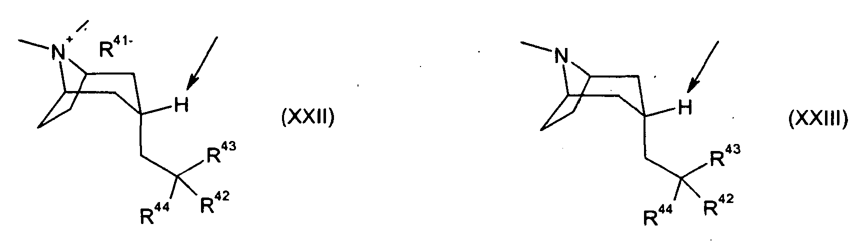 wherein:
the H atom indicated is in the exo position;
R⁴¹⁻ represents an anion associated with the positive charge of the N atom. R1⁻ may be but is not limited to chloride, bromide, iodide, sulfate, benzene sulfonate and toluene sulfonate;
R⁴² and R⁴³ are independently selected from the group consisting of straight or branched chain lower alkyl groups (having for example from 1 to 6 carbon atoms), cycloalkyl groups (having from 5 to 6 carbon atoms), cycloalkyl-alkyl (having 6 to 10 carbon atoms),
heterocycloalkyl (having 5 to 6 carbon atoms) and N or O as the heteroatom,
heterocycloalkyl-alkyl (having 6 to 10 carbon atoms) and N or O as the heteroatom, aryl,
optionally substituted aryl, heteroaryl, and optionally substituted heteroaryl;
R⁴⁴ is selected from the group consisting of (C₁-C₆)alkyl, (C₃-C₁₂)cycloalkyl, (C₃-C₇)heterocycloalkyl, (C₁-C₆)alkyl(C₃-C₁₂)cycloalkyl, (C₁-C₆)alkyl(C₃-C₇)heterocycloalkyl, aryl, heteroaryl, (C₁-C₆)alkyl-aryl, (C₁-C₆)alkyl-heteroaryl, -OR⁴⁵, -CH₂OR⁴⁵, -CH₂OH, -CN, -CF₃, -CH₂O(CO)R⁴⁶, -CO₂R⁴⁷, -CH₂NH₂, -CH₂N(R⁴⁷)SO₂R⁴⁵, -SO₂N(R⁴⁷)(R⁴⁸), - CON(R⁴⁷)(R⁴⁸), -CH₂N(R⁴⁸)CO(R⁴⁶), -CH₂N(R⁴⁸)SO₂(R⁴⁶), -CH₂N(R⁴⁸)CO₂(R⁴⁵), - CH₂N(R⁴⁸)CONH(R⁴⁷);
R⁴⁵ is selected from the group consisting of (C₁-C₆)alkyl, (C₁-C₆)alkyl(C₃-C₁₂)cycloalkyl, (C₁-C₆)alkyl(C₃-C₇)heterocycloalkyl, (C₁-C₆)alkyl-aryl, (C₁-C₆)alkyl-heteroaryl;
R⁴⁶ is selected from the group consisting of (C₁-C₆)alkyl, (C₃-C₁₂)cycloalkyl, (C₃-C₆)heterocycloalkyl, (C₁-C₆)alkyl(C₃-C₁₂)cycloalkyl, (C₁-C₆)alkyl(C₃-C₇)heterocycloalkyl, aryl, heteroaryl, (C₁-C₆)alkyl-aryl, (C₁-C₆)alkyl-heteroaryl;
R⁴⁷ and R⁴⁸ are, independently, selected from the group consisting of H, (C₁-C₆)alkyl, (C₃-C₁₂)cycloalkyl, (C₃-C₇)heterocycloalkyl, (C₁-C₆)alkyl(C₃-C₁₂)cyclalkyl, (C₁-C₆)alkyl(C₃-C₇)heterocycloalkyl, (C₁-C₆)alkyl-aryl, and (C₁-C₆)alkyl-heteroaryl, including, for example:
   (Endo)-3-(2-methoxy-2,2-di-thiophen-2-yl-ethyl)-8,8-dimethyl-8-azonia-bicyclo[3.2.1]octane iodide;
   3-((Endo)-8-methyl-8-aza-bicyclo[3.2.1]oct-3-yl)-2,2-diphenyl-propionitrile;
   (Endo)-8-methyl-3-(2,2,2-triphenyl-ethyl)-8-aza-bicyclo[3.2.1]octane;
   3-((Endo)-8-methyl-8-aza-bicydo[3.2.1]oct-3-yl)-2,2-diphenyl-propionamide;
   3-((Endo)-8-methyl-8-aza-bicyclo[3.2.1]oct-3-yl)-2,2-diphenyl-propionic acid;
   (Endo)-3-(2-cyano-2,2-diphenyl-ethyl)-8,8-dimethyl-8-azonia-bicyclo[3.2.1]octane iodide;
   (Endo)-3-(2-cyano-2,2-diphenyl-ethyl)-8,8-dimethyl-8-azonia-bicyclo[3.2.1]octane bromide;
   3-((Endo)-8-methyl-8-aza-bicyclo[3.2.1]oct-3-yl)-2,2-diphenyl-propan-1-ol;
   *N*-Benzyl-3-((endo)-8-methyl-8-aza-bicyclo[3.2.1]oct-3-yl)-2,2-diphenyl-propionamide;
   (Endo)-3-(2-carbamoyl-2,2-diphenyl-ethyl)-8,8-dimethyl-8-azonia-bicyclo[3.2.1]octane iodide;
   1-Benzyl-3-[3-((endo)-8-methyl-8-aza-bicyclo[3.2.1]oct-3-yl)-2,2-diphenyl-propyl]-urea;
   1-Ethyl-3-[3-((endo)-8-methyl-8-aza-bicyclo[3.2.1]oct-3-yl)-2,2-diphenyl-propyl]-urea;
   *N*-[3-((Endo)-8-methyl-8-aza-bicyclo[3.2.1]oct-3-yl)-2,2-diphenyl-propyl]-acetamide;
   *N*-[3-((Endo)-8-methyl-8-aza-bicyclo[3.2.1]oct-3-yl)-2,2-diphenyl-propyl]-benzamide;
   3-((Endo)-8-methyl-8-aza-bicyclo[3.2.1]oct-3-yl)-2,2-di-thiophen-2-yl-propionitrile;
   (Endo)-3-(2-cyano-2,2-di-thiophen-2-yl-ethyl)-8,8-dimethyl-8-azonia-bicyclo[3.2.1]octane iodide;
   *N*-[3-((Endo)-8-methyl-8-aza-bicyclo[3.2.1]oct-3-yl)-2,2-diphenyl-propyl]-benzene sulfonamide;
   [3-((Endo)-8-methyl-8-aza-bicyclo[3.2.1]oct-3-yl)-2,2-diphenyl-propyl]-urea;
   *N*-[3-((Endo)-8-methyl-8-aza-bicyclo[3.2.1]oct-3-yl)-2,2-diphenyl-propyl]-methane sulfonamide; and/or
   (Endo)-3-{2,2-diphenyl-3-[(1-phenyl-methanoyl)-amino]-propyl}-8,8-dimethyl-8-azonia-bicyclo[3.2.1]octane bromide.

Particular anticholinergic compounds that may be of use include:
(Endo)-3-(2-methoxy-2,2-di-thiophen-2-yl-ethyl)-8,8-dimethyl-8-azonia-bicyclo[3.2.1] octane iodide;
(Endo)-3-(2-cyano-2,2-diphenyl-ethyl)-8,8-dimethyl-8-azonia-bicyclo[3.2.1]octane iodide;
(Endo)-3-(2-cyano-2,2-diphenyl-ethyl)-8,8-dimethyl-8-azonia-bicyclo[3.2.1]octane bromide;
(Endo)-3-(2-carbamoyl-2,2-diphenyl-ethyl)-8,8-dimethyl-8-azonia-bicyclo[3.2.]octane iodide;
(Endo)-3-(2-cyano-2,2-di-thiophen-2-yl-ethyl)-8,8-dimethyl-8-azonia-bicyclo[3.2.1]octane iodide; and/or
(Endo)-3-{2,2-diphenyl-3-[(1-phenyl-methanoyl)-amino]-propyl}-8,8-dimethyl-8-azonia-bicyclo[3.2.1]octane bromide.

The combinations referred to above may conveniently be presented for use in the form of a pharmaceutical composition and thus pharmaceutical compositions comprising a combination as defined above optionally together with a pharmaceutically acceptable carriers and/or excipients.

The individual compounds of such combinations may be administered either sequentially in separate pharmaceutical compositions as well as simultaneously in combined pharmaceutical compositions. Additional therapeutically active ingredients may be suspended in the composition together with a compound of formula (I). Appropriate doses of known therapeutic agents will be readily appreciated by those skilled in the art.

Compounds of formula (I) may be prepared by the methods described below or by similar methods. Thus the following Intermediates and Examples illustrate the preparation of the compounds of formula (I), and are not to be considered as limiting the scope of the disclosure in any way.

### GENERAL EXPERIMENTAL

### Abbreviations

- aq.:: Aqueous
- 9-BBN:: 9-Borabicyclononane
- BBr₃:: Boron tribromide
- BOC (Boc):: *tert*-butoxycarbonyl
- Cs₂CO₃:: Cesium carbonate
- CV:: Column volumes
- DCM:: Dichloromethane
- DIPEA:: *N,N*-Diisopropylethylamine
- DMF:: *N,N*-Dimethylformamide
- DMSO:: Dimethylsulfoxide
- EtOAc:: Ethyl acetate
- EtOH:: Ethanol
- HCl:: Hydrogen chloride
- HPLC:: High Performance Liquid Chromotography
- K₂CO₃:: Potassium chloride
- LCMS:: Liquid Chromatography - Mass Spectroscopy
- mbar:: millibar (pressure)
- MDAP:: Mass-Directed Autopreparative
- MeCN:: Acetonitrile
- MeOH:: Methanol
- MgSO₄:: Magnesium Sulfate
- MIBK:: Methyl isobutyl ketone
- MsCl:: Mesyl chloride
- NaHCO₃:: Sodium hydrogen carbonate
- NaOH:: Sodium hydroxide
- Na₂SO₄:: Sodium sulfate
- NEt₃:: Triethylamine
- NMP:: *N*-methyl pyrrolidinone
- NMR:: Nuclear Magnetic Resonance
- PTFE:: Polytetrafluoroethylene
- Pd/C:: Palladium on activated carbon
- RT:: Retention time
- SiO₂:: Silica
- TBTU:: *O*-1*H*-benzotriazole-1-yl-*N,N,N',N'*-tetramethyluronium tetrafluoroborate
- TFA:: Trifluoroacetic acid
- THF:: Tetrahydrofuran
- TLC:: Thin Layer Chromotography
- TBME:: *tert*-Butylmethyl ether
- h:: Hours
- min:: Minutes

### General Procedures

Flash silica gel refers to Merck Art No. 9385; silica gel refers to Merck Art No. 7734.
SCX cartridges are Ion Exchange SPE columns where the stationary phase is polymeric benzene sulfonic acid. These are used to isolate amines.
SCX2 cartridges are Ion Exchange SPE columns where the stationary phase is polymeric propylsulfonic acid. These are used to isolate amines.

LCMS was conducted on a Supelcosil LCABZ+PLUS column (3.3 cm × 4.6 mm ID) eluting with 0.1 % formic acid and 0.01 M ammonium acetate in water (solvent A) and 0.05% formic acid 5% water in MeCN (solvent B), using the following elution gradient 0.0 - 7 min 0% B, 0.7 - 4.2 min 100% B, 4.2 - 5.3 min 0% B, 5.3 - 5.5min 0% B at a flow rate of 3 m/min⁻¹. The mass spectra were recorded on a Fisons VG Platform spectrometer using electrospray positive and negative mode (ES+ve and ES-ve).

The Flashmaster II is an automated multi-user flash chromatography system, available from Argonaut Technologies Ltd, which utilises disposable, normal phase, SPE cartridges (2 g to 100 g). It provides quaternary on-line solvent mixing to enable gradient methods to be run. Samples are queued using the multi-functional open access software, which manages solvents, flow-rates, gradient profile and collection conditions. The system is equipped with a Knauer variable wavelength UV-detector and two Gilson FC204 fraction-collectors enabling automated peak cutting, collection and tracking.

Mass directed autopreparative (MDAP) HPLC was conducted on a Waters FractionLynx system comprising of a Waters 600 pump with extended pump heads, Waters 2700 autosampler, Waters 996 diode array and Gilson 202 fraction collector on a 10 cm × 2.54 cm internal diameter ABZ+ column, eluting with 0.1 % formic acid in water (solvent A) and 0.1 % formic acid in MeCN (solvent B), using as appropriate elution gradient over 15 min at a flow rate of 20 mlmin⁻¹ and detecting at 200 - 320 nm at room temperature. Mass spectra were recorded on Micromass ZMD mass spectrometer using electro spray positive and negative mode, alternate scans. The software used was MassLynx 3.5 with OpenLynx and FractionLynx options.

The ¹H NMR spectra were recorded on a Bruker AV400 operating at 400 MHz. Standard deuterated solvents were used. Typically, the NMR is taken with a deuterium lock for reference. Optionally, tetramethylsilane is used as internal standard.

Reactions are routinely monitored by methods well known to those skilled in the art, such as TLC, LCMS and/or HPLC. Such methods are used to assess whether a reaction has gone to completion, and reaction times may be varied accordingly.

The XRPD method which is employed to analyse crystalline forms of compounds is as follows:

| Manufacturer | PANalytical - The Netherlands |
|---|---|
| Instrument | X'Pert Pro |
| Diffractometer Type | DY1850 |
| Tube anode | Cu |
| K-Alpha1 wavelength (A°) | 1.54056 |
| K-Alpha2 wavelength (A°) | 1.54439 |
| Ration Alpha 1:2 | 0.50000 |
| Divergence slit | Prog.Div.Slit |
| Receiving slit | Prog.Rec.Slit |
| Generator voltage (kV) | 40 |
| Tube Current (mA) | 45 |
| Detector | X'celerator |
| Data Angle range (°2θ) | 2.000-39.997 |
| Scan type | Continuous |
| Scan step size | 0.0167 |
| Scan step time (seconds) | 31.75 |
| Sample preparation | Flush Silicon wafer |

XRPD analysis was performed on a PANalytical X'Pert Pro X-ray powder diffractometer, model X' Pert Pro PW3040/60, serial number DY1850 using an X'Celerator detector. The acquisition conditions were: radiation: Cu K, generator tension: 40 kV, generator current: 45 mA, start angle: 2.000°2θ, end angle: 39.997 °2θ, step size: 0.0167, time per step: 31.75 seconds. The sample was prepared using flush Silicon wafer.

Differential Scanning Calorimetry (DSC) was performed on a TA instruments Q1000 Differential Scanning Calorimeter equipped with a refrigerated cooling system. Slight variations in the observed peaks are expected based on the specific instrument and pan configuration employed, the analyst's sample preparation technique, and the sample size. Some margin of error is present in the peak assignment reported above. The margin of error is approximately ±5 °C for the peak maximum and ±10 J/g for the heat of fusion.

Compounds were named using ACD/Name PRO 6.02 chemical naming software Advanced Chemistry Developments inc.; Toronto, Ontario, M5H2L3, Canada.

### Intermediates

### Intermediate 1

### 2-{2-[4-[(4-Chlorophenyl)methyl]-1-oxo-2(1H)-phthalazinyl]ethyl}-1H-isoindole-1,3(2H)-dione

Sodium hydride (0.19 g, 4.75 mmol) was suspended in dry DMF (20 ml) under a nitrogen atmosphere, stirred and cooled in an ice-water bath. A solution of 4-[(4-chlorophenyl)methyl]-1(2*H*)-phthalazinone (for example, as disclosed in US patent 1,377,231, see Example 9, Step 1) (1.055 g, 3.90 mmol) in DMF (8 ml) was added slowly, and stirring was continued in the cooling bath for 30 min. The cooling bath was removed and the mixture was stirred at room temperature for 30 min. A solution of N-(2-bromoethyl)phthalimide (commercially available, for example, from Aldrich) (1.12 g, 4.42 mmol) in DMF (12 ml) was added slowly, and the resulting mixture was stirred under a nitrogen atmosphere overnight. The mixture was concentrated *in vacuo*, and the residue was partitioned between water and EtOAc. The layers were separated, and the organic layer was dried (MgSO₄) and concentrated *in vacuo*. The crude product was purified by chromatography on silica (Flashmaster II, 100 g, gradient of 0-50% EtOAc-DCM over 60 min). The appropriate fractions were combined and concentrated *in vacuo* to afford *the title compound* (1.192 g). LCMS RT = 3.44 min, ES+ve *m*/*z* 444/446 [M+H]⁺.

### Intermediate 2

### 2-(2-Aminoethyl)-4-[(4-chlorophenyl)methyl]-1(2H)-phthalazinone

**2-{2-[4-[(4-Chlorophenyl)methyl]-1-oxo-2(1*H*)-phthalazinyl]ethyl}-1*H*-isoindole-1,3(2*H*)-**dione (for example, as prepared for Intermediate 1) (1.192 g, 2.69 mmol) and hydrazine hydrate (about 65% solution, 0.4 ml, 8.02 mmol) were stirred in EtOH (30 ml), and heated at reflux for 4 h. The resulting thick suspension was cooled and the solid removed by filtration, washing. The filtrate was concentrated *in vacuo* to afford *the title compound* (0.66 g). LCMS RT = 2.34 min, ES+ve *m*/*z* 314/316 [M+H]⁺.

### Intermediate 3

### 1,1-Dimethylethyl (2R)-2-{[4-[(4-chlorophenyl)methyl]-1-oxo-2(1H)-phthalazinyl] methyl}-1-pyrrolidinecarboxylate

To a solution of triphenylphosphine (1.86 g, 7.09 mmol) in dry THF (6 ml) was added diisopropyl azodicarboxylate (1.12 ml, 5.69 mmol) at -15 °C. The resulting pale yellow thick suspension was stirred at -15 °C for 2 min. To aid stirring more dry THF (2 ml) was added. The reaction mixture was then treated with a suspension of 4-[(4-chlorophenyl)methyl]-1(2*H*)-phthalazinone, (for example, as disclosed in US patent 1,377,231, Example 9, Step 1) (0.571 g, 2.11 mmol) and *N*-*tert*-butoxycarbonyl-D-prolinol (commercially available, for example, from Fluka), (0.650 g, 3.23 mmol) in dry THF (10 ml) at -15 °C. The reaction mixture was allowed to warm to room temperature and stirred at 20 °C for 23 h. MeOH (20 ml) was then added and the solvents were removed *in vacuo.* The resultant residue was purified by Flashmaster II chromatography (70 g silica cartridge) eluted with 0 - 50% EtOAc-cyclohexane gradient over 40 min. The solvents were removed *in vacuo* to afford the *title compound* as a dark brown oil(1.05 g). LCMS RT = 3.71 min.

### Intermediate 4

### 4-[(4-Chlorophenyl)methyl]-2-[(2R)-2-pyrrolidinylmethyl]-1(2H)-phthalazinone

To a solution of 1,1-dimethylethyl (2*R*)-2-{[4-[(4-chlorophenyl)methyl]-1-oxo-2(1*H*)-phthalazinyl]methyl}-1-pyrrolidinecarboxylate (for example, as prepared for Intermediate 3) (1.05 g, 2.31 mmol) in dry dioxane (12 ml) was added a solution of HCl in 1,4-dioxane (4.0 M, 6 ml). The solution was stirred at 20 °C for 2 h. TFA (1 ml) was added to the mixture and stirred for 30 min, then more TFA (3 × approximately 1 ml) was added at 10 minute intervals until deprotection was completed. The solvent was removed *in vacuo* and the residue applied onto an SCX cartridge (20 g), washed with MeOH (x 2) and then eluted with 10% aq. ammonia in MeOH (2 × 50 ml). The solvents were removed *in vacuo* and the resultant residue purified by Flashmaster II chromatography (50 g silica cartridge) eluted with 0 - 30% MeOH + 1 % NEt₃ - DCM gradient over 40 min to afford the *title compound* as a dark brown foam (0.351 g). LCMS RT = 2.45 min.

### Intermediate 5

### 1,1-Dimethylethyl (2S)-2-{[4-[(4-chlorophenyl)methyl]-1-oxo-2(1H)-phthalazinyl] methyl}-1-pyrrolidinecarboxylate

A solution of di-*tert*-butyl azodicarboxylate (1.15 g, 5 mmol) in THF (5 ml) was added to a stirred solution of triphenylphosphine (1.8 g, 7 mmol) in THF (5 ml), cooled to -15 °C. The resulting thick suspension was treated with a suspension of 4-[(4-chlorophenyl)methyl]-1(2*H*)-phthalazinone (for example, as disclosed in US patent 1,377,231, see Example 9, Step 1) (0.558 g, 2 mmol) and *N*-*tert*-butoxycarbonyl-*D*-prolinol (commercially available from, for example, Fluka) (0.64 g, 3.2 mmol) in THF (5 ml), and then further THF (15 ml) was added to the stirred mixture. Stirring was continued under a nitrogen atmosphere overnight, allowing the temperature to rise to room temperature. The reaction mixture was concentrated *in vacuo*, and the residue was purified by chromatography on silica (Flashmaster II, 100 g, gradient of 0 - 50% EtOAc-cyclohexane over 60 min). The appropriate fractions were combined and concentrated *in vacuo* to afford *the title compound* (0.738 g). LCMS RT = 3.71 min, ES+ve *m*/*z* 454/456 [M+H]⁺.

### Intermediate 6

### 4-[(4-Chlorophenyl)methyl]-2-[(2S)-2-pyrrolidinylmethyl]-1(2H)-phthalazinone

A solution of 1,1-dimethylethyl (2*S*)-2-{[4-[(4-chlorophenyl)methyl]-1-oxo-2(1*H*)-phthalazinyl]methyl}-1-pyrrolidinecarboxylate, (for example, as prepared for Intermediate 5) (738 mg, 1.6 mmol) in dioxane (5 ml) was treated with a solution of HCl in dioxane (4 M, 5 ml), and stirred at room temperature under a nitrogen atmosphere for 1 h. Further HCl in dioxane (4 M, 2 ml) was added, and stirring continued for 40 min more. The reaction mixture was concentrated *in vacuo*. The residue was applied to an SCX catridge (50 g), eluting with MeOH, and then a solution of 10% aq. ammonia in MeOH. The appropriate fractions were combined and concentrated *in vacuo* to afford *the title compound* (0.555 g). LCMS RT = 2.45 min, ES+ve *m*/*z* 354/356 [M+H]⁺.

### Intermediate 7

### (3Z)-3-{[4-(Methyloxy)phenyl]methylidene}-2-benzofuran-1(3H)-one

A mixture of 4-methoxyphenyl acetic acid (commercially available, for example, from Aldrich) (13.948 g, 83.94 mmol), phthalic anhydride (commercially available, for example, from Aldrich) (12.43 g, 83.94 mmol) and sodium acetate (276 mg, 3.4 mmol) was heated in a Dean-Stark apparatus under nitrogen to 240 °C for 2.5 h, allowed to cool to room temperature overnight and then re-heated for 7 h. The mixture was allowed to cool under nitrogen and before it solidified, EtOH was added which caused rapid crystallisation. The crystals were collected by filtration, washed with a little EtOH, and dried to give *the tittle compound* (15.8 g, 75%). LCMS RT = 3.45 min, ES+ve *m*/*z* 253 (M+H)⁺.

### Intermediate 8

### 4-{[4-(Methyloxy)phenyl]methyl}-1(2H)-phthalazinone

A mixture of (3*Z*)-3-{[4-(methyloxy)phenyl]methylidene}-2-benzofuran-1(3*H*)-one (for example, as prepared for Intermediate 7) (15.8 g, 62.7 mmol) and hydrazine sulfate (9 g, 69 mmol) was treated with NaOH solution (2 M, 70 ml), water (70 ml) and EtOH (20 ml) and the mixture was heated to 95 °C for 18 h. More hydrazine sulfate (0.9 g, 6.9 mmol) and NaOH solution (2 M, 10 ml) were added and the mixture heated for an additional 7 h and then allowed to cool to room temperature over the weekend. The reaction mixture was diluted with water (750 ml) and the solid was collected by filtration. The solid was washed with water, dried *in vacuo* to give *the title compound* (15.25 g, 91%) LCMS RT = 2.76 min, ES+ve *m*/*z* 267 (M+H)⁺.

### Intermediate 9

### 1,1-Dimethylethyl (2R)-2-{[4-{[4-(methyloxy)phenyl]methyl}-1-oxo-2(1H)-phthalazinyl]methyl}-1-pyrrolidinecarboxylate

A mixture of 4-{[4-(methyloxy)phenyl]methyl}-1(2*H*)-phthalazinone (for example, as prepared for Intermediate 8) (1.46 g, 5.5 mmol), N-Boc-D-prolinol (commercially available, for example, from Fluka) (1.08 g, 5.36 mmol) in THF (20 ml) at 20 °C was added to a mixture of triphenylphosphine (2.81 g, 10.7 mmol) and di-*tert*-butyl azodicarboxylate (1.85 g, 8.04 mmol) in THF (20 ml) at - 15 °C. The mixture was stirred under nitrogen whilst warming to room temperature overnight. The reaction mixture was evaporated under reduced pressure and the residue was purified by chromatography (Flashmaster II, two silica 100 g cartridges) eluting with 0 to 100% EtOAc-cyclohexane over 60 min. Appropriate fractions were combined and evaporated under reduced pressure to give *the title compound* (2.19 g, 91%). LCMS RT = 3.49 min, ES+ve *m*/*z* 450 (M+H)⁺.

### Intermediate 10

### 4-{[4-(Methyloxy)phenyl]methyl}-2-[(2R)-2-pyrrolidinylmethyl]-1(2H)-phthalazinone

A solution of 1,1-dimethylethyl (2R)-2-{[4-{[4-(methyloxy)phenyl]methyl}-1-oxo-2(1*H*)-phthalazinyl]methyl}-1-pyrrolidinecarboxylate (for example, as prepared for Intermediate 9) (1.4 g, 3.1 mmol) in DCM (20 ml) was treated with TFA (5 ml) at room temperature. The mixture was stirred for 0.45 min and then concentrated under reduced pressure. The residue was purified on an SCX-2 cartridge (20 g) eluting first with MeOH and then with 10% aq. ammonia in MeOH. The ammoniacal fractions were combined and concentrated under reduced pressure to give the *title compound* (1.08 g). LCMS RT = 2.25 min, ES+ve *m*/*z* 350 (M+H)⁺.

### Intermediate 11

### 1,1-Dimethylethyl (2R)-2-[(4-chloro-1-oxo-2(1H)-phthalazinyl)methyl]-1-pyrrolidinecarboxylate

To a solution of triphenylphosphine (3.06 g, 11.6 mmol) in anhydrous THF (26 ml) at -10°C was added diisopropyl azodicarboxylate (1.95 ml, 9.9 mmol). The resulting suspension was stirred at -10 to -5 °C for 10 min. To the suspension was added a suspension of 4-chloro-1(2*H*)-phthalazinone (commercially available, for example, from Acros) (0.8 g, 4.43 mmol), and *N*-Boc-*D*-prolinol (commercially available, for example, from Fluka) (1.15 g, 5.7 mmol), in THF (27 ml). The suspension was allowed to warm to 20 °C and stirred for 1.5 h. The suspension was quenched with MeOH (10 ml) and the solvent removed *in vacuo.* The residue (5.63 g) was purified by MDAP HPLC (100 g silica cartridge) using an EtOAc-cyclohexane gradient to give *the title compound* (2.213 g). LCMS RT = 3.30 min, ES+ve *m*/*z* 364 and 366 (M+H)⁺.

### Intermediate 12

### 4-Chloro-2-[(2R)-2-pyrrolidinylmethyl]-1(2H)-phthalazinone

To a solution of the crude 1,1-dimethylethyl (2*R*)-2-[(4-chloro-1-oxo-2(1*H*)-phthalazinyl)methyl]-1-pyrrolidinecarboxylate (for example, as prepared for Intermediate 11) (2.213 g, 6.1 mmol) in dioxane (10 ml) was added 4.0 M HCl in dioxane (10 ml). The mixture was stirred at 20 °C for 1.5 h. The solvent was removed *in vacuo* and the residue was applied to SCX cartridges (2 × 20 g). The cartridges were eluted with MeOH (2 CV) and then 10% 0.88 ammonia in MeOH (2 CV). The basic fractions were concentrated to give *the title compound* (0.816 g). RT = 1.79 min, ES+ve *m*/*z* 264 and 266(M+H)⁺

### Intermediate 13

### 4-Chloro-2-({(2R)-1-[4-(4-{[3-(hexahydro-1H-azepina1-yl)piopyl]oxy}phenyl)butyl]-2-pyrrolidinyl}methyl)-1(2H)-phthalazinone

To a slight suspension of 4-chloro-2-[(2*R*)-2-pyrrolidinylmethyl]-1(2*H*)-phthalazinone (for example, as prepared for Intermediate 12) (0.206 g, 0.78 mmol) in MeCN (3 ml) was added 4-(4-{[3-(hexahydro-1*H*-azepin-1-yl)propyl]oxy}phenyl)butyl methanesulfonate (for example, as prepared for Intermediate 35) (0.2 g, 0.52 mmol) and then sodium hydrogen carbonate (0.087 g, 1.04mmol). The suspension was heated to 80 °C for 5 days. The mixture was applied to SCX cartridge (20 g) and the cartridge washed with MeOH (2 CV). The cartridge was eluted with 10% 0.88 ammonia MeOH (2 CV). The basic fractions were concentrated *in vacuo* and the residue (0.27 g) was dissolved in MeOH (2 ml) and treated with TFA (0.12 ml, 1.61 mmol). The solution was purified by MDAP (sample split into 4 runs) to give a gum (0.173 g), which was applied to a SCX cartridge (5 g). The cartridge was washed with MeOH (2 CV) and then 10% 0.88 ammonia in MeOH (2 CV). The basic fractions were concentrated *in vacuo* to give the *title compound* (0.109 g). LCMS RT = 2.27 min, ES+ve *m*/*z* 551 (M+H)⁺ and 276 + 277 (M/2+H)⁺

### Intermediate 14

### 1,1-Dimethylethyl 4-hydroxyhexahydro-1H-azepine-1-carboxylate

To a solution of 1,1-dimethylethyl 4-oxohexahydro-1*H*-azepine-1-carboxylate (for example, as disclosed in patent application WO 2000/00203A1, see Example 1A) (2.146 g, 10.0 mmol) in MeOH (60 ml) was added sodium borohydride (commercially available, for example, from Aldrich) (0.426 g, 12.2 mmol), portionwise. The solution was stirred at 20 °C for 1.5 h. The solution was cautiously quenched with MeOH and water (1:1, 10 ml). The solvent was removed *in vacuo.* The residue was partitioned between EtOAc (100 ml) and saturated aq. sodium hydrogen carbonate (100 ml). The phases were separated and the organic extract washed with brine (50 ml). The organic extract was dried over MgSO₄. The solvent was removed *in vacuo* to give *the title compound* as a waxy solid (2.094 g, 97%). LCMS RT = 2.40 min, ES+ve *m*/*z* 216 (M+H)⁺.

### Intermediate 15

### 1,1-Dimethylethyl 4-[4-[(4-chlorophenyl)methyl]-1-oxo-2(1H)-phthalazinyl] hexahydro-1H-azepine-1-carboxylate

To a solution of triphenylphophine (2.77 g, 10.57 mmol) in anhydrous tetrahydrofuran (10 ml) at -15 °C was added diisopropyl azodicarboxylate (1.77 ml, 9 mmol). The resulting suspension was stirred at -15 °C to -20 °C for 5 min. To the mixture at -20 °C was added dropwise a solution 4-[(4-chlorophenyl)methyl]-1(2*H*)-phthalazinone (for example, as disclosed in US patent 1,377,231, see Example 9, Step 1) (0.887 g, 3.23 mmol) and 1,1-dimethylethyl 4-hydroxyhexahydro-1*H*-azepine-1-carboxylate (for example, as prepared for Intermediate 14) (1.0 g, 4.64 mmol) in anhydrous THF (20 ml). The suspension was allowed to gradually warm to 20 °C over 6.5 h. The mixture was quenched with MeOH (10 ml) and the solvent removed *in vacuo*. The residue (7.3 g) was purified by chromatography (Flashmaster II, two silica 100 g cartridges) eluting with 0 to 50% EtOAc-cyclohexane over 60 min to give *the title compound* (2.5 g). LCMS RT = 3.85 min, ES+ve *m*/*z* 468 (M+H)⁺.

### Intermediate 16

### 4-[(4-Chlorophenyl)methyl]-2-(hexahydro-1H-azepin-4-yl)-1(2H)-phthalazinone

To a solution of 1,1-dimethylethyl 4-[4-[(4-chlorophenyl)methyl]-1-oxo-2(1*H*)-phthalazinyl]hexahydro-1*H*-azepine-1-carboxylate (for example, as prepared for Intermediate 15) (2.5 g, 5.34 mmol) in 1,4-dioxane (10 ml) was added HCl in 1,4-dioxane (4.0 M, 10 ml). The solution was stirred at 20 °C for 1.5 h. The solvent was removed *in vacuo* and the residue was applied to a SCX cartridge (50 g, pre-washed with MeOH) and the cartridge washed with MeOH (2 CV). The cartridge was eluted with 10% 0.88 ammonia in MeOH (2 CV). The combined basic fractions were concentrated *in vacuo* to leave a pale yellow oil (1.179 g). The residue was further purified by chromatography (Flashmaster II, 70 g cartridge) eluting with 0 to 30% MeOH containing 1% NEt₃ - DCM to give *the title compound* (0.787 g). LCMS RT = 2.44 min, ES+ve *m*/*z* 368 (M+H)⁺.

Intermediate 16 may also be prepared by using TFA as an acid for deprotection.

### Intermediate 17

### 4-[(4-Chlorophenyl)methyl]-2-{1-[(4-hydroxyphenyl)methyl]hexahydro-1H-azepin-4-yl}-1(2H)-phthalazinone formate salt

A mixture of 4-[(4-chlorophenyl)methyl]-2-(hexahydro-1*H*-azepin-4-yl)-1(2*H*)-phthalazinone (for example, as prepared for Intermediate 16) (100 mg, 0.27 mmol) and 4-hydroxybenzaldehyde (commercially available, for example, from Aldrich) (33.2 mg, 0.27 mmol) in DCM (2 m!) and 1 drop of acetic acid was treated with sodium triacetoxyborohydride (230 mg, 1 mmol) and stirred under nitrogen overnight. More aldehyde (10 mg) and sodium triacetoxyborohydride (57.6 mg) were added. After 2.5 h the reaction mixture was partitioned between EtOAc and sodium bicarbonate solution. The organic phase was washed with sodium bicarbonate solution, dried over MgSO₄ and evaporated. The residue (90 mg) was dissolved in MeOH-DMSO (1:1; 0.8 ml) and purified by MDAP HPLC to give *the title compound* (19.2 mg) LCMS RT = 2.69 min, ES+ve *m*/*z* 474 (M+H)⁺. A second less pure fraction (34.7 mg) was also obtained which was used in the next stage without further purification.

### Intermediate 18

### 4-[(4-Chlorophenyl)methyl]-2-[1-({4-[(3-chloropropyl)oxy]phenyl}methyl)hexahydro-1H-azepin-4-yl]-1(2H)-phthalazinone

A solution of 4-[(4-chlorophenyl)methyl]-2-{1-[(4-hydroxyphenyl)methyl]hexahydro-1*H-*azepin-4-yl}-1(2*H*)-phthalazinone formate salt (for example, as prepared for Intermediate 17) (53.9 mg, 0.1 mmol) in 2-butanone (2 ml) was treated with potassium carbonate (15.7 mg) and 1-bromo-3-chloropropane (commercially available, for example, from Aldrich) (0.1 ml) and the mixture was heated under nitrogen to 80 °C overnight. More potassium carbonate (47.1 mg) and 1-bromo-3-chloropropane (0.056 ml) were added and the mixture was heated for another 4 days. More potassium carbonate (49 mg), 1-bromo-3-chloropropane (0.056 ml) and 2-butanone (2 ml) were added and the mixture was heated for an additional 4 h. The reaction mixture was then evaporated under reduced pressure and the residue was partitioned between EtOAc and sodium bicarbonate solution. The organic phase was washed with sodium bicarbonate solution, dried over MgSO₄ and evaporated. The residue was purified by chromatography (Flashmaster II, 5 g cartridge) eluting with 0 - 25% MeOH-DCM and then on a second silica cartridge (10 g) eluting first with DCM followed by 2%, 4% and 5% MeOH-DCM. Appropriate fractions were combined and evaporated under reduced pressure to give *the title compound* (15 mg) LCMS RT = 3.03 min, ES+ve *m*/*z* 550/552 (M+H)⁺.

### Intermediate 19

### 1,1-Dimethylethyl (2-{4-[4-[(4-chlorophenyl)methyl]-1-oxo-2(1H)-phthalazinyl] hexahydro-1H-azepin-1-yl}ethyl)carbamate

A solution of 4-[(4-chlorophenyl)methyl]-2-(hexahydro-1*H*-azepin-4-yl)-1(2*H*)-phthalazinone (for example, as prepared for Intermediate 16) (0.16 g, 0.43 mmol) in acetone (5 ml) was treated with a solution of 2-({[(1,1-dimethylethyl)oxy]carbonyl}amino)ethyl 4-methylbenzenesulfonate (for example, as disclosed in L.E. Canne, R.L. Winston, S.B.H. Kent, Tet. Lett., 38:3361-4, (1997), see compound 2) (0.15 g, 0.47 mmol) in acetone (2 ml), followed by sodium iodide (64.7 mg, 0.43 mmol) and DIPEA (51.8 µl, 0.43 mmol) and the mixture was heated to 66 °C under nitrogen overnight. The following morning another portion of 2-({[(1,1-dimethylethyl)oxy]carbonyl}amino)ethyl 4-methylbenzenesulfonate (75 mg) and DIPEA (26 µl) were added and the mixture heated for another day. The mixture was then applied to an SCX-2 cartridge (20 g), washed with MeOH and eluted with 10% aq. ammonia in MeOH. The ammoniacal fractions were concentrated under reduced pressure and the residue was purified by chromatography (Flashmaster II, 50 g cartridge) eluting with 0 to 30% MeOH containing 1% NEt₃ - DCM over 30 min to give *the title compound* (133.8 mg). LCMS RT = 2.80 min, ES+ve *m*/*z* 511 (M+H)⁺.

### Intermediate 20

### 2-[1-(2-Aminoethyl)hexahydro-1H-azepin-4-yl]-4-[(4-chlorophenyl)methyl]-1(2H)-phthalazinone hydrochloride salt

A solution of 1,1-dimethylethyl (2-{4-[4-[(4-chlorophenyl)methyl]-1-oxo-2(1*H*)-phthalazinyl]hexahydro-1*H*-azepin-1-yl}ethyl)carbamate (for example, as prepared for Intermediate 19) (133.8 mg) in DCM (5 ml) was treated with a solution of HCl in dioxane (4 M, 1 ml) at room temperature. After 2 h additional HCl (4 M, 0.5 ml) was added and the mixture was evaporated under reduced pressure to give *the title compound* (69.6 mg) LCMS RT = 2.25 min, ES+ve *m*/*z* 411 (M+H)⁺.

### Intermediate 21

### 1,1-Dimethylethyl (3-{4-[4-[(4-chlorophenyl)methyl]-1-oxo-2(1H)-phthalazinyl]hexahydro-1H-azepin-1-yl}propyl)carbamate

A solution of 4-[(4-chlorophenyl)methyl]-2-(hexahydro-1*H*-azepin-4-yl)-1(2*H*)-phthalazinone (for example, as prepared for Intermediate 16) (0.16 g, 0.43 mmol) in acetone (5 ml) was treated with a solution of 3-({[(1,1-dimethylethyl)oxy]carbonyl}amino)propyl 4-methylbenzenesulfonate (for example, as disclosed in S. Kondo, H. Iwasawa, D. Ikeda, Y. Umeda, Y. Ikeda, H. Iinuma, H. Umezawa, J. Antibiotics, 34:1625-7, (1981)) (156 mg, 0.47 mmol) in acetone (2 ml), followed by sodium iodide (64.7 mg, 0.43 mmol) and DIPEA (51.8 µl, 0.43 mmol) and the mixture was heated to 66 °C under nitrogen overnight. The mixture was then applied to an SCX-2 cartridge (20 g), washed with MeOH and eluted with 10% aq. ammonia in MeOH. The ammoniacal fractions were concentrated under reduced pressure and the residue was purified by chromatography (Flashmaster II, 50 g cartridge) eluting with 0 to 30% MeOH containing 1 % NEt₃ - DCM over 30 min to give *the title compound* (241 mg). LCMS RT = 2.81 min, ES+ve *m*/*z* 525 (M+H)⁺.

### Intermediate 22

### 2-[1-(3-aminopropyl)hexahydro-1H-azepin-4-yl]-4-[(4-chlorophenyl)methyl]-1(2H)-phthalazinone hydrochloride salt

A solution of 1,1-dimethylethyl (3-{4-[4-[(4-chlorophenyl)methyl]-1-oxo-2(1*H*)-phthalazinyl]hexahydro-1*H*-azepin-1-yl}propyl)carbamate (for example, as prepared for Intermediate 21) (241.4 mg) in DCM (5 ml) was treated with a solution of HCl in dioxane (4 M, 1 ml) at room temperature. After 2 h the mixture was evaporated under reduced pressure to give *the title compound* (114 mg) LCMS RT = 2.22 min, ES+ve *m*/*z* 425 (M+H)⁺.

### Intermediate 23

### 1,1-Dimethylethyl (4-{4-[4-[(4-chlorophenyl)methyl]-1-oxo-2(1H)-phthalazinyl]hexahydro-1H-azepin-1-yl}butyl)carbamate

A solution of 4-[(4-chlorophenyl)methyl]-2-(hexahydro-1*H*-azepin-4-yl)-1(2*H*)-phthalazinone (for example, as prepared for Intermediate 16) (0.16 g, 0.43 mmol) in acetone (5 ml) was treated with a solution of 4-({[(1,1-dimethylethyl)oxy]carbonyl}amino)butyl 4-methylbenzenesulfonate (for example, as disclosed in W. Hu, E. Reder, M. Hesse, Helv. Chim. Acta, 1996, 79, 2137-51, see compound 6) (163 mg, 0.47 mmol) in acetone (2 ml), followed by sodium iodide (64.7 mg, 0.43 mmol) and DIPEA (51.8 µl, 0.43 mmol) and the mixture was heated to 66 °C under nitrogen overnight. The mixture was then applied to an SCX-2 cartridge (20 g), washed with MeOH and eluted with 10% aq. ammonia in MeOH. The ammoniacal fractions were concentrated under reduced pressure and the residue was purified by chromatography (Flashmaster II, 50 g cartridge) eluting with 0 - 30% MeOH containing 1% NEt₃ - DCM over 30 min to give *the title compound* (118.6 mg). LCMS RT = 2.83 min, ES+ve *m*/*z* 539 (M+H)⁺

### Intermediate 24

### 2-[1-(4-aminobutyl)hexahydro-1H-azepin-4-yl]-4-[(4-chlorophenyl)methyl]-1(2H)-phthalazinone hydrochloride salt

A solution of 1,1-dimethylethyl (4-{4-[4-[(4-chlorophenyl)methyl]-1-oxo-2(1*H*)-phthalazinyl]hexahydro-1*H*-azepin-1-yl}butyl)carbamate (for example, as prepared for Intermediate 23) (118.6 mg) in DCM (5 ml) was treated with a solution of HCl in dioxane (4 M, 1 ml) at room temperature. After 2 h the mixture was evaporated under reduced pressure to give *the title compound* (108.8 mg) LCMS RT = 2.25 min, ES+ve *m*/*z* 439 (M+H)⁺.

### Intermediate 25

### 2-{4-[(3-Chloropropyl)oxy]phenyl}ethanol

4-(2-Hydroxyethyl)phenol (commercially available, for example, from Aldrich), (10 g, 72 mmol) was dissolved in 2-butanone (250 ml) then potassium carbonate (19.9 g, 0.144 mol) was added then 1-bromo-3-chloropropane (commercially available, for example, from Aldrich) (8.54 ml, 0.144 mol) was added and the reaction mixture heated at 80 °C for 18 h. The cooled reaction mixture was diluted with water (500 ml), layers separated and aqueous extracted with DCM (2 × 200 ml). The combined organic extracts were dried (MgSO₄), evaporated *in vacuo* and purified by Flashmaster II (3 × 100g silica cartridges) eluted with 0 - 100% EtOAc-cyclohexane over 40 min to give *the title compound* (14.12 g). LCMS RT = 2.84 min ES+ve *m*/*z* 232 (M+NH₄)⁺

### Intermediate 26

### 2-(4-{[3-(Hexahydro-1H-azepin-1-yl)propyl]oxy}phenyl)ethanol

A solution of 2-{4-[(3-chloropropyl)oxy]phenyl}ethanol (for example, as prepared for Intermediate 25), (14 g, 0.065 mol) in 2-butanone (200 ml) was treated with potassium carbonate (17.96 g, 0.13 mol), potassium iodide (1.24 g, 7.5 mmol), hexahydro-1*H-*azepine (commercially available, for example, from Aldrich) (14.71 ml, 0.1308 mol) and heated at 80 °C under nitrogen for 18 h. The cooled reaction mixture was diluted with water (300 ml), layers separated and the aqueous extracted with DCM (2 × 200 ml). The combined organic extracts were dried (MgSO₄) and evaporated *in vacuo* to give a yellow oil (23 g). A portion of this (10 g) was purified by Flashmaster II (100 g silica cartridge), eluted with 0 - 100% EtOAc-cyclohexane over 15 min, then 100% EtOAc for 10 min, then 0 - 10% (10% aq. ammonia-MeOH)-DCM for 15 min, then 10% of (10% aq. ammonia-MeOH) - DCM for 10 min to give *the title compound* (5.3 g). LCMS RT = 1.9 min, ES+ve *m*/*z* 278 (M+H)⁺.

### Intermediate 27

### 2-(4-{[3-(Hexahydro-1H-azepin-1-yl)propyl]oxy}phenyl)ethyl methanesulfonate

### Preparation A

2-(4-{[3-(Hexahydro-1*H*-azepin-1-yl)propyl]oxy}phenyl)ethanol) (for example, as prepared for Intermediate 26) (273 mg, 0.98 mmol), was dissolved in DCM (5 ml) and treated with DIPEA (0.204 ml, 1.2 mmol) and mesyl chloride (0.093 ml, 1.2 mmol). The mixture was stirred at room temperature for 3 h. Further mesyl chloride (0.020 ml, 0.26 mmol) was added and stirring was continued for 45 min. Saturated aq. sodium hydrogen carbonate (10 ml) was added to the mixture. The layers were separated, and the aqueous was washed with further DCM. The combined DCM extracts were concentrated *in vacuo* to afford *the title compound,* which was used without further purification. LCMS RT = 2.18 min, ES+ve *m*/*z* 356 [M+H]⁺.

### Preparation B

Intermediate 27 may be prepared in analogous manner to Intermediate 35 using 2-(4-{[3-(hexahydro-1*H*-azepin-1-yl)propyl]oxy}phenyl)ethanol (for example, as prepared for Intermediate 26) (0.080 g, 0.29 mmol, to give *the title compound* (0.101 g, 100%). LCMS RT = 2.18 min, ES+ve *m*/*z* 356 (M+H)⁺.

### Intermediate 28

### Ethyl 3-(4-{[3-(hexahydro-1H-azepin-1-yl)propyl]oxy}phenyl)propanoate,

3-(Hexahydro-1*H*-azepin-1-yl)-1-propanol (for example, as disclosed in E. L. Strogryn, J. Med. Chem., 13:864-6, (1970)) (412 mg, 3 mmol) and ethyl (4-hydroxyphenyl)propionate (commercially available, for example, from Maybridge) (641 mg, 3.3 mmol) were added to a stirred mixture of triphenylphosphine (1.02 g, 3.9 mmol) and diisopropyl azodicarboxylate (0.66 ml, 3.9 mmol) in THF (15 ml) at -20 °C. After 10 min, the solution was allowed to warm to 21 °C. After 18 h, no input material was apparent by LCMS. MeOH was added to quench any excess reagent and the mixture was evaporated to dryness. The residue was loaded onto two 100 g Flashmaster II silica cartridges which were run with 0 - 50% EtOAc in cyclohexane over 40 min. The cartridges were re-run using 0 - 25% MeCH in EtOAc over 40 min. The UV-detector did not detect the product so the waste eluate was evaporated to give the crude product (782 mg). This was further purified on a 50 g silica cartridge run in 0 - 50% MeOH in DCM over 40 min to give the *title compound* (306 mg). LCMS RT = 2.39 min, ES+ve *m*/*z* 334 [M+H]⁺

### Intermediate 29

### 3-(4-{[3-(Hexahydro-1H-azepin-1-yl)propyl]oxy}phenyl)-1-propanol

Ethyl 3-(4-{[3-(hexahydro-1*H*-azepin-1-yl)propyl]oxy}phenyl)propanoate (for example, as prepared for Intermediate 28) (306 mg, 0.92 mmol) was stirred under nitrogen in THF (5 ml) and a solution of lithium aluminium hydride in diethyl ether (1 M, 0.5 ml) was added over about 20 seconds at 21 °C. After 30 min, LCMS showed complete reaction. Wet THF was added and after 10 min, Na₂SO₄ was added. The mixture was filtered and the filtrate plus leachings of the solid filter cake were evaporated to dryness. To remove final traces of Na₂SO₄, the residue was dissolved in EtOAc and filtered through a cotton wool plug. Evaporation gave the *title compound* LCMS RT = 2.04 min, ES+ve *m*/*z* 292 [M+H]⁺.

### Intermediate 30

### Mixture of 3-(4-{[3-(hexahydro-1H-azepin-1-yl)propyl]oxy}phenyl)propyl 4-methylbenzenesulfonate and 1-(3-{[4-(3-chloropropyl)phenyl]oxy}propyl)hexahydro-1H-azepine (3:2)

3-(4-{[3-(Hexahydro-1*H*-azepin-1-yl)propyl]oxy}phenyl)-1-propanol (for example, as prepared for Intermediate 29) (146 mg, 0.5 mmol) was stirred in DCM (2 ml) containing pyridine (0.2 ml, 2.5 mmol) with ice-water cooling and tosyl chloride (115 mg, 0.6 mmol) was added. After 10 min the mixture was allowed to warm to 21 °C. After 2 h LCMS indicated reaction was incomplete. More tosyl chloride (60 mg, 0.31 mmol) was added and stirring was continued for 5 h. Reaction was incomplete. The mixture was allowed to stand at 21 °C for three days and then more tosyl chloride (60 mg, 0.31 mmol) and pyridine (0.1 ml, 1.25 mmol) were added. After stirring for a further 3 h, LCMS indicated only a little starting material. The mixture was evaporated to an oil. This was dissolved in DCM and loaded onto a 50 g Flashmaster II silica cartridge. The cartridge was run with 0-50% MeOH in DCM over 30 min. The eluate containing a double peak was evaporated to give the crude *title mixture* (133 mg), tosylate LCMS RT = 2.79 min, ES+ve *mlz 446* [M+H]⁺, chloro-analogue LCMS RT = 2.59 min, ES+ve *m*/*z* 310 and 312 [M+H]⁺ and toluene sulphonic acid RT = 2.31 min ES-ve *m*/*z* 171 [M-H]⁻. This mixture was used in the next step.

### Intermediate 31

### 1-[(3-Chloropropyl)oxy]-4-iodobenzene

A mixture of 4-iodophenol (commercially available, for example, from Aldrich) (20 g), 1-bromo-3-chloropropane (commercially available, for example, from Aldrich) (17.91 g), and potassium carbonate (25.2 g) in 2-butanone (400 ml) was stirred at reflux under a nitrogen atmosphere for 18 h. The mixture was allowed to cool and was filtered. The filtrate was evaporated and the residue was dissolved in cyclohexane and purified by Flashmaster II on a 100 g silica cartridge, eluting with cyclohexane and then 20% EtOAc in cyclohexane. The solvent was evaporated from appropriate fractions giving the *title compound* as a colourless oil which crystallised on standing (15.928 g). LCMS RT = 3.70 min. The solvent was evaporated from a further set of fractions giving additional portion of the *title compound* as a pale yellow oil that solidified on standing (6.668 g). ¹H NMR (CDCl₃) δ 2.23 (2H, quint, J = 6 Hz), 3.74 (2H, t, J = 6 Hz), 4.09 (2H, t, J = 6 Hz), 6.70 (2H, m), 7.57 (2H, m).

### Intermediate 32

### 1-{3-[(4-Iodophenyl)oxy]propyl}hexahydro-1H-azepine

A mixture of 1-[(3-chloropropyl)oxy]-4-iodobenzene (for example, as prepared for Intermediate 31), (15.855 g), sodium iodide (8 g) and hexamethyleneimine (commercially available, for example, from Aldrich) (15.1 ml) in 2-butanone (200 ml) was stirred at 80 °C under a nitrogen atmosphere for about 22 h. The reaction mixture was filtered and the filtrate was evaporated giving a beige residue. This material was triturated with diethyl ether and a pale solid was recovered by filtration. The solid was partitioned between DCM (200 ml) and saturated sodium bicarbonate solution (200 ml). The organic layer was dried over anhydrous Na₂SO₄ and evaporated to give a residue that was purified by Flashmaster II chromatography on a 50 g silica cartridge using a 0 - 30% MeOH containing 1% NEt₃ - DCM gradient over 50 min. The solvent was evaporated from appropriate fractions to give the *title compound* as a yellow solid (1.2708 g). LCMS RT = 2.39 min, ES+ve *m*/*z* 360 (M+H)⁺. The filtrate from the trituration was evaporated yielding a brown residue that was purified by Flashmaster II chromatography on 2 × 100 g silica cartridges using 0-30% MeOH containing 1% NEt₃ - DCM gradient over 50 min. The solvent was evaporated from appropriate fractions giving the *title compound* as a yellow oil (15.9 g). LCMS RT = 2.40 min, ES+ve *m*/*z* 360 (M+H)⁺.

### Intermediate 33

### 4-(4-{[3-(Hexahydro-1H-azepin-1-yl)propyl]oxy}phenyl)-3-butyn-1-ol

A mixture of 1-{3-[(4-iodophenyl)oxy]propyl}hexahydro-1*H*-azepine (for example, as prepared for Intermediate 32) (1.268 g), 3-butyn-1-ol (commercially available, for example, from Aldrich) (668 µl), NEt₃ (2.5 ml), bis(triphenylphosphine)palladium(II) chloride (124 mg) and copper(I) iodide (34 mg) in THF (15 ml) was stirred at room temperature under a nitrogen atmosphere for about 5 h. The mixture was filtered (60 ml PTFE filter tube) and the filtrate was evaporated giving a residue. This material was purified by Flashmaster II chromatography on a 100 g silica cartridge using 0 - 30% MeOH containing 1% NEt₃ - DCM gradient over 60 min. Evaporation of the solvent from appropriate fractions gave a residue that was dissolved in MeOH and loaded onto a SCX ion-exchange cartridge (50 g). The cartridge was washed with MeOH and then eluted with 2 M ammonia in MeOH. Evaporation of the solvent from the ammonia-containing fractions gave the *title compound* as a dark yellow residue (823 mg). LCMS RT = 2.10 min, ES+ve *m*/*z* 302 (M+H)⁺.

### Intermediate 34

### 4-(4-{[3-(Hexahydro-1H-azepin-1-yl)propyl]oxy}phenyl)-1-butanol

To a solution of 4-(4-{[3-(hexahydro-1*H*-azepin-1-yl)propyl]oxy}phenyl)-3-butyn-1-ol (for example, as prepared for Intermediate 33) (823 mg) in EtOH (20 ml) was added 1.25 M HCl in MeOH (3.25 ml) and the resulting mixture was hydrogenated over 10% w/w Pd/C (350 mg) for about 4 h. The reaction mixture was filtered through celite and the solvent was evaporated. The residue was dissolved in DCM (125 ml) and the resulting solution was washed with 2 N NaOH (100 ml) and brine (100 ml) and dried over anhydrous Na₂SO₄. Evaporation of the solvent gave the *title compound* as a dark yellow residue (726 mg). LCMS RT = 2.18 min, ES+ve *m*/*z* 306 (M+H)⁺.

### Intermediate 35

### 4-(4-{[3-(Hexahydro-1H-azepin-1-yl)propyl]oxy}phenyl)butyl methanesulfonate

To a solution of 4-(4-{[3-(hexahydro-1*H*-azepin-1-yl)propyl]oxy}phenyl)-1-butanol (for example, as prepared for Intermediate 34) (0.077 g, 0.25 mmol, in anhydrous DCM (2 ml) was added DIPEA (0.053 ml, 0.30 mmol) and then methanesulfonyl chloride (0.023 ml, 0.30 mmol). The solution was stirred at 20 °C for 2 h. The solution was diluted with DCM (10 ml) and saturated sodium hydrogen carbonate (10 ml). The biphasic mixture was shaken and the phases separated using a hydrophobic frit. The organic phase was removed *in vacuo* to give *the title compound* (0.09 g). LCMS RT = 2.43 min, ES+ve *m*/*z* 384 (M+H)⁺.

### Intermediate 36

### 1-{3-[(4-Bromophenyl)oxy]propyl}hexahydro-1H-azepine

A mixture of 1-bromo-4-[(3-chloropropyl)oxy]benzene (for example, as disclosed in R. Faghih, W. Dwight, A. Vasudevan, J. Dinges, S.E. Conner, T.A. Esbenshade, Y.L. Bennani, A.A. Hancock, Biorg. Med. Chem. Lett., 12:3077-9, (2002), see scheme 1) (25 mmol), hexamethyleneimine (commercially available, for example, from Aldrich) (6 ml, 50 mmol), sodium iodide (300 mg, 2 mmol) and potassium carbonate (3.45 g, 25 mmol) in acetone (50 ml) was heated overnight to 78 °C. The mixture was allowed to cool to room temperature and the solid was removed by filtration. The filtrate was evaporated under reduced pressure and the residue was dissolved in EtOAc, washed with aq. sodium bicarbonate solution, brine, dried over MgSO₄ and evaporated under reduced pressure. The residue was dissolved in MeOH and applied to an SCX-2 cartridge (70 g) eluting first with MeOH, followed by 10% aq. ammonia in MeOH. The ammoniacal fractions were combined and evaporated under reduced pressure to give *the title compound* (6.866 g) LCMS RT = 2.28 min, ES+ve *m*/*z* 312/314 (M+H)⁺.

### Intermediate 37

### 1-[3-({4-[(1E)-4,4-Bis(ethyloxy)-1-buten-1-yl]phenyl}oxy)propyl]hexahydro-1H-azepine

A solution of 3-butenal diethylacetal (commercially available, for example, from Aldrich) (4.14 ml, 24.4 mmol) in THF (7 ml) was treated under nitrogen with 9-BBN solution in THF (0.5 M, 50 ml) in an ice-bath, and the solution was stirred for 3 h. In the meantime a solution of 1-{3-[(4-bromophenyl)oxy]propyl}hexahydro-1*H*-azepine (for example, as prepared for Intermediate 36) (6.866 g, 22 mmol) in DMF (7 ml) was treated with potassium carbonate (10.11 g, 73.2 mmol) and [1,1'-bis(diphenylphosphino)ferrocene]dichloropalladium (536 mg, 0.73 mmol) and then slowly with the above solution. The mixture was then heated to 70 °C under nitrogen for 3 h and then at 80 °C overnight. The mixture was allowed to cool to room temperature, diluted with EtOAc and water. The organic solution was washed with aq. sodium bicarbonate solution, brine, dried (MgSO₄) and evaporated under reduced pressure. The residue (11.12 g) was dissolved in DCM (30 ml) and purified by chromatography (Flashmaster II, 3 × 100 g silica cartridges) eluting with 0 - 15% MeOH containing 1% NEt₃ - DCM over 40 min. Appropriate fractions were combined and evaporated under reduced pressure to give *the title compound* (6.14 g) as a 1:1 mixture with 1-[3-({4-[4,4-bis(ethyloxy)butyl]phenyl}oxy)propyl]hexahydro-1*H*-azepine LCMS RT = 2.59 min, ES+ve *m*/*z* 376/378 (1:1) (M+H)⁺.

### Intermediate 38

### 1-[3-({4-[4,4-Bis(ethyloxy)butyl]phenyl}oxy)propyl]hexahydro-1H-azepine

A solution of 1-[3-({4-[(1*E*)-4,4-bis(ethyloxy)-1-buten-1-yl]phenyl}oxy)propyl]hexahydro-1*H*-azepine (for example, as prepared for Intermediate 37) containing 1-[3-({4-[4,4-bis(ethyloxy)butyl]phenyl}oxy)propyl]hexahydro-1*H*-azepine (1:1, 6.14 g) in EtOH (120 ml) was hydrogenated over 10% Pd/C (0.6 g). After 4 h the catalyst was collected by filtration, washed with EtOH and the filtrate and washings were evaporated under reduced pressure. The residue was purified by chromatography (Flashmaster II, 2 × 100 g silica cartridges) eluting with 0 - 15% MeOH containing 1% NEt₃ - DCM over 40 min. Appropriate fractions were combined and evaporated under reduced pressure to give 3.5 g of impure product, which was purified further by Flashmaster I chromatography on a 100 g silica cartridge eluting with 0 - 15% (10% aq. ammonia in EtOH) - DCM to give a mixture of *the title compound* and 1-[3-(phenyloxy)propyl]hexahydro-1*H*-azepine (1.78 g, 4:1) LCMS RT = 2.59 min ES+ve *m*/*z* 378 (M+H)⁺. Remaining fractions were combined and evaporated under reduced pressure to give less pure product (1.782 g) which was chromatographed on Flashmaster I (100 g silica cartridge) eluting with 0-15% MeOH - EtOAc. Appropriate fractions were combined and evaporated to give *the title compound* (410 mg) LCMS RT = 2.58 min ES+ve *m*/*z* 378 (M+H)⁺.

### Intermediate 39

### 1-[3-({4-[4,4-Bis(methyloxy)butyl]phenyl}oxy)propyl]hexahydro-1H-azepine

A solution of 1-[3-({4-[4,4-bis(ethyloxy)butyl]phenyl}oxy)propyl]hexahydro-1*H*-azepine (for example, as prepared for Intermediate 38) in MeOH was applied to an SCX-2 cartridge (20 g) washing with 3 CV of MeOH. The cartridge was then eluted with 10% aq. ammonia in MeOH and the ammoniacal fractions were combined and evaporated to give *the title compound* (243 mg) LCMS RT = 2.43 min ES+ve *m*/*z* 350 (M+H)⁺.

### Intermediate 40

### 4-(4-{[3-(Hexahydro-1H-azepin-1-yl)propyl]oxy}phenyl)butanal acetate salt

A solution of 1-[3-({4-[4,4-bis(methyloxy)butyl]phenyl}oxy)propyl]hexahydro-1*H*-azepine (for example, as prepared for Intermediate 39) (243 mg, 0.6 mmol) in acetic acid (5 ml) and water (3 ml) was heated to 60 °C for 2 h under nitrogen. The mixture was then evaporated under reduced pressure to give *the title compound* (305 mg) LCMS RT = 2.20 min ES+ve *m*/*z* 304 (M+H)⁺.

### Intermediate 41

### Methyl 5-(4-{[3-(hexahydro-1H-azepin-1-yl)propyl]oxy}phenyl)pentanoate

Triphenylphosphine (1.84 g, 7.0 mmol) was stirred with di-*tert*-butyl azodicarboxylate (1.61 g, 7 mmol) in THF (30 ml) at -20 °C and after 10 min, solutions of 3-(hexahydro-1*H-*azepin-1-yl)-1-propanol (for example, as disclosed in E. L. Strogryn, J. Med. Chem., 1970, 13, 864-866) (1.0 g, 6.35 mmol) and methyl 5-(4-hydroxyphenyl)pentanoate (prepared in a manner analogous to that described in Yi, Ching Sui; Martinelli, Louis C.; Blanton, C. DeWitt, Jr. J. Org. Chem., 43:405-9, (1978) (compound 17), but using sulphuric acid at room temperature instead of hydrochloric acid at reflux) (1.32 g, 6.35 mmol) each in THF (about 4 ml) were added. The solution was left to warm to room temperature over an hour and it was then heated to 75 °C for 48 h by when no further reaction was occurring. The solution was evaporated to dryness and the residue was dissolved in MeOH and loaded onto a SCX-2 cartridge (70 g) which had been preconditioned with MeOH. The cartridge was eluted with MeOH and then with 10% aq. 0.88 ammonia in MeOH to elute the product. Evaporation gave the *title compound* (1.52 g). LCMS RT = 2.55 min, ES+ve *mlz* 348 [M+H]⁺.

### Intermediate 42

### 5-(4-{[3-(Hexahydro-1H-azepin-1-yl)propyl]oxy}phenyl)-1-pentanol

Methyl 5-(4-{[3-(hexahydro-1*H*-azepin-1-yl)propyl]oxy}phenyl)pentanoate (for example, as prepared for Intermediate 41) (1.52 g, 4.37 mmol) was stirred under nitrogen in THF (30 ml) and a solution of lithium aluminium hydride in diethyl ether (1 M, 2.2 ml) was added over about 1 min. After 15 min, LCMS showed complete reaction. Wet THF was added and after 10 min Na₂SO₄ was added. The mixture was filtered and the filtrate was evaporated to give an oil, the *title compound* (0.95 g) LCMS RT = 2.34 min, ES+ve *m*/*z* 320 [M+H]⁺.

### Intermediate 43

### 5-(4-{[3-(Hexahydro-1H-azepin-1-yl)propyl]oxy}phenyl)pentyl methanesulfonate

5-(4-{[3-(Hexahydro-1*H*-azepin-1-yl)propyl]oxy}phenyl)1-pentanol (for example, as prepared for Intermediate 42) (0.12 g, 0.37 mmol) was stirred with mesyl chloride (0.035 ml, 0.45 mmol) in DCM (3 ml) at room temperature under nitrogen and DIPEA (0.078 ml, 0.45 mmol) was added. After 3 h, reaction was complete. The solution was diluted with more DCM and it was washed with sodium bicarbonate solution. The aqueous layer was extracted with more DCM and the combined organic layers were washed with water, dried with MgSO₄ and evaporated to an oil, the *title compound* (148 mg) LCMS RT = 2.60 min, ES+ve *m*/*z* 398 [M+H]⁺.

### Intermediate 44

### 3-[(3-Cyclobutyl-2,3,4,5-tetrahydro-1H-3-benzazepin-7-yl)oxy]-1-propanol

A mixture of 3-cyclobutyl-2,3,4,5-tetrahydro-1*H*-3-benzazepin-7-ol (for example, as disclosed in patent application WO 2004/056369A1, see Example 3) (5 g), potassium carbonate (7.95 g) and 3-bromo-1-propanol (commercially available, for example, from Aldrich) (2.18 ml) in 2-butanone (115 ml) was heated at reflux with stirring for about 18 h. The mixture was filtered and evaporated giving a residue that was dissolved in DCM and purified by Flashmaster II chromatography on a 100 g silica cartridge eluting with 0 - 30% MeOH containing 1% NEt₃ - DCM gradient over 40 min. The solvent was evaporated from appropriate fractions giving a crude sample of the *title compound* (3.375 g), which was further purified by Biotage flash chromatography on a KP-Sil 40 M cartridge eluting with 3% (2 M ammonia in MeOH) in DCM. The solvent was evaporated from appropriate fractions to give the *title compound* (2.3 g). LCMS RT = 1.80 min, ES+ve *m*/*z* 276 (M+H)⁺.

### Intermediate 45

### 3-[(3-Cyclobutyl-2,3,4,5-tetrahydro-1H-3-benzazepin-7-yl)oxy]propyl methanesulfonate

3-[(3-Cyclobutyl-2,3,4,5-tetrahydro-1*H*-3-benzazepin-7-yl)oxy]-1-propanol (for example, as prepared for Intermediate 44) (55 mg, 0.2 mmol), was dissolved in DCM (1.5 ml) and treated with DIPEA (0.042 ml, 0.24 mmol) and mesyl chloride (0.017 ml, 0.24 mmol). The mixture was stirred at room temperature under a nitrogen atmosphere for 2 h, and then concentrated *in vacuo* to afford *the title compound,* which was used in the next step without further purification. LCMS RT = 2.09 min, ES+ve *m*/*z* 354 [M+H]⁺.

### Intermediate 46

### 7-[(3-Chloropropyl)oxy]-3-cyclobutyl-2,3,4,5-tetrahydro-1H-3-benzazepine

To a suspension of 3-cyclobutyl-2,3,4,5-tetrahydro-1*H*-3-benzazepin-7-ol (for example, as disclosed in patent application WO 2004/056369A1, see Example 3) (0.3 g, 1.38 mmol) in acetone (3 ml) was added potassium carbonate (0.253 g, 1.83 mmol) and then 1-bromo-3-chloropropane (commercially available, for example, from Aldrich) (0.150 ml, 1.52 mmol). A further portion of potassium carbonate (0.140 g , 1.0 mmol) was added after 35 min. The suspension was heated to reflux for 23 h. The solvent had evaporated overnight. The mixture was partitioned between EtOAc (50 ml) and water (40 ml). The phases were separated and the organic phase washed with water (2 × 40 ml), dried over MgSO₄ and filtered. The solvent was removed *in vacuo* to give *the title compound* (0.3 g, 74 %). LCMS RT = 2.27 min, ES+ve *m*/*z* 294/296 (M+H)⁺

### Intermediate 47

### 3-Cyclobutyl-8-hydroxy-2,3,4,5-tetrahydro-1H-3-benzazepine7-carbaldehyde

A solution of 3-cyclobutyl-2,3,4,5-tetrahydro-1*H-*3-benzazepin-7-ol (for example, as disclosed in patent application WO 2004/056369A1, see Example 3) (2 g) in THF (20 ml) under nitrogen was treated dropwise with a solution of methylmagnesium bromide in diethyl ether (3 M, 3.1 ml). The suspension was stirred for 10 min and then diluted with toluene (100 ml). Paraformaldehyde (1 g) and NEt₃ (1.5 g) were added and the mixture was heated at 80 °C for 3 h. The cooled mixture was treated with 2 M HCI (50 ml) and stirred for 10 min. The mixture was basified cautiously with aq. sat. sodium bicarbonate and extracted into EtOAc. The dried (Na₂SO₄) organic phase was evaporated and the residue was purified by column chromatography on silica (20 g) eluting with DCM-MeOH-aq. ammonia (95:5:0.5) to give the *title compound* (1.4 g). LCMS RT = 1.55 min.

### Intermediate 48

### 3-Cyclobutyl-2,3,4,5-tetrahydro-1H-3-benzazepine-7-carbaldehyde

A solution of 3-cyclobutyl-8-hydroxy-2,3,4,5-tetrahydro-1*H*-3-benzazepine-7-carbaldehyde (for example, as prepared for Intermediate 47) (8.5 g) and NEt₃ (20 ml) in DMF (60 ml) was treated portionwise with 1,1,1-trifluoro-*N*-phenyl-*N-*[(trifluoromethyl)sulfonyl]methanesulfonamide (commercially available, for example from Aldrich) (14 g). The mixture was stirred for 16 h and palladium (II) acetate (300 mg), 1,3-bis(diphenylphosphino)propane (520 mg), and trioctylsilane (20 ml) were added. The mixture was heated at 75 °C for 2 h under nitrogen, cooled and partitioned between water and DCM. The dried (Na₂SO₄) organic phase was evaporated and the residue was purified by column chromatography on silica (150 g) eluting with DCM-MeOH-aq. ammonia (98:2:0.2) and further purified by column chromatography on silica (150 g) eluting with EtOAc-MeOH-aq. ammonia (95:5:0.5) to give the *title compound* (7.4 g) LCMS RT = 1.56 min.

### Intermediate 49

### Ethyl 2,3,4,5-tetrahydro-1H-3-benzazepine-7-carboxylate

A solution of 1,1-dimthylethyl 7-cyano-1,2,4,5-tetrahydro-3*H*-benzazepine-3-carboxylate (for example, as disclosed in patent application EP 0528369A2, see Example 17) (3.7 g, 13.6 mmol) in premixed trimethylsilylchloride (25 ml) - EtOH (15 ml) was refluxed overnight (bath temperature 71 °C) under static argon. Vigorous effervescence occurred during the initial warm-up to reflux. Over a period of 5 days, additional aliquots of trimethylsilylchloride and EtOH were added until a total of 103 ml trimethylsilylchloride (0.81 mol) and EtOH (69 ml, 1.17 mol) had been added. The reaction was cooled and, with ice-bath cooling, water (100 ml) was slowly added, keeping the temperature < 40 °C. After 15 min. the temperature was increased to 24 °C and 2 N NaOH was added until the pH > 13. The solution was extracted with DCM (2 × 500 ml) and the organic extracts brine washed, dried and evaporated to give the crude product (2.59 g). After removal of 0.13 g, the remainder was purified by silica chromatography on a 200 g Biotage column eluting with DCM-2 M ammonia in MeOH (10:1) to give *the title compound* (2.3 g, 81%). TLC (silica) Rf = 0.10 DCM - 2 N ammonia in MeOH, 20 : 1

### Intermediate 50

### Ethyl 3-cyclobutyl-2,3,4,5-tetrahydro-1H-3-benzazepine-7-carboxylate

A solution of ethyl 2,3,4,5-tetrahydro-1*H*-3-benzazepine-7-carboxylate (for example, as prepared for Intermediate 49) (2.3 g, 10.5 mmol) in DCM (30 ml)-acetic acid (0.3 ml) was stirred under argon with ice-bath cooling and cyclobutanone (commercially available, for example, from Aldrich) (1.24 ml, 16.5 mmol) was added. After 30 min at 10 °C, sodium triacetoxyborohydride (3.5 g, 16.5 mmol) was added portionwise. The ice-bath was removed and the mixture stirred for 17 h at ambient temperature and then poured into DCM (50 ml)-saturated aq. potassium carbonate (50 ml). The 2-phase mixture was basified to pH 13 with 2 N aq. NaOH. The layers was separated, the aqueous extracted with more DCM (80 ml) and the combined organic layers were brine-washed, dried and evaporated to give (*the title compound*) as a crystalline solid (2.8 g, 97%). TLC (silica): Rf = 0.67 DCM - 2N ammonia in MeOH (20 : 1)

### Intermediate 51

### 3-Cyclobutyl-2,3,4,5-tetrahydro-1H-3-benzazepine-7-carboxylic acid

A stirred solution of ethyl 3-cyclobutyl-2,3,4,5-tetrahydro-1*H*-3-benzazepine-7-carboxylate (for example, as prepared for Intermediate 50) (2.7 g, 10 mmol) in EtOH (35 ml) was treated with 2 N NaOH (10 ml). After 27 h at ambient temperature, the solution was carefully acidified to pH 6 with about 10 ml 2 N HCI. The solution was evaporated and the residue treated with chloroform and concentrated to dryness under vacuum. The solid residue was taken up in premixed 1:1 chloroform : MeOH (50 ml) and solids were filtered through celite. The solution was evaporated and the residue treated with chloroform and concentrated to dryness under vacuum to give the *title compound* with about 25 mol% chloroform (3.2 g, quant.). ¹H NMR (400 MHz, CD₃OD): δ 7.84 (1H, d), 7.83 (1H, s), 7.26 (1H, d), 3.63 (1H, m), 3.20 (8H, br. s), 2.35 (4H, m), 1.88 (1H, m), 1.77 (1H, m).

### Intermediate 52

### 4-(2-{[(1,1-Dimethylethyl)(dimethyl)silyl]oxy}ethyl)phenol

4-(2-Hydroxyethyl)phenol (commercially available, for example, from Aldrich) (8.28 g, 0.06 mol) was stirred with *t*-butyldimethylsilyl chloride (9.9 g, 0.066 mol) and imidazole (8.4 g, 0.123 mol) in DMF (60 ml) at 21 °C under nitrogen for 19 h. TLC in 10% EtOAc-cyclohexane indicated complete reaction. The solution was evaporated to syrup which was partitioned between diethyl ether and water plus 2 N HCI to give pH 3. The aqueous layer was extracted with more diethyl ether. The combined organic layers were washed with brine, dried with MgSO₄ and evaporated. The residue was dissolved in DCM and loaded onto a column of silica gel (500 g) which had been set up in DCM. The column was eluted with DCM to give the *title compound* (13.09 g). LCMS RT = 3.78 min, ES+ve *m*/*z* 253 [M+H]⁺

### Intermediate 53

### 1,1-Dimethylethyl 4-{[4-(2-{[(1,1-dimethylethyl)(dimethyl)silyl]oxy}ethyl)phenyl]oxy}-1-piperidinecarboxylate

Di-*tert*-butyl azodicarboxylate (0.963 g, 4.18 mmol) was added portion wise to a stirring mixture of 4-(2-{[(1,1-dimethylethyl)(dimethyl)silyl]oxy}ethyl)phenol (for example, as prepared for Intermediate 52) (0.96 g, 3.8 mmol), *tert*-butyl-4-hydroxy-1-piperidine carboxylate (commercially available, for example, from Aldrich) (0.842 g, 4.18 mol), and triphenylphosphine (1.09 g, 4.18 mmol) in anhydrous THF (10 ml) at 0 °C. Once addition was complete the mixture was allowed to warm to room temperature and was stirred under nitrogen for 3 h. The reaction mixture was concentrated *in vacuo,* then triturated three times with diethyl ether (20 ml) and filtered to remove triphenylphosphine oxide. The diethyl ether was concentrated *in vacuo.* The experiment was repeated on a 1 mmol scale exactly as above and the combined crude materials (3.3 g) were purified on a silica cartridge (100 g) using Flashmaster II. The crude material was first pre-absorbed on to Florisil (100-200 mesh) and the column eluted with 0 - 25% EtOAc-cyclohexane over 65 min with a 5 min flush of 25 - 50% EtOAc-cyclohexane. The appropriate fractions were combined and concentrated *in vacuo* to give *the title compound* (1.46 g). LCMS RT 4.36 min ES+ve *m*/*z* 436 (M+H)⁺, 453 (M+NH₄)⁺

### Intermediate 54

### 2-[4-(4-Piperidinyloxy)phenyl]ethyl trifluoroacetate trifluoroacetate

1,1-Dimethylethyl 4-{[4-(2-{[(1,1-dimethylethyl)(dimethyl)silyl]oxy}ethyl)phenyl]oxy}-1-piperidinecarboxylate (for example, as prepared for Intermediate 53) (1.37 g, 3.14 mmol) was dissolved in DCM (25 ml) then TFA (5.5 ml) was added. The resultant solution was stirred under nitrogen at room temperature for about 17 h. The solution was concentrated *in vacuo,* dissolved in DCM (5 ml) and evaporated *in vacuo,* then the residue was dissolved in toluene (5 ml) to give *the title compound* (1.55 g). LCMS RT = 2.38 min, ES+ve *m*/*z* 318 (M+H)⁺.

### Intermediate 55

### 2-{4-[(1-Cyclobutyl-4-piperidinyl)oxy]phenyl}ethanol

To a stirring solution of 2-[4-(4-piperidinyloxy)phenyl]ethyl trifluoroacetate trifluoroacetate (for example, as prepared for Intermediate 54) (1.49 g, 3.46 mmol) in DCM (60 ml), acetic acid (0.2 ml) and cyclobutanone (commercially available, for example, from Aldrich) (0.775 ml, 10.38 mmol) were added. Sodium triacetoxyborohydride (1.1 g, 5.19 mmol) was added and the mixture stirred for 5 days at room temperature. Further portions of acetic acid (0.2 ml), cyclobutanone (0.775 ml) and sodium triacetoxyborohydride (0.88 g, 2.77 mmol) were added and the mixture stirred for 20 h. The reaction mixture was diluted with DCM (20 ml) and washed with NaOH solution (2 N, 80 ml), dried over Na₂SO₄ and concentrated *in vacuo.* The residue was purified on a SCX ion exchange cartridge (50 g), the column was eluted with MeOH (6 × 45 ml) followed by 2 N ammonia in MeOH solution (6 × 45 ml). The basic fractions were combined and concentrated *in vacuo* to afford the *title compound* (0.668 g). LCMS RT = 1.84 min ES+ve *m*/*z* 276 (M+H)⁺.

### Intermediate 56

### 2-{4-[(1-Cyclobutyl-4-piperidinyl)oxy]phenyl}ethyl methanesulfonate

Prepared in analogous manner to 4-(4-{[3-(hexahydro-1*H*-azepin-1-yl)propyl]oxy}phenyl)butyl methanesulfonate (for example, as prepared for Intermediate 35) using 2-{4-[(1-cyclobutyl-4-piperidinyl)oxy]phenyl}ethanol (for example, as prepared for Intermediate 55) (0.070 g, 0.25 mmol) to give *the title compound* (0.089 g). LCMS RT = 2.11 min, ES+ve *m*/*z* 354 (M+H)⁺.

### Intermediate 57

### 1,1-Dimethylethyl 4-[(4-iodophenyl)oxy]-1-piperidinecarboxylate

Sodium hydride (60% w/w dispersion in mineral oil, 3 g, 75 mmol) was added portionwise to a stirring solution of *t*-butyl 4-hydroxy-1-piperidinecarboxylate (commercially available, for example from Aldrich) (10.064 g, 50 mmol) in N-methyl-2-pyrrolidinone (45 ml). After the initial effervescence had ceased, 1-fluoro-4-iodobenzene (commercially available, for example from Aldrich) (11.1 g, 50 mmol) was added and the resulting mixture was heated at 80 °C under a nitrogen atmosphere for 20 h. The mixture was partitioned between EtOAc (500 ml) and water (400 ml). The organic phase was washed with water (2 × 400 ml), dried over anhydrous MgSO₄ and evaporated to give the *title compound* (19.56 g). LCMS RT = 3.86 min, ES+ve *m*/*z* 404 [M+H]⁺.

### Intermediate 58

### 4-[(4-Iodophenyl)oxy]piperidine trifluoroacetate

A solution of 1,1-dimethylethyl 4-[(4-iodophenyl)oxy]-1-piperidinecarboxylate (for example, as prepared for Intermediate 57) (4 g, 9.9 mmol) in DCM (90 ml) was treated with TFA (17 ml). The resulting mixture was stirred at room temperature for 2 h. The solvent was evaporated *in vacuo* to give *the title compound* (3.85 g). LCMS RT = 2.21 min, ES+ve *m*/*z* 304 (M+H)⁺.

### Intermediate 59

### 1-Cyclobutyl-4-[(4-iodophenyl)oxy]piperidine

To a stirring solution of 4-[(4-iodophenyl)oxy]piperidine trifluoroacetate (for example, as prepared for Intermediate 58) (3.85 g, 9.23 mmol) in DCM (50 ml) with acetic acid (0.57 ml), was added cyclobutanone (commercially available, for example, from Aldrich) (2.22 ml, 29.7 mmol) followed by sodium triacetoxyborohydride (3.15 g, 14.8 mmol). The mixture was stirred at room temperature under nitrogen for about 17 h, before further portions of sodium triacetoxyborohydride (1.57 g, 7.4 mmol) and cyclobutanone (1.11 ml, 14.8 mmol) were added. The mixture was stirred for a further 2 h, and then partitioned between DCM (70 ml) and NaOH solution (2 N, 100 ml). The organic layer was separated and washed with HCI (2 N, 2 × 100 ml), dried over sodium sulfate and the solvent evaporated to give a beige solid. The compound was further purified on two SCX ion exchange cartridges (2 × 20 g). The cartridges were eluted with MeOH, followed by aq. ammonia in MeOH (1:10). The basic fractions were concentrated in *vacuo* to afford the *title compound* (2.066 g). LCMS RT = 2.38 min, ES+ve *mlz* 358 (M+H)⁺.

### Intermediate 60

### 4-(4-[(1-Cyclobutyl-4-piperidinyl)oxy]phenyl)-3-butyn-1-ol

3-Butyn-1-ol (commercially available, for example, from Aldrich) (0.284 ml, 3.74 mmol) was dissolved in THF (15 ml) and NEt₃ (0.659 ml, 4.67 mmol) and the solution stirred at room temperature for 30 seconds with a stream of nitrogen flowing through it. The catalysts, bis(triphenylphosphine) palladium (II) chloride (30 mg) and copper (I) iodide (20 mg) were added followed by 1-cyclobutyl-4-[(4-iodophenyl)oxy]piperidine (for example, as prepared for Intermediate 59) (0.670 g, 1.87 mmol). After stirring at room temperature under nitrogen for about 17 h the solid was removed by filtration and washed with MeOH. The filtrate was concentrated *in vacuo* to remove the solvents then purified further by SCX ion exchange (20 g cartridge). The column was eluted with MeOH (5 × 50 ml) followed by (5 × 50 ml) aq. ammonia in MeOH (1:10). The basic fractions were concentrated *in vacuo* and the residue was re-purified on SCX ion exchange cartridge exactly as above to give *the title compound* (0.525 g). LCMS RT = 2.06 min, ES+ve *mlz* 300 (M+H)⁺.

### Intermediate 61

### 4-{4-[(1-Cyclobutyl-4-piperidinyl)oxy]phenyl}-1-butanol

4-{4-[(1-Cyclobutyl-4-piperidinyl)oxy]phenyl}-3-butyn-1-ol (for example, as prepared for Intermediate 60) (0.52 g, 1.74 mmol) was dissolved in a mixture of MeOH-EtOH (1:9, 50 ml) and acetic acid (0.2 ml, 3.48 mmol) and hydrogenated over 10 wt% Pd/C (130 mg) for about 17 h at room temperature. The catalyst was removed by filtration through a 10 g celite cartridge. The filtrate was concentrated *in vacuo* and the residue was purified on SCX ion exchange cartridge (10 g), the cartridge was eluted with MeOH (5 × 45 ml), followed by (4 × 45 ml) 2 N ammonia in MeOH solution. The basic fractions were concentrated *in vacuo* to afford *the title compound* (0.51 g). LCMS RT = 2.12 min, ES+ve *m*/*z* 304 (M+H)⁺.

### Intermediate 62

### 4-{4-[(1-Cyclobutyl-4-piperidinyl)oxy]phenyl}butyl methanesulfonate

Prepared in analogous manner to 4-(4-{[3-(hexahydro-1*H*-azepin-1-yl)propyl]oxy}phenyl)butyl methanesulfonate (for example, as prepared for Intermediate 35) using 4-{4-[(1-cyclobutyl-4-piperidinyl)oxy]phenyl}-1-butanol (for example, as prepared for Intermediate 61) (0.063 g, 0.2 mmol) to give *the title compound* (0.085 g). LCMS RT = 2.38 min, ES+ve *m*/*z* 381 (M+H)⁺.

### Intermediate 63

### 4-[4-(Methyloxy)phenyl]butyl methanesulfonate

To a cooled, 0 °C, solution of 4-[4-(methyloxy)phenyl]-1-butanol (commercially available, for example, from Aldrich) (0.36 g, 2 mmol) and NEt₃ (1.39 ml, 10 mmol) in dry diethyl ether (10 ml) under nitrogen was added, dropwise, methanesulfonyl chloride (0.46 ml, 6 mmol). After stirring at room temperature for 4 h, the reaction mixture was separated between diethyl ether and water. The organic phase was washed with water, saturated sodium bicarbonate solution and dried over anhydrous MgSO₄. The solvent was removed *in vacuo* to afford the *title compound* as a colourless oil (0.52 g). ¹H NMR (400 MHz, CDCl₃) δ 7.10 (d, J = 8.8 Hz, 2H), 6.84 (d, J = 8.8 Hz, 2H), 4.24 (t, J = 6.3 Hz, 2H), 3.80 (s, 3H), 2.99 (s, 3H), 2.61 (t, J = 7 Hz, 2H), 1.82-1.68 (m, 4H).

### Intermediate 64

### 4-[(4-Chlorophenyl)methyl]-2-[((2R)-1-{4-[4-(methyloxy)phenyl]butyl}-2-pyrrolidinyl)methyl]-1-(2H)-phthalazinone

To a solution of 4-[(4-chlorophenyl)methyl]-2-[(2*R*)-2-pyrrolidinylmethyl]-1(2*H*)-phthalazinone (for example, as prepared for Intermediate 4) (35.35 g, 100 mmol) in 2-butanone (250 ml) under nitrogen was added 4-[4-(methyloxy)phenyl]butyl methanesulfonate (for example, as prepared for Intermediate 63) (31 g, 120 mmol) and potassium carbonate (27.6 g, 200 mmol). The reaction mixture was heated at reflux for 24 h. The solid was removed by filtration and washed with 2-butanone (3 × 100 ml). The combined filtrate and washings were evaporated *in vacuo* and the residue was dissolved in DCM (70 ml). This was applied to a silica Biotage cartridge (800 g) and eluted with DCM (5000 ml) and then 5% MeOH in DCM (5000 ml). The required fractions were evaporated *in vacuo* and dissolved in DCM (70 ml). This was applied to aminopropyl cartridges (8 × 70 g) and eluted with a gradient of 0 - 100% DCM in cyclohexane over 30 min. The required fractions were combined and evaporated *in vacuo* to afford *the title compound* as a pale brown oil (30.96 g). LCMS RT = 2.95 min, ES+ve *mlz* 516/518 [M+H]⁺.

### Intermediate 65

### 4-[(4-Chlorophenyl)methyl]-2-({(2R)-1-[4-(4-hydroxyphenyl)butyl]-2-pyrrolidinyl}methyl)-1(2H)-phthalazinone

To a cooled -60 °C solution of 4-[(4-chlorophenyl)methyl]-2-[((2*R*)-1-{4-[4-(methyloxy)phenyl]butyl}-2-pyrrolidinyl)methyl]-1(2*H*)-phthalazinone (for example, as prepared for Intermediate 64) (24.35 g, 47 mmol) in dry DCM (100 ml) under nitrogen was added, dropwise, a 1.0 M solution of boron tribromide in DCM (52 ml, 52 mmol). The reaction mixture was allowed to warm to room temperature and stirred under nitrogen for 18 h. The reaction mixture was cooled in an ice/water bath and then quenched using 2 N hydrochloric acid (50 ml). The reaction mixture was basified using saturated sodium bicarbonate and extracted using DCM (500 ml). The separated organic phase was dried over anhydrous MgSO₄ and evaporated *in vacuo* to afford *the title compound* as an orange foam (22.04 g). LCMS RT = 2.80 min, ES+ve *m*/*z* 502/504 [M+H] ⁺.

### Intermediate 66

### 4-[(4-Chlorophenyl)methyl]-2-{[(2R)-1-(4-{4-[(3-chloropropyl)oxy]phenyl}butyl)-2-pyrrolidinyl]methyl}-1(2H)-phthalazinone

To a solution of 4-[(4-chlorophenyl)methyl]-2-({(2*R*)-1-[4-(4-hydroxyphenyl)butyl]-2-pyrrolidinyl}methyl)-1(2*H*)-phthalazinone (for example, as prepared for Intermediate 65) (22.03 g, 44 mmol) in 2-butanone (220 ml) under nitrogen was added 1-bromo-3-chloropropane (commercially available, for example, from Aldrich) (5.2 ml, 53 mmol) and potassium carbonate (12.2 g, 88 mmol). The reaction mixture was heated at reflux under nitrogen for 18 h. The solid was removed by filtration and washed with 2-butanone (200 ml). The combined filtrate and washings were evaporated in *vacuo* and the residue was dissolved in DCM (60 ml). This was applied to a silica cartridge (330 g) and eluted with a gradient of 0 - 25% MeOH in DCM over 12 CV. The required fractions were evaporated in *vacuo.* A portion of the residue (19.65 g) was dissolved in 2-butanone (200 ml) under nitrogen and to this was added 1-bromo-3-chloropropane (4.65 ml, 47 mmol) and potassium carbonate (10.8 g, 78.4 mmol). The reaction mixture was heated at reflux under nitrogen for 18 h. More 1-bromo-3-chloropropane (1 ml) was added and the reaction mixture was heated at reflux for a further 5 h. The solid was removed by filtration and washed with 2-butanone (3 × 100 ml). The combined filtrate and washings were evaporated *in vacuo* and the residue was dissolved in DCM (50 ml). This was applied to a silica cartridge (330 g) and eluted with a gradient of 0 - 10% MeOH in DCM over 12 CV. The required fractions were evaporated *in vacuo* to afford *the title compound* as a brown oil(21.68 g). LCMS RT = 3.18 min, ES+ve *m*/*z* 578/580 [M+H]⁺.

### Intermediate 67

### 4-[(4-Chlorophenyl)methyl]-2-[((2S)-1-{4-[4-(methyloxy)phenyl]butyl}-2-pyrrolidinyl)methyl]-1(2H)-phthalazinone

To a suspension of 4-[(4-chlorophenyl)methyl]-2-[(2*S*)-2-pyrrolidinylmethyl]-1(2*H*)-phthalazinone (for example, as prepared for Intermediate 6) (0.457 g, 1.29 mmol) in 2-butanone (25 ml) was added 4-[4-(methyloxy)phenyl]butyl methanesulfonate (0.52 g, 2 mmol) and potassium carbonate (0.276 g, 2 mmol). The reaction mixture was heated at reflux for 20 h. The solid was removed by filtration and washed with 2-butanone (20 ml). The combined filtrate and washings were evaporated *in vacuo* and the residue was dissolved in DCM (3 ml). This was applied to an aminopropyl cartridge (10 g) and eluted with a gradient of 0 - 100% DCM in cyclohexane over 40 min. The required fractions were combined and evaporated *in vacuo* to afford *the title compound* (0.23 g). LCMS RT = 2.99 min, ES+ve *m*/*z* 516/518 [M+H]⁺.

### Intermediate 68

### 4-[(4-Chlorophenyl)methyl]-2-({(2S)-1-[4-(4-hydroxyphenyl)butyl]-2-pyrrolidinyl}methyl)-1(2H)-phthalazinone

To a cooled, -60 °C solution of 4-[(4-chlorophenyl)methyl],-2-[((2*S*)-1-{4--[4-(methyloxy)phenyl]butyl}-2-pyrrolidinyl)methyl]-1(2*H*)-phthalazinone (for example, as prepared for Intermediate 67) (0.23 g, 0.45 mmol) in dry DCM (5 ml) was added, dropwise, a 1.0 M solution of boron tribromide in DCM (2.5 ml, 2.5 mmol). The reaction mixture was allowed to warm to room temperature and stirred under nitrogen for 18 h. The reaction mixture was cooled and then quenched using 2 N hydrochloric acid (5 ml). The reaction mixture was basified using saturated sodium bicarbonate and extracted using DCM (20 ml). The separated organic phase was dried over anhydrous MgSO₄ and evaporated *in vacuo* to afford the *title compound* (0.20 g). LCMS RT = 2.79 min, ES+ve *m*/*z* 502/504 [M+H]⁺.

### Intermediate 69

### 4-[(4-Chlorophenyl)methyl]-2-{[(2S)-1-(4-{4-[(3-chloropropyl)oxy]phenyl}butyl)-2-pyrrolidinyl]methyl}-1(2H)-phthalazinone

To a solution of 4-[(4-chlorophenyl)methyl]-2-({(2*S*)-1-[4-(4-hydroxyphenyl)butyl]-2-pyrrolidinyl}methyl)-1(2*H*)-phthalazinone (for example,as prepared for intermediate 68) (0.22 g, 0.44 mmol) in 2-butanone (10 ml) under nitrogen was added 1-bromo-3-chloropropane (0.052 ml, 0.53 mmol) and potassium carbonate (0.122 g, 0.83 mmol). The reaction mixture was heated at reflux for 18 h. More 1-bromo-3-chloropropane (0.052 ml, 0.53 mmol) was added and the reaction mixture was heated at reflux for a further 5 h. The solid was removed by filtration and washed with 2-butanone (20 ml). The combined filtrate and washings were evaporated *in vacuo* and the residue was dissolved in DCM (2 ml). This was applied to a silica cartridge (10 g) and eluted with a gradient of 0-10% MeOH in DCM over 20 min. The required fractions were evaporated *in vacuo* to afford *the title compound* (0.149 g). LCMS RT = 3.20 min, ES+ve *m*/*z* 578/580/582 [M+H]⁺.

### Intermediate 70

### 1,1-Dimethylethyl (4-chlorophenyl)acetate

(4-Chlorophenyl)acetic acid (commercially available, for example from Aldrich) (13.76 g, 81 mmol) was suspended in toluene (100 ml) under nitrogen. To this was added di-*tert-*butyl dimethylacetal (commercially available, for example, from Aldrich) (50 ml) and the reaction mixture was heated at 80 °C for 18 h. The solvent was removed *in vacuo* and the residue was dissolved in EtOAc (200 ml). The solution was washed with saturated sodium bicarbonate solution (2 × 200 ml) and brine (2 × 200 ml). The organic phase was dried over anhydrous MgSO₄ and evaporated *in vacuo* to give *the title compound* (6.68 g, 36%) as pale brown oil. ¹H NMR (CDCl₃) 7.28 (2H, d, J 8.5 Hz), 7.19 (2H, d, J 8.5 Hz), 3.48 (2H, s), 1.43 (9H, s).

### Intermediate 71

### 4-[(Methyloxy)carbonyl]-3-pyridinecarboxylic acid

To a suspension of pyridine-3,4-dicarboxylic acid anhydride (commercially available, for example from Aldrich) (26.73 g, 180 mmol) in dry THF (250 ml) at -70 °C under nitrogen was added a suspension of sodium methoxide (11.2 g, 2.01 mol) in dry MeOH (50 ml). The reaction mixture was allowed to warm to room temperature and stirred for 18 h. The solvents were removed *in vacuo* and the residue was dissolved in water (350 ml). This was acidified to -pH 2 using concentrated hydrochloric acid. The resultant solid was collected by filtration and washed with water. The solid was dried *in vacuo* at 45 °C to give *the title compound* (14.6 g, 45%) as a white solid. LCMS RT = 0.98 min, ES+ve *mlz* 182 (M+H)⁺.

### Intermediate 72

### Methyl 3-(2-(4-chlorophenyl)-3-[(1,1-dimethylethyl)oxy]-3-oxopropanoyl)-4-pyridine carboxylate

To a solution of 4-[(methyloxy)carbonyl]-3-pyridinecarboxylic acid (for example, as prepared for Intermediate 71) (1.81 g, 10 mmol) in dry DMF (90 ml) under nitrogen was added carbonyl diimidazole (1.7 g, 10.5 mmol). The reaction mixture was heated at 50 °C for 90 min and then cooled to -5 °C in a salt/ice bath. To this was added 1,1-dimethylethyl 4-chlorophenylacetate (for example, as prepared for Intermediate 70) (2.38 g, 10.5 mmol), followed by portionwise addition of sodium hydride (1.4 g of 60% dispersion in mineral oil, 35 mmol) over 15 min. The reaction mixture was stirred at -5 °C for 10 min and then warmed to room temperature. After 2 h the reaction mixture was poured into a saturated solution of ammonium chloride (100 ml). This was extracted using EtOAc (3 × 100 ml). The combined organics were washed with water (2 × 100 ml) and brine (2 × 100 ml). The organic phase was dried (MgSO₄) and the solvent removed *in vacuo*. The residue was dissolved in DCM (5 ml and applied to a 100 g silica cartridge. This was eluted using a gradient of 0-50% EtOAc in cyclohexane over 60 min. The required fractions were evaporated *in vacuo* to give *the title compound* (2.94 g, 75%, mixture of ketone and enol purity 99%) as a pale brown oil. LCMS RT = 3.41 and 3.63 (U-shaped peak) min ES+ve *m*/*z* 390/392 (M+H)⁺.

### Intermediate 73

### Methyl 3-[(4-chlorophenyl)acetyl]-4-pyridinecarboxylate

Methyl 3-{2-(4-chlorophenyl)-3-[(1,1-dimethylethyl)oxy]-3-oxopropanoyl}-4-pyridine carboxylate (for example, as prepared for Intermediate 72) (2.94 g, 7.5 mmol) was dissolved in dry DCM (12 ml) and to this was added TFA (5 ml). The reaction mixture was stirred at room temperature under nitrogen for 20 h. The solvent was removed *in vacuo* and the residue was dissolved in DCM (5 ml). This was applied to a 100 g silica cartridge and eluted with a gradient of 0-100% EtOAc in cyclohexane over 60 min. The required fractions were combined and evaporated *in vacuo* to give *the title compound* (1.59 g, 73%) as a pale orange oil. LCMS RT = 3.02 min ES+ve *m*/*z* 290/292 (M+H)⁺.

### Intermediate 74

### 4-[(4-Chlorophenyl)methyl]pyrido[3,4-d]pyridazin-1(2H)-one

Methyl 3-[(4-chlorophenyl)acetyl]-4-pyridinecarboxylate (for example, as prepared for Intermediate 73) (1.59 g, 5.5 mmol) was dissolved in EtOH (60 ml) and to this was added hydrazine hydrate (commercially available, for example from Aldrich) (0.3 ml, 6 mmol) and a few drops of AcOH. The reaction mixture was heated at reflux for 3 h. The reaction mixture was allowed to cool and the solid was collected by filtration and washed with EtOH (10 ml). The solid was dried *in vacuo* to give *the title compound* (1.17 g, 78%) as a white solid. LCMS RT = 2.73 min, ES+ve *m*/*z* 272/274 (M+H)⁺.

### Intermediate 75

### 4-[(4-Chlorophenyl)methyl]-2-[(2R)-2-pyrrolidinylmethyl]pyrido[3,4-d]pyridazin-1(2H)-one

To a solution of triphenylphosphine (10.42 g, 40 mmol) in anhydrous THF (80 ml) at -10 °C was added a solution of di-*tert*-butyl azodicarboxylate (8.38 g, 36 mmol) in anhydrous THF (60 ml). The solution was allowed to warm to 15 °C and then cooled to 0-5 °C. To the slight suspension was added a suspension of 4-[(4-chlorophenyl)methyl]pyrido[3,4-d]pyridazin-1(2*H*)-one (for example, as prepared for Intermediate 74) and *N*-Boc-D-prolinol (commercially available, for example from Aldrich) (5.14 g, 25.6 mmol) in anhydrous THF (100 ml). The suspension was allowed to warm to ambient temperature and stirred for 23 h. The solvent was removed *in vacuo* to leave an oil (30 g). LCMS RT = 3.48 min, ES+ve *m*/*z* 455/457. To a solution of the crude product (30 g) in 1,4-dioxane (80 ml) was added 4.0 M HCI in 1,4-dioxane (80 ml, 320 mmol). The solution was stirred at ambient temperature for 5 h. The solvent was removed *in vacuo* and the residue was partitioned between 1 M aq. hydrochloric acid (400 ml) and EtOAc (200 ml). The phases were separated and the aq. phase washed with EtOAc (200 ml). The combined organic extracts were washed with 1 M aq. hydrochloric acid (200 ml). The combined aqueous extracts were basified to pH 10 using 2M aq. NaOH (300-350 ml) and the resulting suspension extracted with EtOAc (2 × 400 ml, 1 × 200 ml). The combined organic extracts were concentrated *in vacuo* to leave *the title compound* (8.0 g). LCMS RT = 2.15 min, ES+ve *m*/*z* 355/357 (M+H)⁺.

### Examples

### Example 1

### 4-[(4-Chlorophenyl)methyl]-2-(2-{[4-(4-{[3-(hexahydro-1H-azepin-1-yl)propyl]oxy}phenyl)butyl]amino}ethyl)-1(2H)-phthalazinone

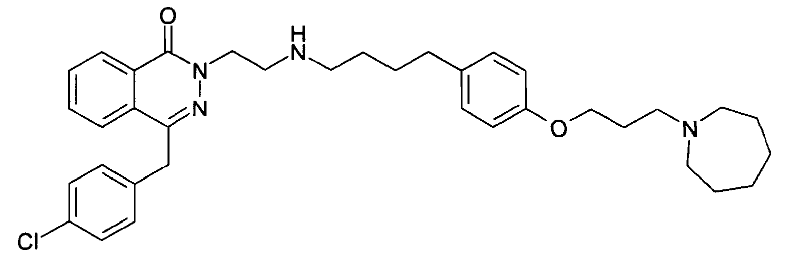

A mixture of 2-(2-aminoethyl)-4-[(4-chlorophenyl)methyl]-1(2*H*)-phthaiazinone (for example, as prepared for Intermediate 2) (190 mg, 0.606 mmol), 4-(4-{[3-(hexahydro-1*H-*azepin-1-yl)propyl]oxy}phenyl)butyl methanesulfonate (for example, as prepared for Intermediate 35) (115 mg, 0.300 mmol), and sodium bicarbonate (50 mg, 0.595 mmol) in MeCN (10 ml) was heated at 80 °C with stirring for 3 days. The cooled reaction mixture was partitioned between water and EtOAc. The aqueous layer was washed with further EtOAc (x2). The combined organic extracts were dried (MgSO₄) and concentrated in *vacuo.* The residue was purified by chromatography on silica (10 g, eluted with DCM-MeOH-aq. ammonia, 200:8:1 then 100:8:1). Appropriate fractions were concentrated separately *in vacuo* to afford impure product. Further purification of one portion by chromatography on silica (1 g, eluted with DCM-MeOH-aq. ammonia, 200:8:1), followed by concentration of the appropriate fraction, gave *the title compound* (5.7 mg). LCMS RT = 2.38 min, ES+ve *m*/*z* 601 [M+H]⁺ and 301 [M/2 +H]⁺. Further purification of a second portion obtained from the first purification, by chromatography on silica (5 g, eluted with DCM-MeOH-aq. ammonia, 200:8:1 then 150:8:1), followed by concentration of the appropriate fractions, gave additional quantities of *the title compound* (32 mg).

### Example 2

### 4-[(4-Chlorophenyl)methyl]-2-(2-{[2-(4-{[3-(hexahydro-1H-azepin-1-yl)propyl]oxy}phenyl)ethyl]amino}ethyl)-1(2H)-phthalazinone

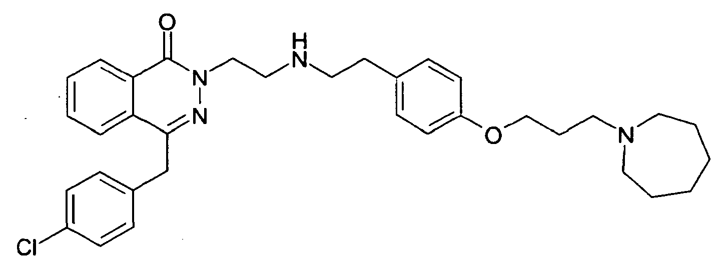

A mixture of 2-(2-aminoethyl)-4-[(4-chlorophenyl)methyl]-1(2*H*)-phthalazinone (for example, as prepared for Intermediate 2) (190 mg, 0.606 mmol), 2-(4-{[3-(hexahydro-1*H-*azepin-1-yl)propyl]oxy}phenyl)ethyl, methanesulfonate (for example, as prepared for Intermediate 27) (112 mg, 0.315 mmol), and sodium bicarbonate (50 mg, 0.595 mmol) in MeCN (10 ml) was heated at 80 °C with stirring for 3 days. The cooled reaction mixture was partitioned between water/brine (1:1) and EtOAc. The aqueous layer was washed with further EtOAc (x2). The combined organic extracts were dried (MgSO₄), and concentrated *in vacuo.* The residue was purified by preparative TLC (4 silica plates), eluted with DCM-MeOH-aq. ammonia (200:8:1), and extracted from the silica using MeOH to give *the title compound* (5.1 mg). LCMS RT = 2.41 min, ES+ve *mlz* 573/575 [M+H]⁺ and 287 [M/2 +H]⁺. From the preparative TLC, further quantities of *the title compound* were obtained (12 mg).

### Example 3

### 4-[(4-Chlorophenyl)methyl]-2-{2-[[4-(4-{[3-(hexahydro-1H-azepin-1-yl)propyl]oxy}phenyl)butyl](methyl)amino]ethyl}-1(2H)-phthalazinone

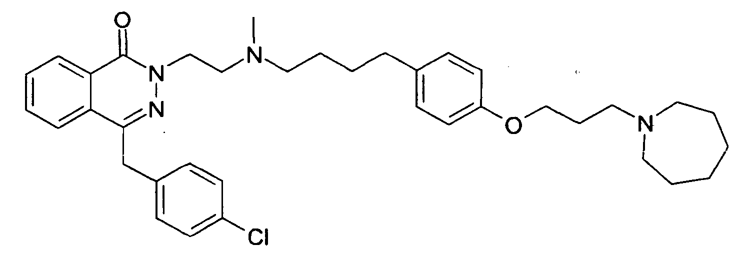

4-[(4-Chlorophenyl)methyl]-2-(2-{[4-(4-{[3-(hexahydro-1*H*-azepin-1-yl)propyl]oxy}phenyl)butyl]amino}ethyl)-1(2*H*)-phthalazinone (for example, as prepared for Example 1) (16 mg, 0.027 mmol) was treated with formaldehyde (37 wt% in water, 2 ml) and formic acid (0.20 ml), and this mixture was heated at 100 °C with stirring for 40 min. After cooling, the mixture was concentrated in *vacuo.* The residue was then heated on a steam bath, under high vacuum for 2 h, to give *the title compound* (11 mg) without further purification. LCMS RT = 2.47 min, ES+ve *m*/*z* 615 [M+H]⁺ and 308/309 [M/2 +H]⁺.

### Example 4

### 4-[(4-Chlorophenyl)methyl]-2-{2-[[2-(4-{[3-(hexahydro-1H-azepin-1-yl)propyl]oxy}phenyl)ethyl](methyl)amino]ethyl}-1(2H)-phthalazinone

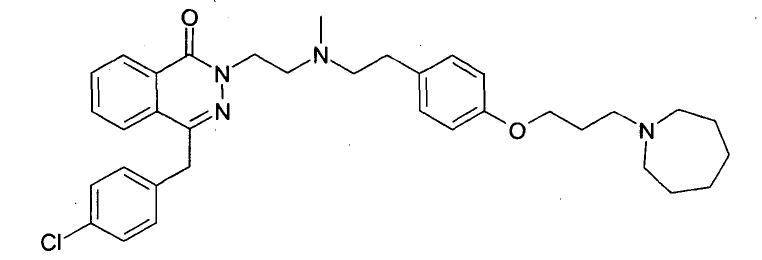

4-[(4-Chlorophenyl)methyl]-2-(2-{[2-(4-{[3-(hexahydro-1*H*-azepin-1-yl)propyl]oxy}phenyl) ethyl]amino}ethyl)-1(2*H*)-phthalazinone (for example, as prepared for Example 2) (12.3 mg, 0.021 mmol) was treated with formaldehyde (37 wt% in water, 2 ml) and formic acid (0.20 ml), and this mixture was heated at 100 °C with stirring for 1 h. After cooling, the mixture was concentrated *in vacuo.* The residue was then heated on a steam bath, under high vacuum for 2 h, to give *the title compound* (8.5 mg) without further purification. LCMS RT = 2.30 min, ES+ve *m*/*z* 587/589 [M+H]⁺ and 294/295 [M/2 +H]⁺.

### Example 5

### 4-[(4-Chlorophenyl)methyl]-2-({(2S)-1-[4-(4-{[3-(hexahydro-1H-azepin-1-yl)propyl]oxy}phenyl)butyl]-2-pyrrolidinyl}methyl)-1(2H)-phthalazinone

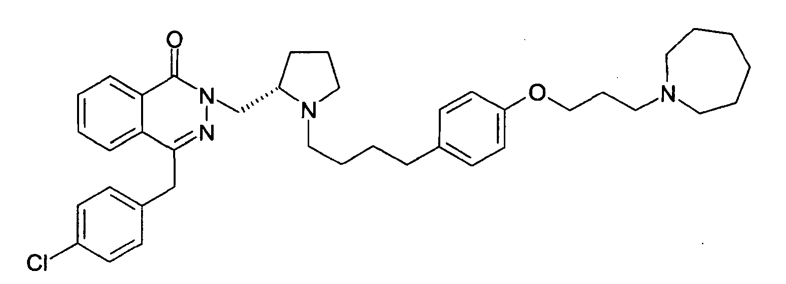

To a solution of 4-[(4-chlorophenyl)methyl]-2-{[(2*S*)-1-(4-{4-[(3-chloropropyl)oxy]phenyl}butyl)-2-pyrrolidinyl]methyl}-1(2*H*)-phthalazinone (for example, as prepared for Intermediate 69) (0.149 g, 0.26 mmol) in 2-butanone (5 ml) under nitrogen was added potassium iodide (commercially available, for example, from Aldrich) (0.086 g, 0.52 mmol), potassium carbonate (0.072 g, 0.52 mmol) and hexamethylene imine (0.059 ml, 0.52 mmol). The reaction mixture was heated at reflux for 41 h. The solid was removed by filtration and washed with 2-butanone (20 ml). The combined filtrate and washings were evaporated *in vacuo* and the residue was dissolved in MeOH-DMSO (2 ml, 1:1). This was applied to a C18 reverse phase cartridge (20 g) and eluted using a gradient of 0 - 50% MeCN (0.05% TFA) in water (0.05% TFA) over 40 min. The required fractions were evaporated *in vacuo* and the residue was dissolved in MeOH. This was applied to an amino propyl cartridge (10 g) and eluted with MeOH. The required fractions were evaporated *in vacuo* to afford *the title compound* (0.092 g). LCMS RT = 2.68 min, ES+ve *m*/*z* 641/643 [M+H]⁺. ¹H NMR (400 MHz, MeOD-*d*₄) δ 8.85 (m, 1H), 7.90 (m, 1H), 7.86-7.88 (m, 2H), 7.29-7.24 (m, 4H), 7.00 (d, J = 8.5 Hz, 2H), 6.77 (d, J = 8.5 Hz, 2H), 4.33 (dd, J = 4.8, 13 Hz, 1H), 4.30 (s, 2H), 4.11 (dd, J = 8,13 Hz, 1H), 3.93 (t, J = 6.3 Hz, 2H), 3.11 (m, 1H), 2.98 (m, 1H), 2.80 (m, 1H), 2.70-2.91 (m, 6H), 2.47 (m, 2H), 2.31 (m, 1H), 2.24 (m, 1H), 1.92 (m, 2H), 1.85-1.72 (m, 4H), 1.70-1.58 (m, 8H), 1.55-1.46 (m, 4H).

### Example 6

### 4-[(4-Chlorophenyl)methyl]-2-({(2R)-1-[5-(4-{[3-(hexahydro-1H-azepin-1-yl)propyl]oxy}phenyl)pentyl]-2-pyrrolidinyl}methyl)-1(2H)-phthalazinone

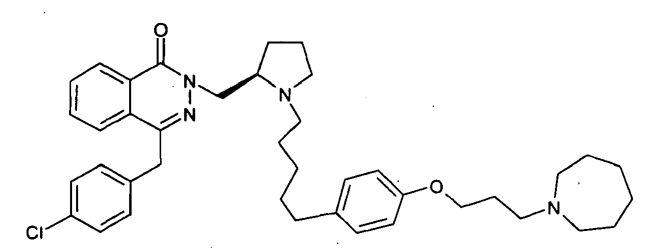

5-(4-{[3-(Hexahydro-1*H*-azepin-1-yl)propyl]oxy}phenyl)pentyl methanesulfonate (for example, as prepared for Intermediate 43) was stirred with 4-[(4-chlorophenyl)methyl]-2-[(2*R*)-2-pyrrolidinylmethyl]-1(2*H*)-phthalazinone (for example, as prepared for Intermediate 4) (126 mg, 0.36 mmol) in MeCN (10 ml) at 80 °C under nitrogen containing sodium bicarbonate (60 mg, 0.72 mmol) for six days when reaction appeared almost complete. The mixture was evaporated to dryness and the residue in DCM was loaded onto a 20 g silica cartridge which had been preconditioned with DCM. The cartridge was eluted with DCM-EtOH-0.88 aq. ammonia solution (200:8:1) and then (100:8:1) to give impure product in three fractions (52 mg, 74 mg and 25 mg). The 74 mg and 25 mg portions were combined and loaded onto 2 × 20 × 20 cm silica plates (1 mm thick layer) which were developed twice in DCM-EtOH-0.88 aq. ammonia solution (100:8:1). The main band was taken off and eluted to give the *title compound* (50 mg). LCMS RT = 2.58 min, ES+ve *m*/*z* 655 [M+H]⁺, ES+ve *mlz* 328 [½M+H]⁺.

### Example 7

### 2-({(2R)-1-[4-(4-{[3-(Hexahydro-1H-azepin-1-yl)propyl]oxy}phenyl)butyl]-2-pyrrolidinyl}methyl)-4-{[4-(methyloxy)phenyl]methyl}-1(2H)-phthalazinone diformate

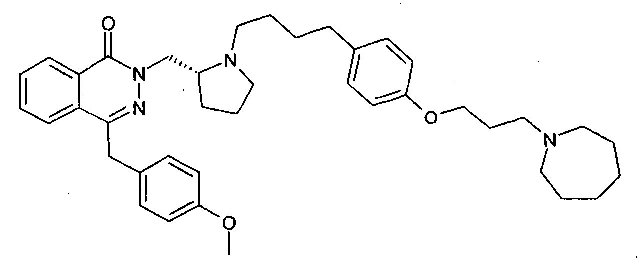

A solution of 4-{[4-(methyloxy)phenyl]methyl}-2-[(2*R*)-2-pyrrolidinylmethyl]-1(2*H*)-phthalazinone (for example, as prepared for Intermediate 10) (409 mg, 1.1 mmol) and 4-(4-{[3-(hexahydro-1*H*-azepin-1-yl)propyl]oxy}phenyl)butanal (for example, as prepared for Intermediate 40) (305 mg) in DCM (5 ml) and acetic acid (2 ml) was treated with sodium triacetoxyborohydride (0.42 g, 2 mmol) under nitrogen. The mixture was stirred at room temperature and then another portion of 4-(4-{[3-(hexahydro-1*H*-azepin-1-yl)propyl]oxy}phenyl)butanal (211 mg) and sodium triacetoxyborohydride (0.42 g, 2 mmol) were added. LCMS indicated a mixture of starting material and product (1:1). 1-[3-({4-[4,4-Bis(ethyloxy)butyl]phenyl}oxy)propyl]hexahydro-1*H*-azepine (for example, as prepared for Intermediate 38) (150 mg) was added to the reaction mixture and stiiring was continued for a further 3 days. The solvent was removed under reduced pressure and the residue was partitiond between EtOAc and aq. sodium bicarbonate solution. The organic solution was washed with aq. sodium bicarbonate solution, brine, dried (MgSO₄), and evaporated under reduced pressure. The residue (714 mg) was dissolved in DCM and purified by chromatography (Flashmaster II, 100 g silica cartridge) eluting with 0 - 15% MeOH containing 1 % NEt₃ - DCM over 60 min. Appropriate fractions were combined and evaporated under reduced pressure to give the free base of *the title compound* (335 mg). This was dissolved in MeOH-DMSO (2:1, 2.4 ml) and purified by MDAP HPLC to give the *title compound* (193 mg) LCMS RT = 2.37 min, ES+ve *mlz* 637 (M+H)⁺, 319 (M/2+H)⁺; ES-ve *m*/*z* 681 (M+HCO₂)⁻.

### Example 8

### 2-({(2R)-1-[4-(4-{[3-(Hexahydro-1H-azepin-1-yl)propyl]oxy}phenyl)butyl]-2-pyrrolidinyl}methyl)-4-[(4-hydroxyphenyl)methyl]-1(2H)-phthalazinone

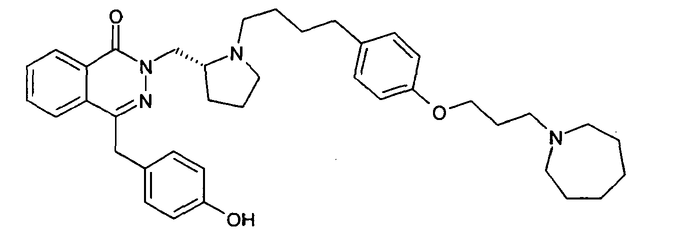

A solution of 2-({(2*R*)-1-[4-(4-{[3-(hexahydro-1*H*-azepin-1-yl)propyl]oxy}phenyl)butyl]-2-pyrrolidinyl}methyl)-4-{[4-(methyloxy)phenyl]methyl}-1(2*H*)-phthalazinone diformate (for example, as prepared for Example 7) (100 mg, 0.13 mmol) in DCM (10 ml) was cooled in an ice-bath under nitrogen and then treated with boron tribromide solution in hexanes (1 M, 0.3 ml), followed by another portion (0.3 ml) after 2 h. The mixture stood at room temperature for a total of 2 days and 4 h and then the solvents were removed under reduced pressure. The residue was dissolved in MeOH-DMSO (1:1, 2 ml) and purified by MDAP HPLC to give 18 mg which was re-purified by MDAP HPLC to give *the title compound* (13 mg) LCMS RT = 2.37 min, ES+ve *m*/*z* 623 (M+H)⁺, 312 (M/2+H)⁺.

### Example 9

### 4-[(4-Fluorophenyl)methyl]-2-({(2R)-1-[4-(4-{[3-(hexahydro-1H-azepin-1-yl)propyl]oxy}phenyl)butyl]-2-pyrrolidinyl}methyl)-1(2H)-phthalazinone

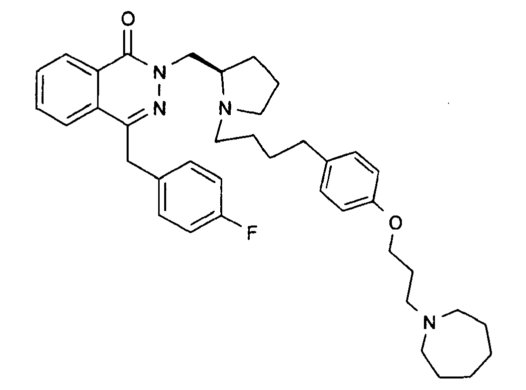

To a solution of the 4-chloro-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone (for example, as prepared for Intermediate 13) (0.055 g, 0.1 mmol) in anhydrous THF (4 ml) under nitrogen was added tetrakis(triphenylphosphine)palladium(0) (0.013 g, 0.01 mmol) and then 4-fluoro-benzyl zinc chloride (commercially available, for example, from Aldrich) (0.5 M, 0.6 ml), in THF at ambient temperature. The solution was heated to 60 °C for 1 h. To the mixture was added tetrakis(triphenylphosphine)palladium(0) (0.014 g, 0.01 mmol) and then 4-fluoro-benzyl zinc chloride in THF (0.5 M, 0.6 ml) at ambient temperature. The mixture was heated to 80 °C for 4 h. To the mixture was added more tetrakis(triphenylphosphine)palladium(0) (0.014 g, 0.01 mmol) and then 4-fluoro-benzyl zinc chloride in THF (0.5M, 2.5 ml) at ambient temperature. The mixture was heated to 80 °C for 2.5 h. The reaction was quenched with MeOH (2 ml). The mixture was applied to a SCX cartridge (20 g) and the cartridge washed with MeOH (2 CV). The cartridge was eluted with 10% 0.88 ammonia in MeOH (2 CV) and the basic fractions concentrated in *vacuo* to leave a gum (0.038 g). The crude was purified by MDAP (0.1 ml of TFA added prior to MDAP) to give a gum (0.009 g), which was applied to a SCX cartridge (5 g). The cartridge was eluted with 10% 0.88 ammonia in MeOH (2 CV) and the basic fractions concentrated *in vacuo* give *the title compound* (0.005 g). LCMS RT = 2.42 min, ES+ve *m*/*z* 625 (M+H)⁺ and 313 (M/2+H)⁺

### Example 10

### 4-[(4-Chlorophenyl)methyl]-2-{1-[(4-{[3-(hexahydro-1H-azepin-1-yl)propyl]oxy}phenyl)methyl]hexahydro-1H-azepin-4-yl}-1(2H)-phthalazinone

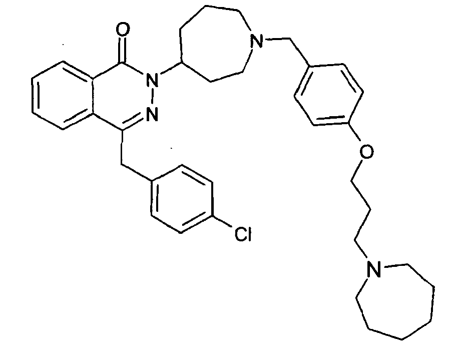

A solution of 4-[(4-chlorophenyl)methyl]-2-[1-({4-[(3-chloropropyl)oxy]phenyl}methyl)hexahydro-1*H*-azepin-4-yl]-1(2*H*)-phthalazinone (for example, as prepared for Intermediate 18) (15 mg, 0.027 mmol) in 2-butanone (1 ml) was treated with sodium iodide (10 mg), hexamethyleneimine (commercially available, for example, from Aldrich) (0.1 ml) and the mixture was heated under nitrogen to 75 °C for 2 h. More hexamethyleneimine (0.1 ml) was added and the mixture was heated at the same temperature overnight. The reaction mixture was concentrated under reduced pressure and the residue was dissolved in MeOH-DMSO (1:1; 1 ml) and purified by MDAP HPLC. Appropriate fractions were combined to give *the title compound* (10.7 mg) LCMS RT = 2.54 min ES+ve *m*/*z* 613 (M+H)⁺, 307 (M/2+H)⁺.

### Example 11

### 4-[(4-Chlorophenyl)methyl]-2-{1-[2-(4-{[3-(hexahydro-1H-azepin-1-yl)propyl]oxy}phenyl)ethyl]hexahydro-1H-azepin-4-yl}-1(2H)-phthalazinone

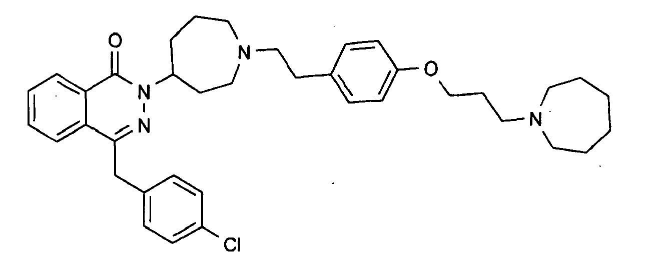

Prepared in analogous manner to 4-[(4-chlorophenyl)methyl]-2-[1-(2-{4-[(1-cyclobutyl-4-piperidinyl)oxy]phenyl}ethyl)hexahydro-1*H*-azepin-4-yl]-1(2*H*)-phthalazinone (Example 21) using 2-(4-{[3-(hexahydro-1*H*-azepin-1-yl)propyl]oxy}phenyl)ethyl methanesulfonate (for example, as prepared for Intermediate 27) (0.101 g, 0.284 mmol) to give *the title compound* (0.0044 g). LCMS RT = 2.54 min, ES+ve *mlz* 314 and 315 (M+H)⁺.

### Example 12

### 4-[(4-Chlorophenyl)methyl]-2-{1-[3-(4-{[3-(hexahydro-1H-azepin-1-yl)propyl]oxy}phenyl)propyl]hexahydro-1H-azepin-4-yl}-1(2H)-phthalazinone

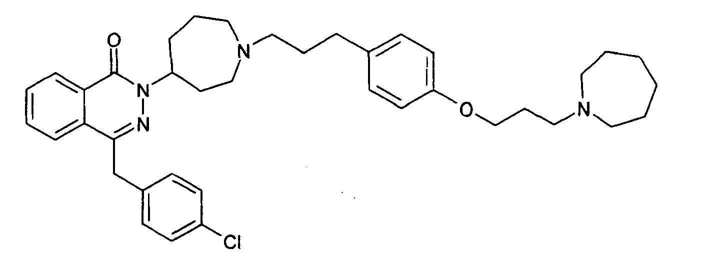

A mixture of 3-(4-{[3-(hexahydro-1*H*-azepin-1-yl)propyl]oxy}phenyl)propyl 4-methylbenzenesulfonate and 1-(3-{[4-(3-chloropropyl)phenyl]oxy}propyl)hexahydro-1*H-*azepine (3:2) (for example, as prepared for Intermediate 30) (42 mg, about 0.12 mmol of alkylating agent) was stirred with 4-[(4-chlorophenyl)methyl]-2-(hexahydro-1*H*-azepin-4-yl)-1(2*H*)-phthalazinone, (for example, as prepared for Intermediate 16) (33 mg, 0.09 mmol) in acetone (4 ml) containing sodium iodide (18 mg, 0.12 mmol) and potassium carbonate (70 mg) under reflux for three days. Reaction was incomplete so more sodium iodide (18 mg, 0.12 mmol) and potassium carbonate (70 mg) were added. After refluxing for a further day, reaction was judged sufficient to isolate product. The solid was removed by filtration and the filtrate was concentrated and loaded onto 2 × 20 × 20 cm silica plates (1 mm thick layer) which were developed in DCM-EtOH-0.880 aq. ammonia solution (50:8:1). The main band was eluted from the silica using MeOH-DCM (1:1) and the elute was evaporated to give the *title compound* (28 mg). LCMS RT = 2.52 min, ES+ve *m*/*z* 321 [½M+H]⁺

### Example 13

### 4-[(4-Chlorophenyl)methyl]-2-{1-[3-(4-{[3-(hexahydro-1H-azepin-1-yl)propyl]oxy}phenyl)propyl]hexahydro-1H-azepin-4-yl}-1(2H)-phthalazinone

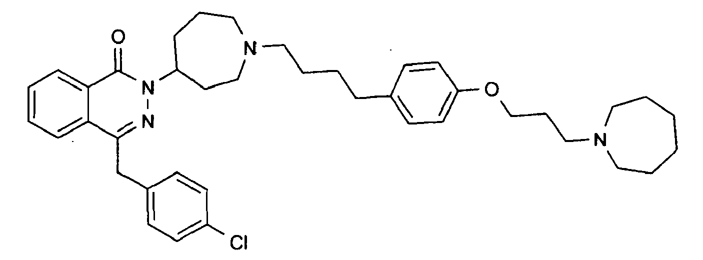

To a solution of 4-[(4-chlorophenyl)methyl]-2-(hexahydro-1*H*-azepin-4-yl)-1(2*H*)-phthalazinone (for example, as prepared for Intermediate 16) (0.072 g, 1.96 mmol) in anhydrous DCM (2 ml) was added DIPEA (0.041 ml, 2.3 mmol) and then 4-(4-{[3-(hexahydro-1*H*-azepin-1-yl)propyl]oxy}phenyl)butyl methanesulfonate (for example, as prepared for Intermediate 35) (0.090 g, 0.23 mmol). The solution was stirred at 20 °C for 26 h and then heated to 74 °C for 20 h. The mixture was applied to a SCX cartridge (20 g) and the cartridge washed with MeOH (2 CV). The cartridge was eluted with 10% 0.88 ammonia in MeOH (2 CV). The basic fractions were concentrated *in vacuo* and residue purified by MDAP. The appropriate fractions were combined and the solvent removed *in vacuo.* The residue was dissolved in DCM (5 ml). To the solution was added DIPEA (0.034 ml, 0.19 mmol) and then acetyl chloride (0.1 ml, 1.4 mmol). The solution was stirred at 20 °C for 15 - 20 min and then quenched with MeOH (1 ml). The solution was applied to a SCX cartridge (10 g, pre-washed with MeOH) and the cartridge washed with MeOH (2 CV). The cartridge was eluted with 10% 0.88 ammonia in MeOH (2 CV). The basic fractions were concentrated *in vacuo* to give *the title compound* (0.0323 g). LCMS RT = 2.58 min, ES+ve *m*/*z* 328 and 329 (M/2+H)⁺.

### Example 14

### 4-[(4-Chlorophenyl)methyl]-2-[((2S)-1-{3-[(3-cyclobutyl-2,3,4,5-tetrahydro-1H-3-benzazepin-7-yl)oxy]propyl}-2-pyrrolidinyl)methyl]-1(2H)-phthalazinone, diformate salt

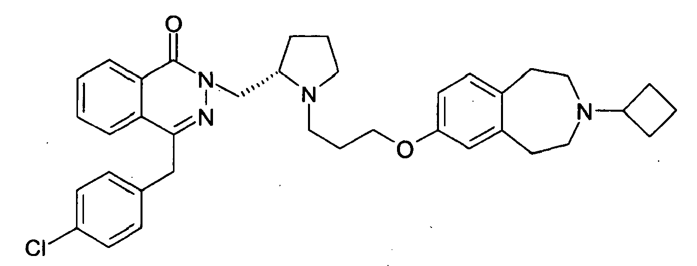

4-[(4-Chlorophenyl)methyl]-2-[(2*S*)-2-pyrrolidinylmethyl]-1(2*H*)-phthalazinone (for example, as prepared for Intermediate 6) (30 mg, 0.08 mmol), 3-[(3-cyclobutyl-2,3,4,5-tetrahydro-1*H*-3-benzazepin-7-yl)oxy]propyl methanesulfonate (for example, as prepared for Intermediate 45) (50 mg, 0.14 mmol), DIPEA (0.02 ml, 0.13 mmol), and sodium iodide (20 mg, 0.13 mmol) were combined in DMF (1.5 ml), and heated in a microwave oven at 150 °C for 20 min, then a further 20 min. The mixture was applied to an SCX cartridge (20 g), eluting with MeOH, and then a solution of 10% aq. ammonia in MeOH. The appropriate fraction was concentrated *in vacuo.* The crude material was purified by preparative TLC (2 silica plates), eluted with DCM-MeOH-aq. ammonia (100:8:1), and extracted from silica using DCM-EtOH-aq.ammonia (100:8:1). The material thus obtained was treated with formic acid, and then dried under nitrogen flow to give *the title compound* (2.4 mg) as the diformate salt. LCMS RT = 2.45 min, ES+ve *m*/*z* 611 [M+H]⁺ and 306 [M/2 +H]⁺.

### Example 15

### 4-[(4-Chlorophenyl)methyl]-2-[((2R)-1-{3-[(3-cyclobutyl-2,3,4,5-tetrahydro-1H-3-benzazepin-7-yl)oxy]propyl}-2-pyrrolidinyl)methyl]-1(2H)-phthalazinone

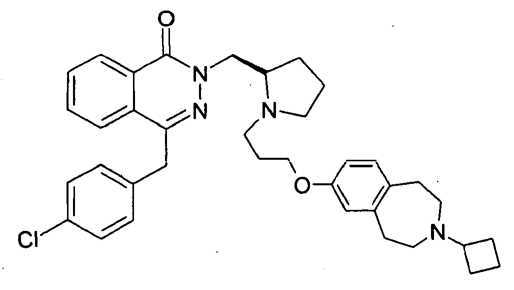

A suspension of 4-[(4-chlorophenyl)methyl]-2-[(2*R*)-2-pyrrolidinylmethyl]-1(2*H*)-phthalazinone (for example, as prepared for Intermediate 4) (32 mg, 0.09 mmol), 7-[(3-chloropropyl)oxy]-3-cyclobutyl-2,3,4,5-tetrahydro-1*H*-3-benzazepine, (for example, as prepared for Intermediate 46) (32 mg, 0.108 mmol), sodium iodide (16 mg, 0.108 mmol) and DIPEA (0.019 ml, 0.108 mmol) in dry DMF (1.5 ml) was heated to 150 °C for 15 min in a microwave oven and again for a further 20 min. The crude mixture was then poured onto an SCX-2 cartridge (20 g), washed with MeOH (× 2) and then eluted with 10% aq. ammonia in MeOH. The ammoniacal fractions were concentrated under reduced pressure and the resultant oil was purified by MDAP HPLC. The required fractions were concentrated and the resultant residue then dissolved in DCM (about 3 ml) and treated with DIPEA (0.007 ml) and acetyl chloride (0.003 ml, 0.04 mmol). The solution was stirred at 20 °C for 20 min and then quenched with MeOH (1 ml). The mixture was poured onto an SCX -2 cartridge (10 g) and eluted with 10% aq. ammonia in MeOH. The solvents were removed *in vacuo* and the resultant oil purified by MDAP HPLC. The resultant concentrated residue was then applied onto a preparative TLC plate and eluted with DCM-EtOH-aq. ammonia (100:8:1). The appropriate band was collected, washed with the same eluent and the combined filtrates were concentrated *in vacuo* to afford the *title compound* (4.5 mg). LCMS RT = 2.40 min. ES+ve *m*/*z* 611 [M+H]⁺, 306 [M/2 +H]⁺.

### Example 16

### 4-[(4-Chlorophenyl)methyl]-2-(1-{3-[(3-cyclobutyl-2,3,4,5-tetrahydro-1H-3-benzazepin-7-yl)oxy]propyl}hexahydro-1H-azepin-4-yl)-1(2H)-phthalazinone formate salt

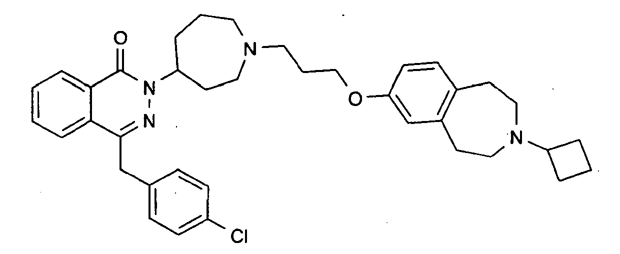

To a solution of 7-[(3-chloropropyl)oxy]-3-cyclobutyl-2,3,4,5-tetrahydro-1*H*-3-benzazepine (for example, as prepared for Intermediate 46) (0.095 g, 0.32 mmol) in DMF (3 ml) was added 4-[(4-chlorophenyl)methyl]-2-(hexahydro-1*H*-azepin-4-yl)-1(2*H*)-phthalazinone (for example, as prepared for Intermediate 16) (0.097 g, 0.26 mmol) and then DIPEA (0.056 ml, 0.32 mmol). The solution was stirred at 20 °C for 20 h. The solution was heated to 70 °C for 2 h. The solution was heated to 150 °C for 30 min in a microwave oven. The crude mixture was applied to a SCX cartridge (20 g, pre-washed with MeOH). The cartridge was washed with MeOH (2 CV) and then eluted with 10% 0.88 ammonia in MeOH (2 CV). The basic fractions were concentrated *in vacuo* and the residue (0.137 g) was further purified by MDAP HPLC to give *the title compound* (0.068 g, 40 %). LCMS RT = 2.48 min ES+ve *m*/*z* 625 (M+H)⁺, 313/314 (M/2+H)⁺.

### Example 17

### 4-[(4-chlorophenyl)methyl]-2-{1-[(3-cyclobutyl-2,3,4,5-tetrahydro-1H-3-benzazepin-7-yl)methyl]hexahydro-1H-azepin-4-yl}-1(2H)-phthalazinone diformate salt

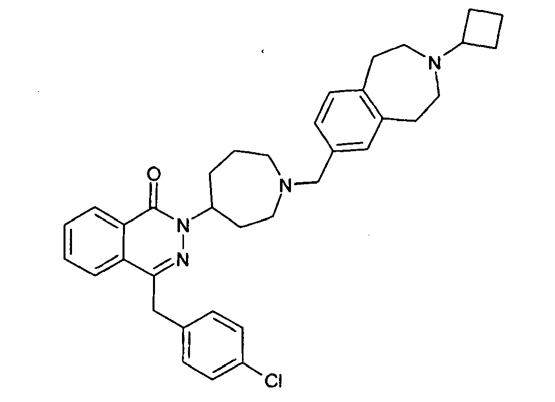

A mixture of 4-[(4-chlorophenyl)methyl]-2-(hexahydro-1*H*-azepin-4-yl)-1(2*H*)-phthalazinone (for example, as prepared for Intermediate 16) (36.7 mg, 0.1 mmol) and 3-cyclobutyl-2,3,4,5-tetrahydro-1*H*-3-benzazepine-7-carbaldehyde (for example, as prepared for Intermediate 48) (22.9 mg, 0.1 mmol) in DCM (1 ml) was treated with acetic acid (1 drop) and sodium triacetoxyborohydride (43 mg, 0.2 mmol) and the mixture was stirred under nitrogen overnight. More sodium triacetoxyborohydride (42.4 mg, 0:2 mmol) and DCM (1 ml) were added and after 2 h, more aldehyde (6 mg) was added. After a further 2 h the reaction mixture was partitioned between EtOAc (10 ml) and sodium bicarbonate solution (10 ml). The organic phase was washed with another 10 ml of sodium bicarbonate solution, dried over MgSO₄ and evaporated under reduced pressure. The residue (46.5 mg) was dissolved in DCM (1.5 ml) and treated with tosyl chloride (5 mg) and stirred over the weekend. The reaction mixture was evaporated under reduced pressure and the residue (50.6 mg) was dissolved in MeOH-DMSO (1:1; 1 ml) and purified by MDAP HPLC. Evaporation of the appropriate fractions gave the title compound (12.6 mg) LCMS RT = 2.40 min, ES+ve *m*/*z* 581 (M+H)⁺, 291 (M/2+H)⁺.

### Example 18

### N-(2-{4-[4-[(4-Chlorophenyl)methyl]-1-oxo-2(1H)-phthalazinyl]hexahydro-1H-azepin-1-yl}ethyl)-3-cyclobutyl-2,3,4,5-tetrahydro-1H-3-benzazepine-7-carboxamide diformate salt

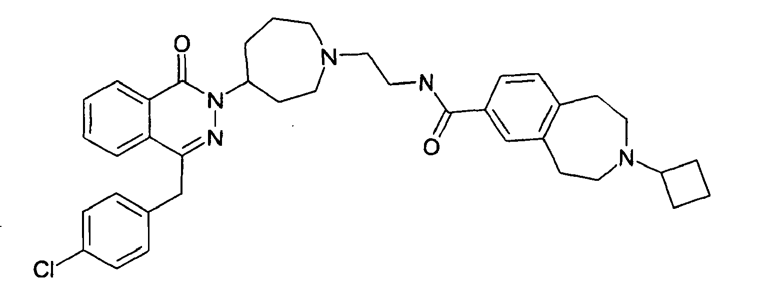

A mixture of 2-[1-(2-aminoethyl)hexahydro-1*H*-azepin-4-yl]-4-[(4-chlorophenyl)methyl]-1(2*H*)-phthalazinone hydrochloride salt (for example, as prepared for Intermediate 20) (69.6 mg), 3-cyclobutyl-2,3,4,5-tetrahydro-1*H*-3-benzazepine-7-carboxylic acid (for example, as prepared for Intermediate 51) (39.5 mg, 0.17 mmol) in DMF (1.02 ml) was treated with NEt₃ (0.24 ml) and TBTU (80.3 mg) and the mixture was stirred overnight at room temperature. The reaction mixture was applied to an SCX-2 cartridge (20 g) and washed with MeOH, and then eluted with 10% aq. ammonia in MeOH. The ammoniacal fractions were concentrated under reduced pressure to give a residue (243 mg) which was dissolved in MeOH-DMSO (1:1; 2.4 ml) and then purified by MDAP HPLC to give after evaporation of the appropriate fractions 87.5 mg. This residue was dissolved in MeOH and applied to an SCX-2 cartridge (10 g), washed with MeOH and then eluted with 10% aq. ammonia in MeOH. The ammoniacal fractions were evaporated under reduced pressure to give the free base of the title compound (33 mg). This was dissolved in MeOH and treated with formic acid (0.3 ml) and evaporated under reduced pressure to give the title compound (40.4 mg) LCMS RT = 2.38 min ES+ve *m*/*z* 638 (M+H)⁺, 320 (M/2+H)⁺.

### Example 19

### N-(3-{4-[4-[(4-chlorophenyl)methyl]-1-oxo-2(1H)-phthalazinyl]hexahydro-1H-azepin-1-yl}propyl)-3-cyclobutyl-2,3,4,5-tetrahydro-1H-3-benzazepine-7-carboxamide diformate salt

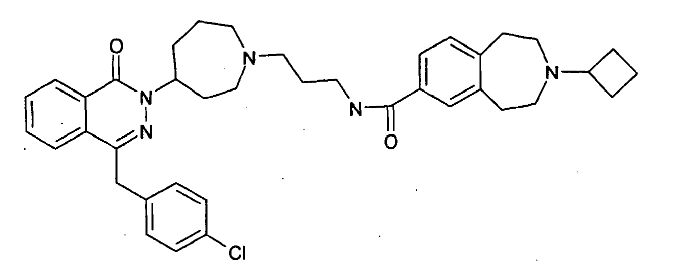

A mixture of 2-[1-(3-aminopropyl)hexahydro-1*H*-azepin-4-yl]-4--[(4-chlorophenyl)methyl]-1(2*H*)-phthalazinone hydrochloride salt (for example, as prepared for Intermediate 22) (114 mg), 3-cyclobutyl-2,3,4,5-tetrahydro-1*H*-3-benzazepine-7-carboxylic acid (for example, as prepared for Intermediate 51) (72.6 mg, 0.27 mmol) in DMF (1.62 ml) was treated with NEt₃ (0.35 ml) and TBTU (128 mg) and the mixture was stirred overnight at room temperature. The reaction mixture was applied to an SCX-2 cartridge (20 g) and washed with MeOH, and then eluted with 10% aq. ammonia in MeOH. The ammoniacal fractions were concentrated under reduced pressure to give a residue (253 mg) which was dissolved in MeOH-DMSO (1:1; 2.5 ml) and then purified by MDAP HPLC to give after evaporation of the appropriate fractions 99 mg. This residue was dissolved in MeOH and applied to an SCX-2 cartridge (10 g), washed with MeOH and then eluted with 10% aq. ammonia in MeOH. The ammoniacal fractions were evaporated under reduced pressure to give the free base of *the title compound* (55.7 mg). This was dissolved in MeOH and treated with formic acid (0.3 ml) and evaporated under reduced pressure to give *the title compound* (72.6 mg) LCMS RT = 2.37 min ES+ve *mlz* 652 (M+H)⁺, 327 (M/2+H)⁺.

### Example 20

### N-(4-{4-[4-[(4-Chlorophenyl)methyl]-1-oxo-2(1H)-phthalazinyl]hexahydro-1H-azepin-1-yl}butyl)-3-cyclobutyl-2,3,4,5-tetrahydro-1H-3-benzazepine-7-carboxamide diformate salt

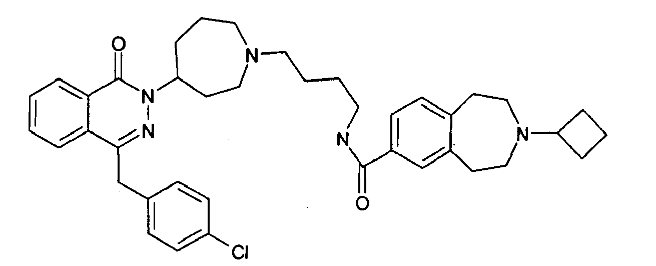

A mixture of 2-[1-(4-aminobutyl)hexahydro-1*H*-azepin-4-yl]-4-[(4-chlorophenyl)methyl]-1(2*H*)-phthalazinone hydrochloride salt (for example, as prepared for Intermediate 24) (108.8 mg), 3-cyclobutyl-2,3,4,5-tetrahydro-1*H*-3-benzazepine-7-carboxylic acid (for example, as prepared for Intermediate 51) (58 mg, 0.25 mmol) in DMF (1.5 ml) was treated with NEt₃ (0.5 ml) and TBTU (120.4 mg) and the mixture was stirred overnight at room temperature. The reaction mixture was applied to an SCX-2 cartridge (20 g) and washed with MeOH, and then eluted with 10% aq. ammonia in MeOH. The ammoniacal fractions were concentrated under reduced pressure to give a residue (217.9 mg) which was dissolved in MeOH-DMSO (1:1; 2.2 ml) and then purified by MDAP HPLC to give after evaporation of the appropriate fractions *the title compound* (81.9 mg) LCMS RT = 2.40 min ES+ve *m*/*z* 666 (M+H)⁺, 334 (M/2+H)⁺.

### Example 21

### 4-[(4-Chlorophenyl)methyl]-2-[1-(2-{4-[(1-cyclobutyl-4-piperidinyl)oxy]phenyl}ethyl)hexahydro-1H-azepin-4-yl]-1(2H)-phthalazinone

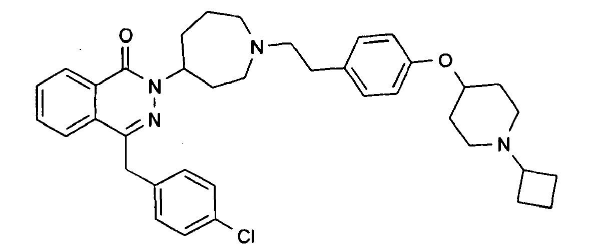

To a solution of 4-[(4-chlorophenyl)methyl]-2-(hexahydro-1*H*-azepin-4-yl)-1(2*H*)-phthalazinone (for examples, as prepared for Intermediate 16) (0.072 g, 0.196 mmol) in anhydrous DCM (2 ml) was added DIPEA (0.0434 ml, 0.244 mmol) and then 2-{4-[(1-cyclobutyl-4-piperidinyl)oxy]phenyl}ethyl methanesulfonate (for example, as prepared for Intermediate 56) (0.089 g, 0.25 mmol). The solution was stirred at 20 °C for 26 h and then heated to 74 °C for 20 h. The mixture was applied to a SCX cartridge (20 g) and the cartridge washed with MeOH (2 CV). The cartridge was eluted with 10% 0.88 ammonia in MeOH (2 CV). The combined basic fractions were concentrated *in vacuo* and residue purified by MDAP. The appropriate fractions were combined and the solvent removed in *vacuo.* The residue was dissolved in DCM (5 ml). To the solution was added DIPEA (0.020 ml, 0.11 mmol) and then acetyl chloride (0.1 ml, 1.4 mmol). The solution was stirred at 20 °C for 15 - 20 min and then quenched with MeOH (1 ml). The solution was applied to a SCX cartridge (10 g) and the cartridge washed with MeOH (2 CV). The cartridge was eluted with 10% 0.88 ammonia in MeOH (2 CV). The basic fractions were concentrated *in vacuo.* The residue was applied to a preparative plate (20 × 20 cm, 1 mm thickness) and eluted with 15:100:1 MeOH-DCM-NEt₃. The product obtained from the preparative plate was applied to a SCX cartridge (1g) and the cartridge washed with MeOH (2 CV). The cartridge was eluted with 10% 0.88 ammonia in MeOH (2 CV). The basic fractions were concentrated using a stream of nitrogen to give *the title compound* (0.0048g). LCMS RT = 2.51 min, ES+ve *m*/*z* 313 and 314 (M/2+H)⁺.

### Example 22

### 4-[(4-Chlorophenyl)methyl]-2-[1-(4-{4-[(1-cyclobutyl-4-piperidinyl)oxy]phenyl}butyl)hexahydro-1H-azepin-4-yl]-1(2H)-phthalazinone

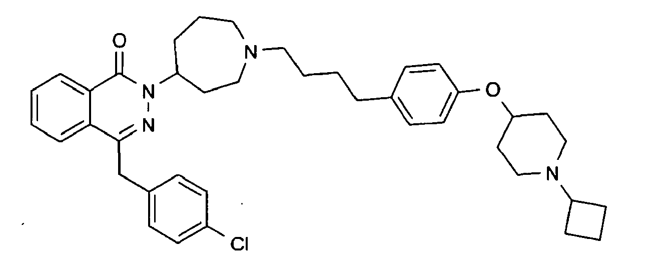

Prepared in analogous manner to 4-[(4-chlorophenyl)methyl]-2-[1-(2-{4-[(1-cyclobutyl-4-piperidinyl)oxy]phenyl}ethyl)hexahydro-1*H*-azepin-4-yl]-1(2*H*)-phthalazinone (Example 21) using 4-{4-[(1-cyclobutyl-4-piperidinyl)oxy]phenyl}butyl methanesulfonate (for example, as prepared for Intermediate 62) (0.0849 g, 0.22 mmol) to give *the title compound* (0.004 g). LCMS RT = 2.62 min, ES+ve *m*/*z* 327 and 328 (M/2+H)⁺.

### Example 23

### 4-[(4-Chlorophenyl)methyl]-2-({(2R)-1-[4-(4-{[3-(hexahydro-1H-azepin-1-yl)propyl] oxy}phenyl)butyl]-2-pyrrolidinyl}methyl)pyrido[3,4-d]pyridazin-1(2H)-one

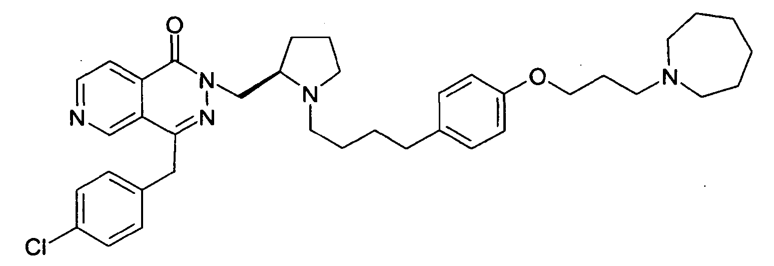

A mixture of 4-[(4-chlorophenyl)methyl]-2-[(2*R*)-2-pyrrolidinylmethyl]pyrido[3,4-*d*]pyridazin-1(2*H*)-one (for example, as prepared for Intermediate 75) (54 mg, 0.15 mmol), 4-(4-{[3-(hexahydro-1*H*-azepin-1-yl)propyl]oxy}phenyl)butyl methanesulfonate (for example, as prepared for Intermediate 35) (78 mg, 0.20 mmol) and sodium bicarbonate (28 mg, 0.33 mmol) in MeCN (3 ml) was heated at 80°C with stirring for 6 days under a nitrogen atmosphere. The cooled reaction mixture was filtered, and the filtrate was concentrated *in vacuo.* The residue was partitioned between water and DCM, using a hydrophobic frit. The aqueous layer was diluted with brine and washed with further DCM (x6), using the hydrophobic frit. The combined organic extracts were concentrated *in vacuo* and the residue was purified by mass-directed auto-preparative HPLC. The relevant fractions were concentrated, to give two batches of material, each containing a different impurity. Each of these batches was further separately purified by chromatography on silica [2 g, eluted with 2%-4% (10% aq. ammonia in MeOH) - DCM]. Concentration of the appropriate fractions from these two purifications gave pure product from one (16 mg), and impure material from the second. This later material was further purified by mass-directed auto-preparative HPLC, to give a further portion of pure product (3 mg, as formate salt). The two portions of pure product were combined and dried *in vacuo,* to remove the formic acid, and thus give the *title compound* as the free base (18 mg, 19%). LCMS RT = 2.42 min, ES+ve *m*/*z* 642 [M+H]⁺ and 322 [M/2+H]⁺. ¹H NMR (400 MHz, DMSO-*d*₆) δ 9.23 (s, 1H), 8.89 (d, *J*=5.5 Hz, 1H), 8.17 (d, *J*=5 Hz, 1H), 7.27-7.33 (m, 4H), 7.00 (d, *J*=8 Hz, 2H), 6.76 (d, *J*=8 Hz, 2H), 4.37 (s, 2H), 4.30 (dd, *J*=13, 4.5 Hz, 1H), 4.11 (dd, *J*=13, 8 Hz, 1H), 3.94 (t, *J*=6 Hz, 2H), 3.07-3.14 (m, 1H), 2.94-3.01 (m, 1H), 2.75-2.85 (m, 1H), 2.65-2.73 (m, 6H), 2.48 (t, *J*=6.5 Hz, 2H), 2.20-2.35 (m, 4H), 1.89-1.97 (m, 2H), 1.58-1.88 (m, 12H), 1.45-1.57 (m, 4H).

### Example 24 A

### 4-[(4-Chlorophenyl)methyl]-2-({(2R)-1-[4-(4-{[3-(hexahydro-1H-azepin-1-yl)propyl]oxy}phenyl)butyl]-2-pyrrolidinyl}methyl)-1(2H)-phthalazinone free base

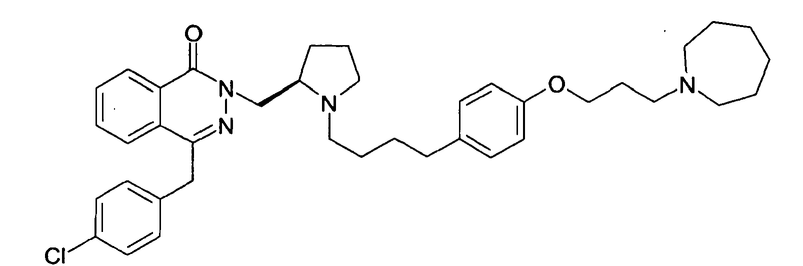

### Method 1

To a solution of 4-[(4-chlorophenyl)methyl]-2-{[(2*R*)-1-(4-{4-[(3-chloropropyl)oxy]phenyl}butyl)-2-pyrrotidinyl]methyl}-1(2*H*)-phthalazinone (for example, as prepared for Intermediate 66) (20 g, 34.6 mmol) in 2-butanone (200 ml) under nitrogen was added potassium iodide (11.5 g, 69.2 mmol), potassium carbonate (9.6 g, 69.2 mmol) and hexamethylene imine (commercially available, for example, from Aldrich) (7.8 ml, 69.2 mmol). The reaction mixture was heated at reflux for 41 h. The solid was removed by filtration and washed with 2-butanone (2 × 100 ml). The combined filtrate and washings were evaporated *in vacuo* and the residue was dissolved in MeOH-DMSO (30 ml, 1:1). This was applied to a C18 reverse phase cartridge (2 × 330 g). This was eluted using a gradient of 0 - 50% MeCN (0.05% TFA) in water (0.05% TFA) over 12 CV. The required fractions were evaporated *in vacuo* and the residue was dissolved in MeOH. This was applied to amino propyl cartridges (4 × 70 g) and eluted with MeOH. The required fractions were evaporated *in vacuo* to afford *the title compound* as an orange gum (10.74 g). LCMS RT = 2.67 min, ES+ve *m*/*z* 641/643 [M+H]⁺. ¹H NMR (400 MHz, CDCl₃) δ 8.46 (m, 1H), 7.74 - 7.62 (m, 3H), 7.26 (d, *J* = 8.5 Hz, 2H), 7.20 (d, *J* = 8.5 Hz, 2H), 7.06 (d, *J* = 8.5 Hz, 2H), 6.81 (d, *J* = 8.5 Hz, 2H), 4.42 (dd, *J* = 4,13 Hz, 1H), 4.24 (s, 2H), 4.07 (dd, *J* = 8,13 Hz, 1H), 3.98 (t, *J* = 6.3 Hz, 2H), 3.16 (m, 1H), 2.97 (m, 1H), 2.90 (m, 1H), 2.65 (m, 6H), 2.55 (m, 2H), 2.37 (m, 1H), 2.21 (m, 1H), 1.93 (m, 2H), 1.89 - 1.52 (m, 16H).

### Method 2

A mixture of 4-[(4-chlorophenyl)methyl]-2-[(2*R*)-2-pyrrolidinylmethyl]-1(2*H*)-phthalazinone (for example, as prepared for Intermediate 4) (1.017 g, 2.87 mmol), 4-(4-{[3-(hexahydro-1*H*-azepin-1-yl)propyl]oxy}phenyl)butyl methanesulfonate (for example, as prepared for Intermediate 35) (1.115 g, 2.91 mmol) and sodium bicarbonate (474 mg, 5.64 mmol) in dry MeCN (50 ml) was heated at 80 °C with stirring for 5 days under a nitrogen atmosphere. The cooled reaction mixture was partitioned between water (70 ml) and EtOAc (70 ml). The aqueous layer was washed with further EtOAc (2 × 50 ml). The combined organic extracts were dried (MgSO₄), and concentrated *in vacuo.* The residue (1.35 g) was dissolved in DMF (10 ml), and divided into ten portions. Each was diluted with TFA (0.5 ml). Each portion was purified by preparative HPLC, using a Kromasil C8 column (25 cm × 5 cm), eluting with a gradient of 5% to 45% of (0.25% TFA in MeCN) in (0.25% TFA in water) over 40 min, followed by holding the final concentration for a further 15 mins. The relevant fractions from each run were combined and concentrated *in vacuo,* to leave an aqueous solution. This was applied to an Amberchrom CG-1 61 M column (25 cm × 2.5 cm) to adsorb the compound. The column was washed with water to remove excess TFA and eluted with MeCN, to afford the product as the trifluoroacetate salt. An SCX cartridge (20 g) was washed with MeOH, then with MeCN. A portion of the above product (0.98 g) was dissolved in MeCN and applied to the SCX cartridge, eluting with MeCN, and then a solution of 10% aq. ammonia in MeCN (200 ml). The appropriate fractions were concentrated *in vacuo* to give *the title compound* as an orange gum (651 mg). LCMS RT = 2.52 min, ES+ve *m*/*z* 641 [M+H]⁺ and 321/322 [M/2 +H]⁺. ¹H NMR (400 MHz, DMSO-*d*₆) δ ppm 8.38 (dd, *J* = 7.7, 1.6 Hz, 1 H), 7.93 (m, 1 H), 7.86 (m, 1 H), 7.82 (m, 1 H), 7.30 (m, 4 H), 7.03 (d, *J* = 8.5 Hz, 2 H), 6.80 (d, *J* = 8.5 Hz, 2 H), 4.36 (m, 1 H), 4.33 (s, 2 H), 4.14 (dd, *J* = 13.1, 8.0 Hz, 1 H), 3.98 (t, *J* = 6.1 Hz, 2 H), 3.14 (m, 1 H), 3.03 (dd, *J* = 7.8, 4.5 Hz, 1 H), 2.84 (m, 1 H), 2.75 (m, 6 H), 2.50 (t, *J* = 6.9 Hz, 2 H), 2.31 (m, 2 H), 1.97 (m, 2 H), 1.82 (m, 4 H), 1.68 (m, 8 H), 1.55 (m, 4 H).

### Example 24 B

### 4-[(4-Chlorophenyl)methyl]-2-({(2R)-1-[4-(4-{[3-(hexahydro-1H-azepin-1-yl)propyl]oxy}phenyl)butyl]-2-pyrrolidinyl}methyl)-1(2H)-phthalazinone, diformate salt

Starting from 2.25 mmol of 4-[(4-chlorophenyl)methyl]-2-[(2*R*)-2-pyrrolidinylmethyl]-1(2*H*)-phthalazinone (for example, as prepared for Intermediate 4), and following the preparation for Example 24 A, method 2, the residue after initial aqueous work-up was treated as follows:
The crude material (1.20 g) was dissolved in DCM (5 ml) and treated with DIPEA (0.087 ml, 0.499 mmol) and acetyl chloride (0.036 ml, 0.506 mmol), and the mixture was stirred at room temperature for 45 min. The reaction mixture was applied to an SCX cartridge (50 g), eluting with MeOH (× 2), and then a solution of 10% aq. ammonia in MeOH. The appropriate fractions were concentrated *in vacuo* to give an orange oil (853 mg). A portion of this (548 mg) was dissolved in a mixture of MeOH (6 ml) and DMSO (1 ml) and this solution was purified by MDAP HPLC. Relevant fractions were combined and concentrated *in vacuo* to give the *title compound* as an orange gum (384 mg). LCMS RT = 2.42 min, ES+ve *m*/*z* 641/643 [M+H]⁺ and 321/322 [M/2+H]⁺.

### Example 24 C

### 4-[(4-Chlorophenyl)methyl]-2-({(2R)-1-[4-(4-{[3-(hexahydro-1H-azepin-1-yl)propyl] oxy}phenyl)butyl]-2-pyrrolidinyl}methyl)-1(2H)-phthalazinone, 1,5-naphthalene disulfonate monohydrate salt

### Method 1

4-[(4-Chlorophenyl)methyl]-2-({(2R)-1-[4-(4-{[3-(hexahydro-1*H*-azepin-1-yl)propyl]oxy} phenyl)butyl]-2-pyrrolidinyl}methyl)-1(2*H*)-phthalazinone (for example as prepared for Example 24 A) (400 mg) was dissolved in MeOH (4.44 ml). A solution of 1,5-naphthalene disulfonic acid (232 mg) in MeOH (1 ml) was added and the resulting gummy solution was heated with an air gun. Small amounts of solid began to form and on cooling a solid precipitated. The slurry was stirred at RT for approximately 1 h. MeOH (2 ml) was added to mobilise the slurry, which was heated and cooled again, and left to stir for a further hour. The solid was isolated by filtration and dried *in vacuo* at 40 °C to *give the* title compound (464.5 mg, 73%).

### Method 2

To 4-[(4-chlorophenyl)methyl]-2-({(2*R*)-1-[4-(4-{[3-(hexahydro-1*H*-azepin-1-yl)propyl]oxy} phenyl)butyl]-2-pyrrolidinyl}methyl)-1(2*H*)-phthalazinone (for example as prepared for Example 24 A) (3.82 g, 5.96 mmol) was added water (200 ml) and hydrochloric acid (2N. 12 ml). The reaction mixture was heated to 90 °C to obtain a clear solution. To this was added a solution of 1,5-naphthalenedisulfonic acid monohydrate (2.2 g, 6 mmol) in water (100 ml) over 20 min. The suspension was stirred at 90 °C for 20 min and then allowed to cool to room temperature. The solid was collected by filtration and washed with water (100 ml). The solid was air-dried for 3 days and then *in vacuo* for 20 h to give the *title compound* (5.1 g, 92%) as a white solid. LCMS RT = 2.58 min, ES+ve *m*/*z* 641 (M+H)⁺.

### Method 3: 4-[(4-Chlorophenyl)methyl]-2-({(2R)-1-[4-(4-{[3-(hexahydro-1H-azepin-1-yl)propyl]oxy}phenyl)butyl]-2-pyrrolidinyl}methyl)-1(2H)-phthalazinone, 1,5-naphthalene disulfonate monohydrate salt may also be prepared according to the following method.

For this method the following abbreviations are used:

| | |
|---|---|
| eq: | equivalent (1 eq = 1 mole reagent per 1 mole of starting material) |
| vol: | volume (1 vol = 1 ml per gram starting material) |
| vol/vol: | volume/volume |
| wt: | weight (1 wt = 1 g reagent per 1 g starting material) |
| wt/vol: | weight/volume |

### Intermediate 76 (Stage 1)

### (3E)-3-[(4-chlorophenyl) methylidene]-2-benzofuran-1(3H)-one

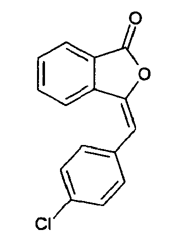

4-Chlorophenylacetic acid (commercially available, for example, from Aldrich) (1.00 eq), phthalic anhydride (commercially available, for example, from Aldrich) (1.10 eq) and sodium acetate (0.04 eq) are mixed in NMP (3 vol). The resulting suspension is heated to approximately 200 °C and the resulting brown solution is stirred over 2 days. During the reaction NMP/ water (0.45 vol) is distilled off at ambient pressure. After checking complete conversion (99%, HPLC) the reaction mixture is cooled to approximately 70 °C over 1 h and EtOH (4.5 vol) is added over 1 h at approximately 70 °C. The resulting brown solution is cooled to approximately 50 °C over 1.5 h during which it turns into a brown suspension. At approximately 50 °C, EtOH (3.8 vol) is added over 1 h and the resulting brown suspension is cooled to approximately 2°C over 1 h and is stirred at approximately 0-5 °C for 1 h. The brown solid is isolated by filtration through a suction strainer, washed with cold aq. EtOH (EtOH/water = 1/1, vol/vol, approximately 2 °C, 3 × 1 vol) and dried on a suction strainer under nitrogen. The product is obtained as a pale brown and humid solid. The loss on drying is determined and the material is taken into the next stage. Yield (corrected for loss on drying and ¹H NMR assay): 80%.
¹H NMR (400MHz, CDCl₃), δ 6.37, (s, 1H), 7.38 (d, 2H), 7.58 (t, 1H), 7.77 (m, 4H), 7.95 (d, 1H)

### Intermediate 77 (Stage 2)

### 4-{[4-chlorophenyl]methyl}-1(2H)-phthalazinone

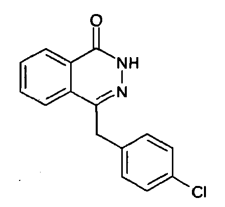

(3*E*)-3-[(4-Chlorophenyl)methylidene]-2-benzofuran-1(3*H*)-one (as prepared, for example, in stage 1) (1.0 eq, corrected for loss on drying) is suspended in EtOH (3.7 vol) and heated to approximately 85 °C at slight reflux. A solution of hydrazine hydrate (commercially available, for example, from Aldrich) (1.2 eq) in EtOH (0.63 vol) is added through a dropping funnel over 1 h. At the end of the addition, EtOH (0.63 vol) is added through the dropping funnel into the reaction suspension in order to remove traces of hydrazine hydrate. The reaction suspension is heated at approximately 85 °C at slight reflux for 14 h. It is cooled to approximately 20 °C and a sample is taken to check the conversion (99% conversion, HPLC). Acetone (0.35 vol) is added to the reaction mixture over 30 min (exothermic reaction). The quenched suspension is stirred for at least 1 h and is then cooled to approximately 2 °C over 30 min and stirred at approximately 2 °C for 1 h. The product is isolated by filtration through a suction strainer and is washed with cold EtOH (approximately 0-5 °C, 3 × 1.9 vol). The pale brown solid is completely dried on the suction strainer *in vacuo* under nitrogen. The *title compound* is obtained as a pale brown solid. Yield (corrected for ¹H NMR assay): 90-95%.
¹H NMR (400MHz, DMSO-d₆), δ 4.30 (s, 2H), 7.35 (m, 4H), 7.88 (m, 3H), 8.26 (d, 1H), 12.62 (s, 1H)

### Intermediate 78 (Stage 3a)

### 1,1-dimethylethyl 2-{[(methylsulfonyl)oxy]methyl}-1-pyrrolidine carboxylate

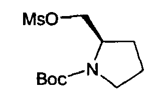

A solution of *N*-Boc-D-prolinol (commercially available, for example, from Aldrich) (1.0 eq) in MIBK (9.5 vol) is cooled to approximately 2°C and NEt₃ (1.03 vol) is added. MsCl (1.2 eq) is added through a dropping funnel over 1 h and a white suspension forms. The dropping funnel is washed with additional MIBK (0.5 vol). The reaction mixture is warmed to approximately 22°C and stirred for 2 h. A sample is taken to check the conversion (complete conversion by TLC). Water (5.0 vol) is added. The phases are separated (good and quick phase separation). The organic phase is washed with saturated aq. NaHC0₃ (5.0 vol) and finally with water (5.0 vol) (good and quick phase separations). The organic phase is dried by filtering over a suction strainer filled with MgSO₄ (0.46 wt). The volume of the dried organic phase is determined (12.40 vol). The organic phase is concentrated to 43% wt/vol (based on *N*-Boc-D-prolinol/solution) by distillation *in vacuo* at approximately 40 °C to the final volume (2.20 vol). A loss on drying sample is taken and evaporated to dryness (approximately 40°C, <100 mbar); forming a yellow oil which is taken for analysis. The concentrated yellow organic phase (2.0 vol) is used immediately in the alkylation reaction. Yield (corrected for loss on drying and ¹H NMR assay): 100%.

### Intermediate 4 (Stage 3b)

### 4-[(4-chlorophenyl)methyl]-2-[(2R)-2-pyrrolidinylmethyl]-1(2H)-phthalazinone, HCI salt

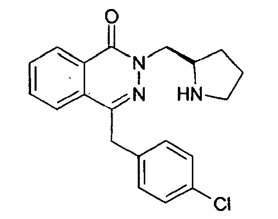

A suspension of 4-{[4-chlorophenyl]methyl}-1(2*H*)-phthalazinone (as prepared, for example, in stage 2) (1.0 eq) and Cs₂CO₃ (2.5 eq) in MIBK (9.7 vol) is heated to approximately 100 °C. A freshly prepared solution of 1,1-dimethylethyl 2-{[(methylsulfonyl)oxy]methyl}-1-pyrrolidine carboxylate (as prepared, for example, in stage 3a) in MIBK (1.2 eq calculated for *N*-Boc-D-prolinol) is added dropwise over 2 h at approximately 100 °C. The dropping funnel is washed with MIBK (0.2 vol) which is added to the reaction mixture. The reaction mixture is stirred for 17 h at approximately 100 °C. A brown suspension is formed. After cooling to approximately 50 °C, a sample is taken to check the conversion (99% conversion, HPLC). The reaction mixture is cooled to approximately 22 °C and water (16.7 vol) is added to the reaction mixture, followed by the addition of MIBK (16.7 vol). The phases are separated. The volume of the organic phase (18.8 vol) is determined and it is concentrated to 50% w/vol (4-{[4-chlorophenyl]methyl}-1(2H)-phthalazinone/solution) by distillation *in vacuo* (approximately 45 °C, < 100 mbar). HCl in iso-propyl alcohol (5-6 M, 3 eq, 2.0 vol) is added to the concentrated organic phase at approximately 22 °C. The formation of gas is observed and a pale brown suspension forms over about 1 h. The reaction mixture is stirred at approximately 22 °C for 14 h. A sample is taken to check the conversion (complete conversion, HPLC). The pale brown suspension is cooled to approximately 1 °C over 2 h and the product is isolated by filtration through a fritted funnel, and is washed with cold MIBK (3 × 1 vol). A white solid results, which is dried on the suction strainer and subsequently *in vacuo* (45 °C, < 20 mbar). The *title compound* (as HCl salt) is obtained as a white solid. Yield (corrected for ¹H NMR assay): 86%.
¹H NMR (400MHz, DMSO-*d*₆), δ 1.76 (m, 1H), 1.95 (m, 2H), 2.14 (m, 1H), 3.15 (m, 1H), 3.27 (m, 1H), 3.91 (m, 1H), 4.36 (d, 2H), 4.47 (m, 2H), 7.35 (d, 2H), 7.41 (d, 2H), 7.80-8.00 (m, 3H), 8.31 (d, 1H), 8.92 (bs, 1H), 9.48 (bs, 1H)

### Intermediate 63 (Stage 4a)

### 4-[4-(methyloxy)phenyl]butyl methanesulfonate

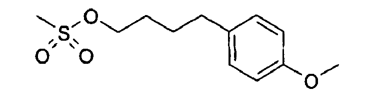

To a solution of 4-(4-methoxyphenyl)-1-butanol (commercially available, for example, from Aldrich) (1.0 eq) in MIBK (9.5 vol) is added NEt₃ (1.5 eq, 1.16 vol) at approximately 21 °C. The resulting solution is cooled to approximately 10-15 °C and MsCl (1.2 eq, 0.52 vol) is added over 1 h, keeping the temperature at approximately 16 °C. A white suspension forms immediately. At the end of the addition, the dropping funnel is washed with MIBK (0.5 vol) which is transferred into the reaction flask. The reaction mixture is warmed to approximately 22 °C over 3 h and stirred at approximately 22 °C for 15 h. A pale yellow suspension is present. A sample is taken to check the conversion (complete conversion, HPLC). The reaction mixture is cooled to approximately 10-15 °C and water (5.6 vol) is added, keeping the temperature below approximately 18 °C. The emulsion is stirred over 10 min at approximately 22°C. The phases are separated. The organic phase is washed with saturated aq. NaHCO₃ (5.6 vol) and finally with water (5.6 vol). The organic phase is dried by filtering over a suction strainer filled with MgSO₄ (0.5 wt) and the MgSO₄ is washed with MIBK (2 × 0.2 vol). The volume of the dried organic phase is determined (12.40 vol). The organic phase is concentrated to 40% w/vol (4-(4-methoxyphenyl)-1-butanol /solution) by distillation *in vacuo* at approximately 45 °C to 2.20 vol. A sample for loss on drying is taken and evaporated to dryness (approximately 40 °C, < 100 mbar); a yellow oil remains which is taken for analysis. The product containing yellow organic phase (2.5 vol) is used in the subsequent alkylation reaction (stage 4b). Yield (corrected for loss on drying and ¹H NMR assay): 101%.
¹H NMR (DMSO-*d*₆) δ 1.53-1.71 (m, 4H), 2.52-2.57 (m, 2H), 3.11-3.20 (s, 3H), 3.68-3-76 (s, 3H), 4.15-4.26 (m, 2H), 6.81-6.87 (m, 2H), 7.08-7.15 (m, 2H)

### Intermediate 64 (Stage 4b)

### 4-[(4-chlorophenyl)methyl]-2-[((2R)-1-{4-[4-(methyloxy)phenyl]butyl}-2-pyrrolidinyl) methyl]-1(2H)-phthalazinone

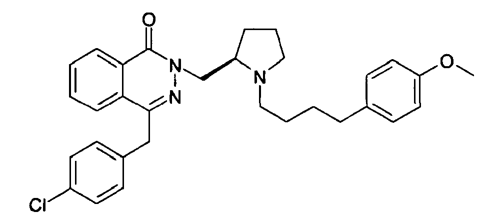

4-[(4-Chlorophenyl)methyl]-2-[(2*R*)-2-pyrrolidinylmethyl]-1(2*H*)-phthalazinone, HCl salt (as prepared for example, in stage 3b) (1.0 eq) and K₂CO₃ (5.0 eq, 1.77 wt) are mixed in MIBK (16.5 vol) and the resulting light brown suspension is heated to approximately 135 °C at reflux. A clear orange solution of 4-[4-(methyloxy)phenyl]butyl methanesulfonate (as prepared for example in stage 4a) (2.4 eq, 1.59 wt) in MIBK (4.9 vol) is added at reflux over 1 h. The resulting yellow-brown suspension is stirred at reflux for 20 h. A sample is taken to check the conversion (88.5% conversion, HPLC). Water (24.7 vol) is added at approximately 19 °C over 5 min (slightly exothermic). The turbid orange brown mixture thus formed is stirred for 15 min at approximately 20°C. The phases are separated. The organic phase is dried by filtration through a suction strainer filled with MgSO₄ (0.92 wt); the MgSO₄ is washed with MIBK (2 × 4.1 vol). The solvent of the resulting organic phase is completely removed *in vacuo* (approximately 40-45 °C, 600 mbar to full suction). The obtained crude product (2.45 wt, dark brown oil, HPLC purity: 73.50% area/area) is combined with a crude product obtained in the same manner (2.41 wt) and purified by plug filtration (SiO₂). Therefore, the combined crude material (4.86 wt) is dissolved in DCM and put on a suction strainer filled with SiO₂ (45.7 wt, height: 24.5 cm, diameter: 30 cm) to wash out impurities with DCM (823 vol). The eluent is gradually changed from DCM only to DCM:MeOH = 10:1 in order to elute the title compound. The product is obtained in solution (494 vol). Removal of the solvents by distillation *in vacuo* (45 °C, 600 mbar to full suction) results in the *title compound* (2.46 wt) as a pale brown oil. Yield (corrected for ¹H NMR assay): 86%
¹H NMR (DMSO-*d*₆) δ 1.32-1.53 (m, 4H), 1.61-1.79 (m, 4H), 2.08-2.18 (m, 1H), 2.20-2.27 (m, 1H), 2.37-2.45 (m, 2H), 2.66-2.76 (m, 1H), 2.84-2.93 (m, 1H), 2.96-3.04 (m, 1H), 3.69-3.71 (m, 3H), 3.89-3.98 (m, 1H), 4.18-4.26 (m, 1H), 4.28-4.36 (m, 2H), 6.77-6.83 (m, 2H), 6.98-7.04 (m, 2H), 7.33-7.39 (m, 4H), 7.79-7.90 (m, 2H), 7.91-7.97 (m, 1H), 8.26-8.31 (m, 1H).

### Intermediate 65 (Stage 5)

### 4-[(4-chlorophenyl)methyl]-2-({(2R)-1-[4-(4-hydroxyphenyl)butyl]-2-pyrrolidinyl} methyl)-1(2H)-phthalazinone

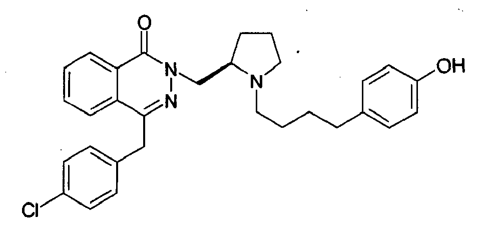

A solution of 4-[(4-chlorophenyl)methyl]-2-[((2*R*)-1-{4-[4-(methyloxy)phenyl]butyl}-2-pyrrolidinyl)methyl]-1(2*H*)-phthalazinone (as prepared, for example, in stage 4b) (1.0 eq) in DCM (4.2 vol) is cooled to approximately 0 °C. A solution of BBr₃ (1.8 eq, 0.33 vol) in DCM (3.4 vol) is added over 20 min keeping the temperature below approximately 2 °C. The reaction mixture is stirred overnight at approximately 20 °C. A sample is taken to check the conversion (90% conversion, HPLC). Additional BBr₃ (0.2 eq, 0.05 vol) is added at approximately -1 °C over 10 min. The reaction mixture is warmed to approximately 20 °C. After approximately 5 h, another sample is taken to check the conversion (96% conversion, HPLC). Additional BBr₃ (0.2 eq, 0.05 vol) is added and the reaction is stirred at approximately 25 °C overnight and another sample is taken to check the conversion (> 99% conversion, HPLC). The reaction mixture is cooled to approximately 15°C and aq. HCl (2 N, 2.4 vol) is added dropwise over 15 min keeping the temperature below about 19 °C. After approximately 2/3 of the addition of HCl, exothermic reaction behaviour is observed. After complete addition, a brown suspension is formed which contains some brown, oily material. Aq. sat. NaHCO₃ (5.1 vol) is slowly added over 20 min at approximately 11°C, keeping the temperature below about 13 °C. A dark, slightly turbid emulsion is formed. The reaction mixture is warmed to approximately 20 °C over 15 min and the phases are separated. The aqueous phase is back extracted with DCM (4.28 vol). The combined organic phases are dried by filtration over a suction strainer filled with MgSO₄ (0.69 wt), then the MgSO₄ is washed with DCM (3 × 1.7 vol). The dried organic phase is dark and clear. During removal of the solvent *in vacuo* (600 mbar - full suction, 35-40 °C) a brown foam forms. The obtained brown solid (HPLC purity: 73.22% area/area) contains residual DCM and is dried again over weekend (35°C, <20 mbar). The redried material displays decreased HPLC purity (63.98% area/area). The crude is divided into 2 equal portions (2 × 0.50 wt) which are purified by column chromatography (2 columns; SiO₂ (2 × 2.74 wt); height = 20.5 cm, diameter = 14 cm; DCM:MeOH = 20:1). The fractions containing product are combined and concentrated *in vacuo* (approximately 35°C, 600 mbar to full suction). The *title compound* is obtained as a light brown foamy solid. Yield (corrected for ¹H NMR assay): 65%.
1H NMR (DMSO-*d*₆) δ 1.39-1.72 (m, 4H), 1.82-2.05 (m, 3H), 2.12-2.24 (m, 1H), 2.35-2.48 (m, 2H), 3.00-3.25 (m, 2H,), 3.26-3.54 (m, 1H), 3.56-3.71 (m, 1H), 3.80-3.97 (m, 1H), 4.25-4.43 (m, 2H), 4.49-4.62 (m, 2H), 6.58-6.74 (m, 2H), 6.91-7.03 (m, 2H), 7.27-7.48 (m, 4H), 7.79-8.04 (m, 3H), 8.23-8.39 (m, 1H), 9.06-9.37 b, 2H)

### Intermediate 66 (Stage 6)

### 4-[(4-chlorophenyl)methyl]-2-{[(2R)-1-(4-{4-[(3-chloropropyl) oxy]phenyl}butyl)-2-pyrrolidinyl]methyl}-1(2H)-phthalazinone

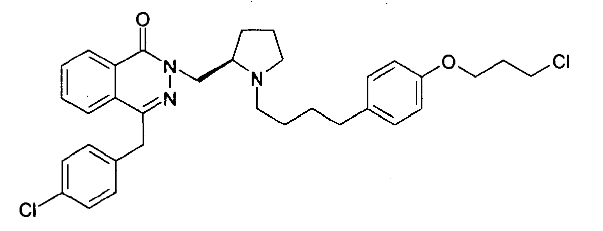

4-[(4-Chlorophenyl)methyl]-2-({(2*R*)-1-[4-(4-hydroxyphenyl)butyl]-2-pyrrolidinyl}methyl)-1(2*H*)-phthalazinone (as prepared, for example, in stage 5) (1.0 eq) and K₂CO₃ (4.0 eq, 1.1 wt) are mixed in 2-butanone (7.1 vol) at approximately 20°C. To the resulting brown suspension, is added a solution of 1-bromo-3-chloropropane (2.0 eq, 0.40 vol) in 2-butanone (2.9 vol). The brown mixture is heated to reflux for 25 h. A sample is taken to check the conversion (98% conversion, HPLC). At approximately 20 °C, water (14.8 vol) is added over 5 min (slightly exothermic). MIBK (14.8 vol) is added and the orange mixture is stirred for 25 min; the phases are separated. The organic phase is dried by filtration over a suction strainer filled with Na₂SO₄ (1.90 wt) and the Na₂SO₄ is washed with MIBK (2 × 2.4 vol). Evaporation of the solvents *in vacuo* (40 °C, 600 mbar - full suction) results in the *title compound* (as a mixture of chloro- and bromo-derivative) as a brown oil. Yield (corrected for HPLC purity): 91 %.
¹H NMR (CDCl₃) δ 1.47-1.91 (m, 6H), 2.16-2.27 (m, 3H); 2.33-2.43 (m, 1H), 2.51-2.60 (m, 2H), 2.80-3.04 (m, 2H), 3.08-3.23 (m, 1H), 3.47-3.86 (m, 3H), 4.01-4.14 (m, 3H), 4.18-4.30 (m, 2H), 4.36-4.50 (m, 1H), 6.74-6.88 (m, 2H), 7.01-7.14 (m, 2H), 7.15-7.34 (m, 4H), 7.57-7.77 (m, 3H), 8.37-8.52 (m, 1H).

### Example 24A (Stage 7)

### 4-[(4-chlorophenyl)methyl]-2-({(2R)-1-[4-(4-{[3-(hexahydro-1H-azepin-1-yl)propyl] oxy}phenyl)butyl]-2-pyrrolidinyl}methyl)-1(2H)-phthalazinone, free base

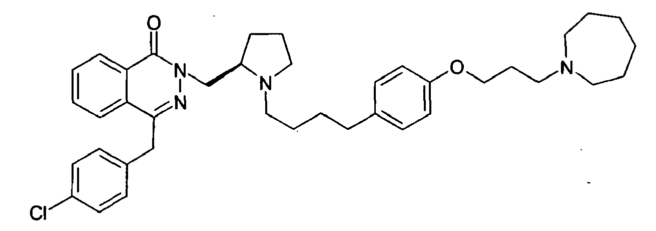

4-[(4-Chlorophenyl)methyl]-2-{[(2*R*)-1-(4-{4-[(3-chloropropyl)oxy]phenyl}butyl)-2-pyrrolidinyl]methyl}-1(2*H*)-phthalazinone (as prepared, for example, in stage 6) (1.0 eq), KI (3.0 eq, 0.86 wt), K₂CO₃ (3.0 eq, 0.72 wt) and hexamethyleneimine (commercially available, for example, from Aldrich) (3.0 eq, 0.59 vol) are mixed in MIBK (10.9 vol) and the resulting brown suspension is heated at reflux for 18 h. A sample is taken to check the conversion (complete conversion, HPLC). The light brown suspension is cooled to approximately 30°C and water (6.9 vol) is added over 5 min. After stirring for 20 min the phases are separated. The aqueous phase is back extracted with MIBK (3.96 vol). Removal of the solvents *in vacuo* (40-50 °C, 600 mbar - full suction) results in the *title compound* as a brown oil. Yield (corrected for ¹H NMR assay): 83%.
¹H NMR (CDCl₃) δ 1.41-2.05 (m, 14H), 2.13-2.27 (m, 1H), 2.29-2.44 (m, 1H), 2.47-2.72 (m, 6H), 2.81-3.02 (m, 2H), 3.08-3.22 (m, 1H), 3.92-4.11 (m, 3H), 4.20-4.28 (m, 2H), 4.34-4-49 (m, 1H), 6.72-6.87 (m, 2H), 6.96-7.12 (m, 2H), 7.14-7.31 (m, 4H), 7.59-7.77 (m, 3H), 8.38-8.52 (m, 1H).

### Example 24C (Stage 8)

### 4-[(4-chlorophenyl)methyl]-2-({(2R)-1-[4-(4-{[3-(hexahydro-1H-azepin-1-yl)propyl] oxy}phenyl)butyl]-2-pyrrolidinyl}methyl)-1(2H)-phthalazinone, 1,5-naphthalene disulfonate monohydrate salt

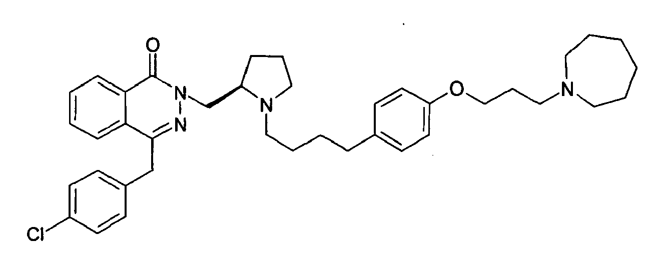

### Preparation 1:

An orange solution of 4-[(4-chlorophenyl)methyl]-2-({(2*R*)-1-[4-(4-{[3-(hexahydro-1*H-*azepin-1-yl)propyl]oxy}phenyl)butyl]-2-pyrrolidinyl}methyl)-1(2*H*)-phthalazinone (as prepared, for example, in stage 7) (1.0 eq) in MeOH (41.41 vol) is cooled to approximately 15 °C. Aqueous HCl (2 N, 41.4 vol) is added over 20 min while keeping the temperature below about 18 °C. The solvents are distilled off (approximately 80 °C, 600 mbar to full suction) and an orange-brown oil remains, which is dissolved in water (32.1 vol). The resulting orange-brown, slightly turbid solution is heated to approximately 100 °C (reflux) and MeOH (61.1 vol) is added. To the resulting yellow solution, a solution of 1,5-naphthalenedisulfonic acid tetrahydrate (1.0 eq, 0.57 wt) in water (6.0 vol) is added over 2 min. The solution remains yellow and is cooled from approximately 58 °C to approximately 20 °C over 90 min. A white suspension forms, which is filtered through a suction strainer at approximately 20 °C. The solid is washed with aq. MeOH (MeOH:water = 1:1, 2 × 10.4 vol) and the recovered pale brown material is dried *in vacuo* (approximately 50 °C, full suction). The *title compound* is obtained as a pale brown solid. Yield (corrected for HPLC purity): 69%.

### Preparation 2:

A brown solution of 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone (as prepared, for example, in stage 7) (1.0 eq) in MeOH (39.9 vol) is cooled to approximately 15 °C. Aqueous HCl (2N, 42.6 wt) is added over 20 min keeping the temperature below about 18 °C. The solvents are distilled off (approximately 80°C, 600 mbar to full suction) and an orange-brown oil remains which is dissolved in water (32.9 vol). The resulting orange-brown, slightly turbid solution is washed with EtOAc (1 × 41.2 vol, 1 × 39.5 vol) and a white emulsion forms, which separates into two phases. The inorganic phase is evaporated to dryness and an orange-brown oil remains. The oil is dissolved in MeOH (79.9 vol) and the resulting orange-brown solution is heated at approximately 90 °C at reflux. To the solution is added a solution of 1,5-naphthalenedisulfonic acid tetrahydrate (1.0 eq, 0.56 wt) in water (4.8 vol) over 2 min. The solution remains clear and is cooled to approximately 2 °C over 100 min. A white solid suspension results, which is stirred for 30 min at approximately 2 °C. The solid is isolated by filtration (good) and is washed with cold aq. MeOH (3 × 11.0 vol). The brown solid is dried *in vacuo* (50 °C, 10 mbar, 18 h). The pale brown material contains grains and is crushed mechanically. The title compound is obtained as a pale brown solid. Total recovery (corrected for HPLC purity): 74%.
¹H NMR (400MHz, DMSO-*d*₆), δ 1.30-2.28 (m, 18H), 3.13 (m, 4H), 3.20 (m, 2H), 3.27-3.53 (m, 9H), 3.62 (m, 1H), 3.85 (m, 1H), 3.94 (m, 2H), 4.31 (m, 2H), 4.55 (d, 2H), 6.82 (m, 2H), 7.05 (d, 2H), 7.29-7.51 (m, 6H), 7.79-8.05 (m, 5H), 8.31 (m, 1H), 8.86 (d, 2H), 9.15 (m, 2H)

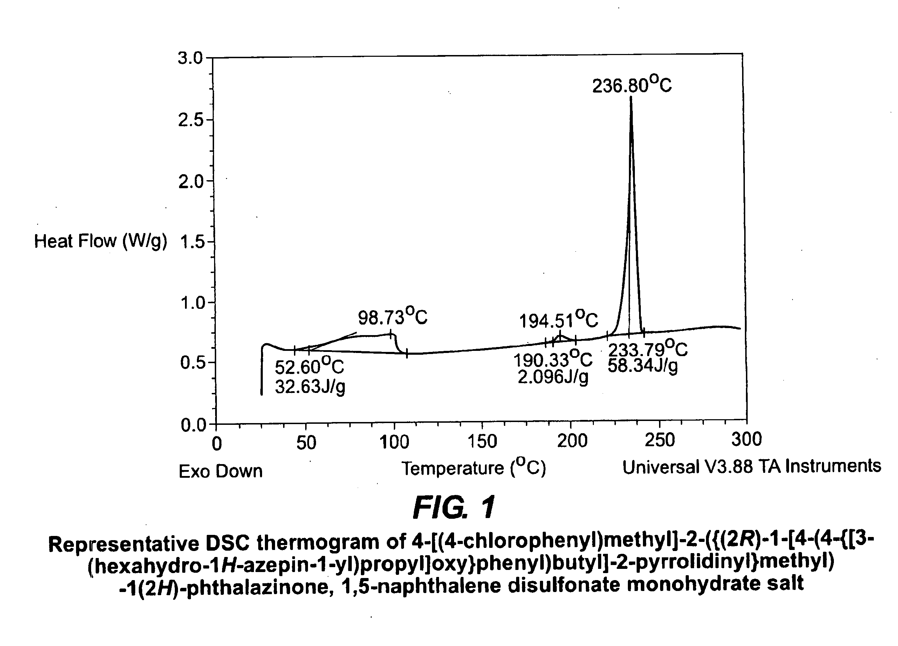

The DSC thermogram plots the differential rate of heating in watts per second against temperature. The DSC thermogram of crystalline 4-[(4-chlorophenyl)methyl]-2-({(2*R*)-1-[4-(4-{(3-(hexahydro-1*H*-azepin-1-yl)propyl]oxy}phenyl)butyl]-2-pyrrolidinyl}methyl)-1(2*H*)-phthalazinone, 1,5-naphthalene disulfonate monohydrate salt (for example, as prepared for Example 24C) displays three broad endotherms at approximately 53 °C ±5; 190 °C ±5 and 234 °C ±5 which correspond to the loss of water, a small endothermic event and the melt respectively. The enthalpy of fusion determined by integrating the melt peak is 58 J/g ±10. A representative DSC thermogram is shown in Figure 1.

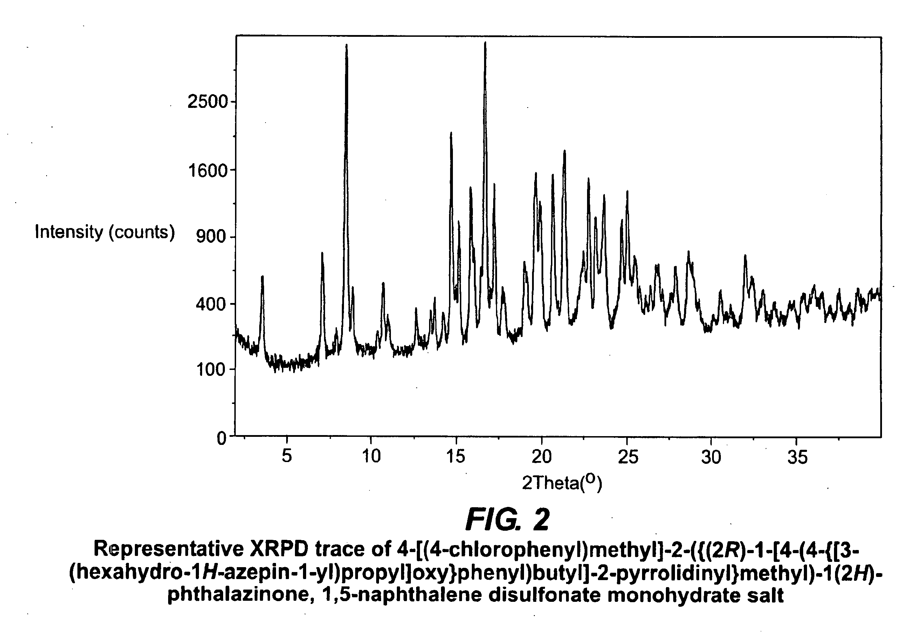

A representative XRPD pattern of 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone, 1,5-naphthalene disulfonate monohydrate salt (for example, as prepared for Example 24C) is shown in figure 2. The peak angles for this form are tabulated below.

| **Two theta (°)** | **d-spacing ( )** |
|---|---|
| 8.5 | 10.4 |
| 14.8 | 6.0 |
| 15.2 | 5.8 |
| 15.9 | 5.6 |
| 16.7 | 5.3 |
| 17.3 | 5.1 |
| 19.7 | 4.5 |
| 20.7 | 4.3 |
| 21.3 | 4.2 |
| 22.8 | 3.9 |
| 23.7 | 3.8 |
| 25.1 | 3.5 |

### Example 24D

### 4-[(4-Chlorophenyl)methyl]-2-({(2R)-1-[4-(4-{[3-(hexahydro-1H-azepin-1-yl)propyl] oxy}phenyl)butyl]-2-pyrrolidinyl}methyl)-1(2H)-phthalazinone, dihydrochloride salt

4-[(4-Chlorophenyl)methyl]-2-({(2*R*)-1-[4-(4-{[3-(hexahydro-1*H*-azepin-1-yl)propyl]oxy} phenyl)butyl]-2-pyrrolidinyl}methyl)-1(2*H*)-phthalazinone (for example, as prepared for Example 24 A) (3.85 g, 6.0 mmol) was dissolved in MeOH (100 ml) and 2N hydrochloric acid (12 ml, 24 mmol). The solvent was removed *in vacuo.* The residue was dissolved in MeOH (50 ml) and then evaporated. This was repeated 3 times. The residue was dried *in vacuo* to give *the title compound* (4.3 g, 100%) as a crunchy foam. LCMS RT = 3.41 min, ES+ve m/z 641 (M+H)⁺. ¹H NMR (400 MHz, DMSO-d₆) δ 10.60 (1H, br s), 10.49 (1H, br s), 8.30 (1H, dd, *J* = 7.5, 1.5 Hz), 7.96 (1H, d, *J* = 7.5 Hz), 7.88-7.93 (1H, m), 7.84-7.89 (1H, m), 7.38 (2H, d, *J* = 8.5 Hz), 7.34 (2H, d, *J* = 8.5 Hz), 7.09 (2H, d, *J* = 8.5 Hz), 6.84 (2H, d, *J* = 8.5 Hz), 4.62 (1H, dd, *J* = 14.0, 4.5 Hz), 4.55 (1H, dd, *J* = 14.0, 7.0 Hz), 4.37 (1H, d, *J* = 16.5 Hz), 4.33 (1H, d, *J* = 16.5 Hz), 4.00 (2H, t, *J* = 6.0 Hz), 3.77-3.85 (1H, m), 3.55-3.64 (1H, m), 3.31-3.46 , (3H, m), 3.15-3.22 (2H, m), 3.02-3.14 (4H, m), 2.47-2.53 (2H, m), 2.07-2.23 (4H, m), 1.49-1.99 (14H, m).

### Biological Data

The compounds of the invention may be tested for *in vitro* and/or *in vivo* biological activity in accordance with the following or similar assays.

### H1 receptor cell line generation and FLIPR assay protocol

### 1. Generation of histamine H1 cell line

The human H1 receptor is cloned using known procedures described in the literature [Biochem. Biophys. Res. Commun., 201(2):894 (1994)]. Chinese hamster ovary (CHO) cells stably expressing the human H1 receptor are generated according to known procedures described in the literature [Br. J. Pharmacol., 117(6):1071 (1996)].

### Histamine H1 functional antagonist assay: Determination of functional pKi values

The histamine H1 cell line is seeded into non-coated black-walled clear bottom 384-well tissue culture plates in alpha minimum essential medium (Gibco/Invitrogen, cat no. 22561-021), supplemented with 10% dialysed foetal calf serum (Gibco/Invitrogen cat no. 12480-021) and 2 mM L-glutamine (Gibco/Invitrogen cat no 25030-024) and is maintained overnight at 5% CO₂, 37 °C.

Excess medium is removed from each well to leave 10 µl. 30 µl loading dye (250 µM Brilliant Black, 2 µM Fluo-4 diluted in Tyrodes buffer + probenecid (145 mM NaCl, 2.5 mM KCI, 10 mM HEPES, 10 mM D-glucose, 1.2 mM MgCl₂, 1.5 mM CaCl₂, 2.5 mM probenecid, pH adjusted to 7.40 with NaOH 1.0 M)) is added to each well and the plates are incubated for 60 min at 5% CO₂, 37 °C.

10 µl of test compound, diluted to the required concentration in Tyrodes buffer + probenecid (or 10 µl Tyrodes buffer + probenecid as a control) is added to each well and the plate is incubated for 30 min at 37 °C, 5% CO₂. The plates are then placed into a FLIPR™ (Molecular Devices, UK) to monitor cell fluorescence (λₑₓ = 488 nm, λ_{EM} = 540 nm) in the manner described in Sullivan et al., (In: Lambert DG (ed.), Calcium Signaling Protocols, New Jersey: Humana Press, 1999, 125-136) before and after the addition of 10 µl histamine at a concentration that results in the final assay concentration of histamine being EC₈₀.

Functional antagonism is indicated by a suppression of histamine induced increase in fluorescence, as measured by the FLIPR™ system (Molecular Devices). By means of concentration effect curves, functional affinities are determined using standard pharmacological mathematical analysis.

### Histamine H1 functional antagonist assay: Determination of antagonist pA2 and duration

The histamine H1 receptor expressing CHO cells are seeded into non-coated black-walled clear bottom 96-well tissue culture plates as described above.

Following overnight culture, growth medium is removed from each well, washed with 200 µl phosphate buffered saline (PBS) and is replaced with 50 µl loading dye (250 µM Brilliant Black, 1 µM Fluo-4 diluted in Tyrodes buffer + probenecid (145 mM NaCl, 2.5 mM KCI, 10mM HEPES, 10mM D-glucose, 1.2 mM MgCl₂, 1.5 mM CaCl₂, 2.5 mM probenecid, pH adjusted to 7.40 with NaOH 1.0 M)). Cells are incubated for 45 min at 37 °C. The loading buffer is removed and the cells are washed as above, and 90 µl of Tyrodes buffer + probenecid is added to each well. 10 µl of test compound, diluted to the required concentration in Tyrodes buffer + probenecid (or 10 µl Tyrodes buffer + probenecid as a control) is added to each well and the plate is incubated for 30 min at 37 °C, 5% CO₂.

The plates are then placed into a FLIPR™ (Molecular Devices, UK) to monitor cell fluorescence (λₑₓ = 488 nm, λ_{EM} = 540 nm) in the manner described in Sullivan et al., (In: Lambert DG (ed.), Calcium Signaling Protocols, New Jersey: Humana Press, 1999, 125-136) before and after the addition of 50 µl histamine over a concentration range of 1 mM - 0.1 nM. The resultant concentration response curves are analysed by non-linear regression using a standard four parameter logistic equation to determine the histamine EC₅₀, the concentration of histamine required to produce a response of 50% of the maximum response to histamine. The antagonist pA2 is calculated using the following standard equation: pA2 = log(DR-1)-log[B] where DR = dose ratio, defined as EC₅₀antagonist-treated/EC₅₀control and [B] = concentration of antagonist.

To determine the antagonist duration, cells are culture overnight in non-coated black-walled clear bottom 96-well tissue culture plates, are washed with PBS and are incubated with a concentration of antagonist chosen to give an approximate DR in the range 30 - 300. Following the 30 min antagonist incubation period, the cells are washed two or three times with 200 µl of PBS and then 100 µl Tyrodes buffer is added to each well to initiate antagonist dissociation. Following incubation for predetermined times, typically 30 - 270 min at 37 °C, the cells are then washed again with 200 µl PBS and are incubated with 100 µl Tyrodes buffer containing Brilliant Black, probenecid and Fluo-4 for 45 min at 37 °C, as described above. After this period, the cells are challenged with histamine in the FLIPR™ as described above. The dose ratio at each time point is used to determine the fractional H1 receptor occupancy by the following equation: fractional receptor occupancy = (DR-1)/DR. The decrease in receptor occupancy over time approximates to a straight line and is analysed by linear regression. The slope of this straight line fit is used as an index of the dissociation rate of the antagonist. The dose ratios for antagonist treated cells and for antagonist treated and washed cells at each time point are used to calculate a relative dose ratio (rel DR) which is also used as an index of antagonist duration. Antagonists with long duration of action produce rel DR values close to 1, and antagonists with short duration of action produce rel DR values that approaches the dose ratio value obtained for antagonist treatment alone.

### 2. H3 receptor cell line generation, membrane preparation and functional GTPγS assay protocols

### Generation of histamine H3 cell line

The histamine H3 cDNA is isolated from its holding vector, pCDNA3.1 TOPO (InVitrogen), by restriction digestion of plasmid DNA with the enzymes BarnH1 and Not-1 and is ligated into the inducible expression vector pGene (InVitrogen) digested with the same enzymes. The GeneSwitch™ system (a system where in transgene expression is switched off in the absence of an inducer and switched on in the presence of an inducer) is performed as described in US Patents: 5,364,791; 5,874,534; and 5,935,934. Ligated DNA is transformed into competent DH5α *E. coli* host bacterial cells and is plated onto Luria Broth (LB) agar containing Zeocin™ (an antibiotic which allows the selection of cells expressing the *sh ble* gene which is present on pGene and pSwitch) at 50 µgml⁻¹. Colonies containing the re-ligated plasmid are identified by restriction analysis. DNA for transfection into mammalian cells is prepared from 250 ml cultures of the host bacterium containing the pGeneH3 plasmid and is isolated using a DNA preparation kit (Qiagen Midi-Prep) as per manufacturers guidelines (Qiagen).

CHO K1 cells previously transfected with the pSwitch regulatory plasmid (InVitrogen) are seeded at 2×10⁶ cells per T75 flask in Complete Medium, containing Hams F12 (GIBCOBRL, Life Technologies) medium supplemented with 10% v/v dialysed foetal bovine serum, L-glutamine, and hygromycin (100 µgml⁻¹), 24 h prior to use. Plasmid DNA is transfected into the cells using Lipofectamine plus according to the manufacturer's guidelines (InVitrogen). 48 h post transfection, cells are placed into complete medium supplemented with 500 µgml⁻¹ Zeocin™.

10-14 days post selection, 10 nM Mifepristone (InVitrogen) is added to the culture medium to induce the expression of the receptor. 18 h post induction, cells are detached from the flask using ethylenediamine tetra-acetic acid (EDTA; 1:5000; InVitrogen), following several washes with PBS, pH 7.4 and are resuspended in Sorting Medium containing Minimum Essential Medium (MEM), without phenol red, and are supplemented with Earles salts and 3% Foetal Clone II (Hyclone). Approximately 1×10⁷ cells are examined for receptor expression by staining with a rabbit polyclonal antibody, 4a, raised against the *N*-terminal domain of the histamine H3 receptor, are incubated on ice for 60 min, followed by two washes in sorting medium. Receptor bound antibody is detected by incubation of the cells for 60 min on ice with a goat anti rabbit antibody, conjugated with Alexa 488 fluorescence marker (Molecular Probes). Following two further washes with Sorting Medium, cells are filtered through a 50 µm Filcon™ (BD Biosciences) and then are analysed on a FACS Vantage SE Flow Cytometer fitted with an Automatic Cell Deposition Unit. Control cells are non-induced cells treated in a similar manner. Positively stained cells are sorted as single cells into 96-well plates, containing Complete Medium containing 500 µgml⁻¹ Zeocin™ and are allowed to expand before reanalysis for receptor expression via antibody and ligand binding studies. One clone, 3H3, is selected for membrane preparation.

### Membrane preparation from cultured cells

All steps of the protocol are carried out at 4 °C and with pre-cooled reagents. The cell pellet is resuspended in 10 volumes of homogenisation buffer (50 mM *N*-2-hydroxyethylpiperazine-*N*'-2-ethanesulfonic acid (HEPES), 1 mM ethylenediamine tetra-acetic acid (EDTA), pH 7.4 with KOH, supplemented with 10⁻⁶ M leupeptin (acetyl-leucyl-leucyl-arginal; Sigma L2884), 25 µgml⁻¹ bacitracin (Sigma B0125), 1 mM phenylmethylsulfonyl fluoride (PMSF) and 2×10⁻⁶ M pepstain A (Sigma)). The cells are then homogenised by 2 × 15 second bursts in a 1 litre glass Waring blender, followed by centrifugation at 500 g for 20 min. The supernatant is then spun at 48,000 g for 30 min. The pellet is resuspended in homogenisation buffer (4× the volume of the original cell pellet) by vortexing for 5 sec, followed by homogenisation in a Dounce homogeniser (10-15 strokes). At this point the preparation is aliquoted into polypropylene tubes and stored at -80 °C.

### Histamine H3 functional antagonist assay

### For each compound being assayed, in a solid white 384 well plate, is added:-

(a) 0.5 µl of test compound diluted to the required concentration in DMSO (or 0.5 µl DMSO as a control);
(b) 30 µl bead/membrane/GDP mix which is prepared by mixing Wheat Germ Agglutinin Polystyrene LeadSeeker® (WGA PS LS) scintillation proximity assay (SPA) beads with membrane (prepared in accordance with the methodology described above) and diluting in assay buffer (20 mM *N*-2-hydroxyethylpiperazine-*N*'-2-ethanesulfonic acid (HEPES) + 100 mM NaCl + 10 mM MgCl₂, pH 7.4 NaOH) to give a final volume of 30 µl which contains 5 µg protein, 0.25 mg bead per well and 10 µM final assay concentration of guanosine 5' diphosphate (GDP) (Sigma, diluted in assay buffer) incubating at room temperature for 60 min on a roller;
(c) 15 µl 0.38 nM [³⁵S]-GTPγS (Amersham; Radioactivity concentration = 37 MBqml⁻¹; Specific activity = 1160 Cimmol⁻¹), histamine (at a concentration that results in the final assay concentration of histamine being EC₈₀).

After 2-6 h, the plate is centrifuged for 5 min at 1500 rpm and counted on a Viewlux counter using a 613/55 filter for 5 minplate⁻¹. Data is analysed using a 4-parameter logistic equation. Basal activity is used as minimum, i.e. histamine not added to well.

### Intranasal Challenge Method: Whole Body Plethysmography

### (a)Sensitisation

Female Dunkin-Hartley guinea pigs 150-250 g are sensitised twice daily for 5 days (week 1) with ovalbumin (OVA) and aluminium hydroxide (Al(OH)₃ or Alum) in physiological saline, 25 µl/nostril. Solution is made up at 20 µg/ml OVA, 180 mg/ml Alum. During weeks 2 and 3, animals receive 25 µl/nostril of OVA (5 mg/ml) once daily. During Week 4 guinea pigs will be entered into study but are continually sensitized as per weeks 2 and 3 until the day before dosing with compound or vehicle.

### (b) CompoundNehicle Pretreatment

Pretreatment with test compound is performed at various times prior to histamine challenge. Efficacy dose-response curves are determined 1 h after dosing whereas duration of action is studied up to 7 days post dose. Test compounds are formulated as solutions in 0.9% sterile saline or suspensions in 0.9% sterile saline/tween80.

Guinea pigs were anaesthetised with isoflurane (5%, 2-3 I/min O₂), placed in a supine position, and 25 µl of test compound or vehicle dosed into each nostril using a Gilson pipette. After dosing, animals remain supine for at least 30 seconds during recovery from anaesthesia.

### (c) Histamine Challenge Protocol

At 30 min before the time of histamine challenge, guinea pigs are dosed with atropine sulphate (Sigma A0257, dissolved in saline), 1 mg/kg i.p. Animals are then placed into whole body plethysmograph systems (Buxco® Electronics) where the parameter PenH area under curve (AUC) is recorded as outlined in Hamelmann E., Schwarze, J., Takeda, K., Oshiba, A., Larsen, L., Irvin, C.G. and Gelfand, E.W., Am. J. Respir. Crit. Care Med. 156:766-775 (1997). A 10 min baseline AUC is recorded and if this value is over 1000, the animals are excluded.

After the stipulated pre-dose time has been reached, guinea pigs are re-anaesthetised with isoflurane and dosed with either 15 mM histamine or phosphate-buffered saline (PBS), (25 µl per nostril). On recovery from anaesthesia, animals are returned to the individual plethysmograph chambers and 4×10 min consecutive PenH AUC recordings are made. These recordings are summed to give a cumulative AUC over 40 min post histamine challenge for each animal. Data are analysed using ANOVA with post-hoc Fishers LSD test (general linear models, Statistica®) and finally Hochberg adjustment. Inhibition of histamine-induced congestion is determined by statistically significant differences between the mean responses of compound pre-treated groups compared to the vehicle pre-treated, histamine-challenged group.

### CNS penetration

### (i) CNS penetration by bolus administration

Compounds are dosed intravenously at a nominal dose level of 1 mg/kg to male CD Sprague Dawley rats. Compounds are formulated in 5% DMSO/45% PEG200/50% water. Blood samples are taken under terminal anaesthesia with isoflurane at 5 min post-dose and the brains are also removed for assessment of brain penetration. Blood samples are taken directly into heparinised tubes. Blood samples are prepared for analysis using protein precipitation and brain samples are prepared using extraction of drug from brain by homogenisation and subsequent protein precipitation. The concentration of parent drug in blood and brain extracts is determined by quantitative LC-MS/MS analysis using compound-specific mass transitions.

### (ii) CNS penetration following intravenous infusion at steady state

A loading dose of the compounds is given to male CD Sprague Dawley rats at a nominal dose level of 0.4 mg/kg. The compounds are then infused intravenously for 4 h at a nominal dose level of 0.1 mg/kg/h. Compounds are formulated in 2% DMSO/30% PEG200/68% water. Serial or terminal blood samples are taken at 0.5, 1.5, 2.5, 3, 3.5 and 4 h post dose. The final blood sample is collected under terminal anaesthesia with isoflurane and the brains are also removed for assessment of brain penetration. Blood samples are taken directly into heparinised tubes. Blood samples are prepared for analysis using protein precipitation and brain samples are prepared using extraction of drug from brain by homogenisation and subsequent protein precipitation. The concentration of parent drug in blood and brain extracts is determined by quantitative LC-MS/MS analysis using compound-specific mass transitions.

### Rat pharmacokinetics

Compounds are dosed to male CD Sprague Dawley rats by single intravenous or oral administration at a nominal dose level of 1mg/kg and 3mg/kg respectively. Compounds are formulated in 5% DMSO/45% PEG200/50% water. An intravenous profile is obtained by taking serial or terminal blood samples at 0.083, 0.25, 0.5, 1, 2, 4, and 7 h post dose (for some studies 12 and 24 h samples may be taken). An oral profile is obtained by taking serial or terminal blood samples at 0.25, 0.5, 1, 2, 4, 7 and 12 h post dose (for some studies 24 and 30 h samples may be taken). Blood samples are taken directly into heparinised tubes. Blood samples are prepared by protein precipitation and subjected to quantitative analysis by LC-MS/MS using compound-specific mass transitions. Drug concentration-time profiles are generated and non-compartmental PK analysis used to generate estimates of half-life, clearance, volume of distribution and oral bioavailability.

### Dog pharmacokinetics

Compounds are dosed to male Beagle dogs by single intravenous or oral administration at a nominal dose level of 1 mg/kg and 2 mg/kg respectively. The study is carried out according to a crossover design such that the same dog is used for both dosing events and the dosing events occurred 1 week apart. Compounds are formulated in 5%DMSO/45%Peg200/50%water. An intravenous profile is obtained by taking serial blood samples at 0.083, 0.25, 0.5, 0.75, 1, 2, 4, 6 and 12 h post dose (for some studies 24 h samples may be taken). An oral profile is obtained by taking serial blood samples at 0.25, 0.5, 0.75, 1, 2, 4, 6, 12 and 24 h post dose. Blood samples are taken directly into heparinised tubes. Blood samples are prepared by protein precipitation and subjected to quantitative analysis by LC-MS/MS using compound-specific mass transitions. Drug concentration-time profiles are generated and non-compartmental PK analysis used to generate estimates of half-life, clearance, volume of distribution and oral bioavailability.

### Results

The compounds of Examples 1 to 23 were tested in the above or similar assays/methods and showed:
(i) The compounds of the Examples had an average pKᵢ (pK_{b}) at H1 greater than approximately 7. The compound of Example 7 had an average pKᵢ (pK_{b}) at H1 greater than approximately 8.
   The compounds of Examples 1-3, 5-9, 11-13, 15, 18 and 23 had average pA2 values at H1 of greater than approximately 7. The compounds of Examples 3, 8, 11, 12, 13, 15 and 18 had average pA2 values at H1 of greater than approximately 8. The compounds of Examples 6, 7, 9 and 23 had average pA2 values at H1 of greater than approximately 9.
(ii) The compounds of the Examples had an average pKᵢ (pK_{b}) at H3 of greater than approximately 8. The compounds of Examples 1, 2, 5-10, 12, 14-20 and 23 had an average pKᵢ (pK_{b}) at H3 of greater than approximately 9.
(iii) The compounds of Examples 7, 8, 9, 11, 12, 13, 15, 18, and 23 had (at one or more time points) a longer duration of action than azelastine in the histamine H1 functional antagonist assay.
(iv) The compounds of Example 7 and 13 demonstrated lower CNS penetration than azelastine. The compound of Example 16 demonstrated comparable CNS penetration to azelastine.

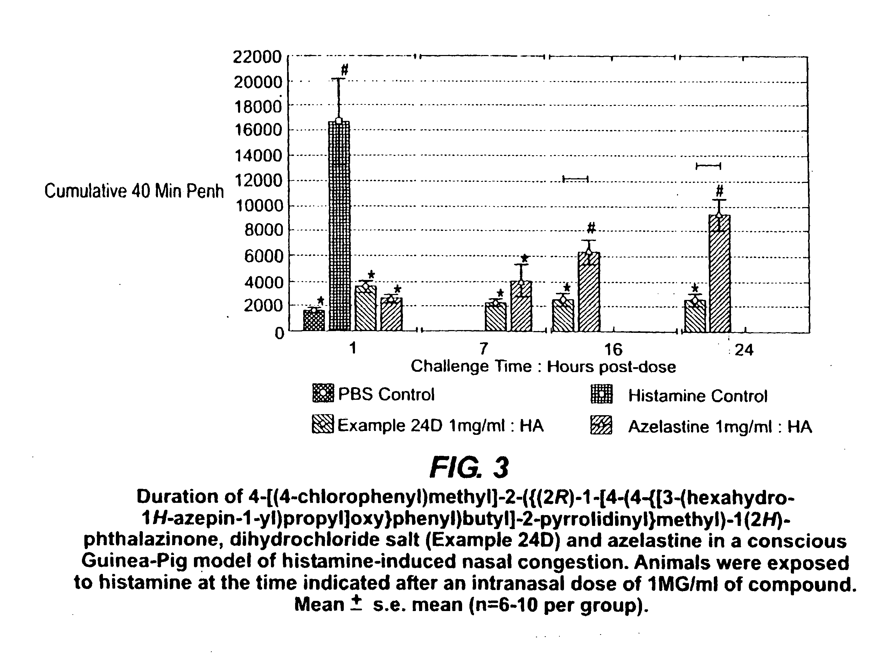

The compound of Example 24 and various salts thereof were tested in the above or similar assays/methods and showed:
i) an average pKᵢ (pK_{b}) at H1 of approximately 7.8 and an average pA2 value at H1 of approximately 8.9.
ii) an average pKᵢ (pK_{b}) at H3 of approximately 9.6.
iii) at one or more time points, a significantly longer duration of action than azelastine in the histamine H 1 functional antagonist assay.
iv) a statistically significant inhibition of nasal congestion at 24 hours after dosing compared to azelastine in the Guinea Pig whole body plethysmography model, (Figure 3). v) lower CNS penetration than azelastine.

## Claims

1. A compound of formula (I) 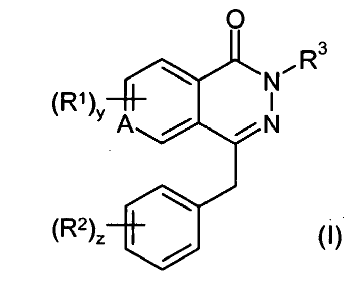 wherein
A represents N or CH;
R¹ and R² each independently represent halogen, C₁₋₆alkyl, C₁₋₆alkoxy, hydroxyl or trifluoromethyl;
y and z each independently represent 0, 1 or 2;
R³ represents the group -(CH₂)ₐNR⁴R⁵ or a group of formula (i) 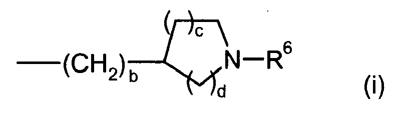 in which
a represents 1, 2 or 3;
b represents 0 or 1;
c represents 0, 1 or 2 and d represents 0, 1, 2 or 3, such that c and d cannot both be 0;
R⁴ represents hydrogen or C₁₋₆alkyl and R⁵ and R⁶ each independently represent a group selected from the formulae (a), (b) or (c) 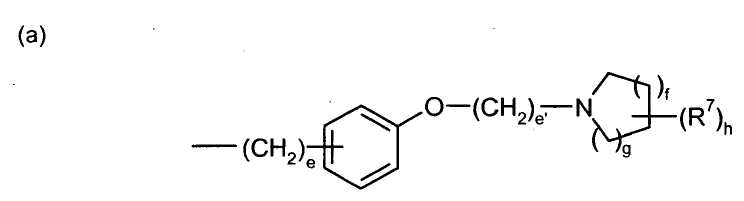 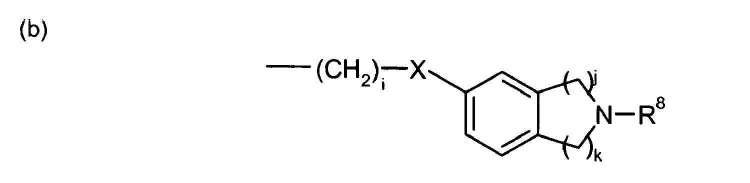 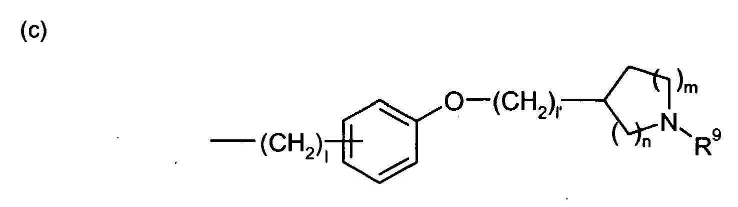 in which, for formula (a)
e represents 1 to 6;
e' represents 2 to 4;
f represents 0, 1 or 2 and g represents 0, 1, 2 or 3, such that f and g cannot both be 0;
h represents 0, 1 or 2;
R⁷ represents C₁₋₃alkyl;
in which, for formula (b)
i represents 1 to 6;
X represents either a bond, O or -N(R¹⁰)C(O)-, in which R¹⁰ represents hydrogen or C₁₋₆alkyl;
j and k each represent 1 or each represent 2;
R⁸ represents hydrogen, C₃₋₆cycloalkyl or C₁₋₆alkyl;
in which, for formula (c)
I represents 1 to 6;
I' represents 0 to 3;
m represents 0, 1 or 2 and n represents 0, 1, 2 or 3, such that m and n cannot both be 0, and such that I' plus n must represent 1, 2 or 3;
R⁹ represents hydrogen, C₃₋₆cycloalkyl or C₁₋₆alkyl;
or a salt thereof.

2. A compound according to claim 1, wherein A represents CH.

3. A compound according to claim 1 or claim 2, wherein z represents 1, and R² is substituted in the 4-position (para).

4. A compound according to any of claims 1 to 3, wherein R³ represents a group of formula (i).

5. A compound according to claim 4, wherein R⁶ represents a group of formula (a).

6. A compound according to any of claims 1 to 3, wherein R³ represents the group -(CH₂)ₐNR⁴R⁵ and R⁵ represents a group of formula (b).

7. A compound which is:
4-[(4-Chlorophenyl)methyl]-2-(2-{[4-(4-{[3-(hexahydro-1*H*-azepin-1-yl)propyl]oxy}phenyl)butyl]amino}ethyl)-1(2*H*)-phthalazinone;
4-[(4-Chlorophenyl)methyl]-2-(2-{[2-(4-{[3-(hexahydro-1*H*-azepin-1-yl)propyl]oxy} phenyl)ethyl]amino}ethyl)-1(2*H*)-phthalazinone;
4-[(4-Chlorophenyl)methyl]-2-{2-[[4-(4-{[3-(hexahydro-1*H*-azepin-1-yl)propyl]oxy} phenyl)butyl](methyl)amino]ethyl}-1(2*H*)-phthalazinone;
4-[(4-Chlorophenyl)methyl]-2-{2-[[2-(4-{[3-(hexahydro-1*H*-azepin-1-yl)propyl]oxy} phenyl)ethyl](methyl)amino]ethyl}-1(2*H*)-phthalazinone;
4-[(4-Chlorophenyl)methyl]-2-({(2*R*)-1-[5-(4-{[3-(hexahydro-1*H*-azepin-1-yl)propyl] oxy}phenyl)pentyl]-2-pyrrolidinyl}methyl)-1(2*H*)phthalazinone;
2-({(2*R*)-1-[4-(4-{[3-(Hexahydro-1*H*-azepin-1-yl)propyl]oxy}phenyl)butyl]-2-pyrrolidinyl}methyl)-4-{[4-(methyloxy)phenyl]methyl}-1(2*H*)-phthalazinone;
2-({(2*R*)-1-[4-(4-{[3-(Hexahydro-1*H*-azepin-1-yl)propyl]oxy}phenyl)butyl]-2-pyrrolidinyl}methyl)-4-[(4-hydroxyphenyl)methyl]-1(2*H*)-phthalazinone;
4-[(4-Fluorophenyl)methyl]-2-({(2*R*)-1-[4-(4-{[3-(hexahydro-1*H*-azepin-1-yl)propyl]oxy} phenyl)butyl]-2-pyrrolidinyl}methyl)-1(2*H*)-phthalazinone;
4-[(4-Chlorophenyl)methyl]-2-{1-[(4-{[3-(hexahydro-1*H*-azepin-1-yl)propyl]oxy}phenyl) methyl]hexahydro-1*H*-azepin-4-yl}-1(2*H*)-phthalazinone;
4-[(4-Chlorophenyl)methyl]-2-{1-[2-(4-{[3-(hexahydro-1*H*-azepin-1-yl)propyl]oxy} phenyl)ethyl]hexahydro-1*H*-azepin-4-yl}-1(2*H*)-phthalazinone;
4-[(4-Chlorophenyl)methyl]-2-{1-[3-(4-{[3-(hexahydro-1*H*-azepin-1-yl)propyl]oxy} phenyl)propyl]hexahydro-1*H*-azepin-4-yl}-1(2*H*)-phthalazinone;
4-[(4-Chlorophenyl)methyl]-2-{1-[3-(4-{[3-(hexahydro-1*H*-azepin-1-yl)propyl]oxy} phenyl)propyl]hexahydro-1*H*-azepin-4-yl}-1(2*H*)-phthalazinone;
4-[(4-Chlorophenyl)methyl]-2-[((2*S*)-1-{3-[(3-cyclobutyl-2,3,4,5-tetrahydro-1*H*-3-benzazepin-7-yl)oxy]propyl}-2-pyrrolidinyl)methyl]-1(2*H*)-phthalazinone;
4-[(4-Chlorophenyl)methyl]-2-[((2*R*)1-{3-[(3-cyclobutyl-2,3,4,5-tetrahydro-1*H*-3-benzazepin-7-yl)oxy]propyl}-2-pyrrolidinyl)methyl]-1(2*H*)-phthalazinone;
4-[(4-Chlorophenyl)methyl]-2-(1-{3-[(3-cyclobutyl-2,3,4,5-tetrahydro-1*H*-3-benzazepin-7-yl)oxy]propyl}hexahydro-1*H*-azepin-4-yl)-1(2*H*)-phthalazinone;
4-[(4-Chlorophenyl)methyl]-2-{1-[(3-cyclobutyl-2,3,4,5-tetrahydro-1*H*-3-benzazepin-7-yl)methyl]hexahydro-1H-azepin-4-yl}-1(2*H*)-phthalazinone;
*N*-(2-{4-[4-[(4-Chlorophenyl)methyl]-1-oxo-2(1*H*)-phthalazinyl]hexahydro-1*H*-azepin-1-yl}ethyl)-3-cyclobutyl-2,3,4,5-tetrahydro-1*H*-3-benzazepine-7-carboxamide;
*N*-(3-{4-[4-[(4-Chlorophenyl)methyl]-1-oxo-2(1*H*)-phthalazinyl]hexahydro-1*H*-azepin-1-yl}propyl)-3-cyclobutyl-2,3,4,5-tetrahydro-1*H*-3-benzazepine-7-carboxamide;
*N*-(4-{4-[4-[(4-Chlorophenyl)methyl]-1-oxo-2(1*H*)-phthalazinyl]hexahydro-1*H*-azepin-1-yl}butyl)-3-cyclobutyl-2,3,4,5-tetrahydro-1*H*-3-benzazepine-7-carboxamide;
4-[(4-Chlorophenyl)methyl]-2-[1-(2-{4-[(1-cyclobutyl-4-piperidinyl)oxy]phenyl}ethyl) hexahydro-1*H*-azepin-4-yl]-1(2*H*)-phthalazinone;
4-[(4-Chlorophenyl)methyl]-2-[1-(4-{4-[(1-cyclobutyl-4-piperidinyl)oxy]phenyl}butyl) hexahydro-1*H*-azepin-4-yl]-1(2*H*)-phthalazinone; or
4-[(4-Chlorophenyl)methyl]-2-({(2*R*)-1-[4-(4-{[3-(hexahydro-1*H*-azepin-1-yl)propyl] oxy}phenyl)butyl]-2-pyrrolidinyl}methyl)pyrido[3,4-*d*]pyridazin-1(2*H*)-one;
or a salt thereof.

8. A compound which is: 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone, or a salt thereof.

9. A compound which is: 4-[(4-chlorophenyl)methyl]-2-({(2*S*)-1-[4-(4-{[3-(hexahydro-1*H*-azepin-1-yl)propyl]oxy}phenyl)butyl]-2-pyrrolidinyl}methyl)-1(2*H*)-phthalazinone, or a salt thereof.

10. A compound which is: 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone in the form of its free base.

11. A compound which is: 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone, in the form of a 1,5-naphthalene disulfonate salt.

12. A compound which is: 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone, in the form of a 1,5-naphthalene disulfonate monohydrate salt.

13. A compound according to any of claims 1 to 9, or a pharmaceutically acceptable salt thereof.

14. A compound as defined in any of claims 1 to 9, or a pharmaceutically acceptable salt thereof for use in therapy.

15. A compound which is: 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl}methyl)-1(2*H*)-phthalazinone, or a 1,5-naphthalene disulfonate monohydrate salt thereof for use in therapy.

16. A compound according to claim 14 or claim 15, for use in the treatment of inflammatory and/or allergic diseases of the respiratory tract.

17. A compound according to claim 16, for use in the treatment of allergic rhinitis.

18. A compound according to claim 16, for use in the treatment of non-allergic rhinitis.

19. A composition which comprises a compound of formula (I) as defined in any of claims 1 to 9, or a pharmaceutically acceptable salt thereof, and one or more pharmaceutically acceptable carriers and/or excipients.

20. A composition which comprises compound which is: 4-[(4-chlorophenyl)methyl]-2-({(2*R*)-1-[4-(4-{[3-(hexahydro-1*H*-azepin-1-yl)propyl]oxy} phenyl)butyl]-2-pyrrolidinyl}methyl)-1(2*H*)-phthalazinone, or a 1,5-naphthalene disulfonate monohydrate salt, and one or more pharmaceutically acceptable carriers and/or excipients.

21. A composition according to claim 19 or claim 20, wherein said composition is adapted for intranasal delivery.

22. A combination comprising a compound as defined in any of claims 1 to 9, or a pharmaceutically acceptable salt thereof, and one or more other therapeutic agents.

23. A combination comprising a compound which is: 4-[(4-chlorophenyl)methyl]-2-({(2R)-1-[4-(4-{[3-(hexahydro-1*H*-azepin-1-yl)propyl]oxy}phenyl)butyl]-2-pyrrolidinyl} methyl)-1(2*H*)-phthalazinone, or a 1,5-naphthalene disulfonate monohydrate salt, and one or more other therapeutic agents.

24. A combination according to claim 22 or claim 23 wherein the one or more other therapeutic agents comprises 6α,9α-difluoro-17α-[(2-furanylcarbonyl)oxy]-11β-hydroxy-16α-methyl-3-oxo-androsta-1,4-diene-17β-carbothioic acid S-fluoromethyl ester (fluticasone furoate).

25. A combination according to any of claims 22 to 24 wherein said combination is adapted for intranasal delivery.

26. The use of a compound as defined in any of claims 1 to 9, or a pharmaceutically acceptable salt thereof, in the manufacture of a medicament for the treatment of inflammatory and/or allergic diseases of the respiratory tract.

27. The use of a compound which is: 4-[(4-chlorophenyl)methyl]-2-({(2*R*)-1-[4-(4-{[3-(hexahydro-1*H*-azepin-1-yl)propyl]oxy}phenyl)butyl]-2-pyrrolidinyl} methyl)-1(2*H*)-phthalazinone, or a 1,5-naphthalene disulfonate monohydrate salt in the manufacture of a medicament for the treatment of inflammatory and/or allergic diseases of the respiratory tract.

28. The use according to claim 26 or claim 27, in which the disease is allergic rhinitis.

29. The use according to claim 26 or claim 27, in which the disease is non-allergic rhinitis.

## Patentansprüche

1. Verbindung der Formel (I): 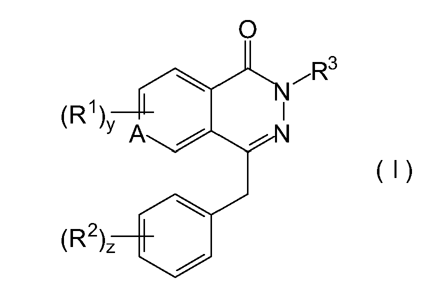 worin
A N oder CH darstellt;
R¹ und R² jeweils unabhängig voneinander Halogen, C₁₋₆-Alkyl, C₁₋₆-Alkoxy, Hydroxyl oder Trifluormethyl darstellen;
y und z jeweils unabhängig voneinander 0, 1 oder 2 darstellen;
R³ die Gruppe -(CH₂)ₐ-NR⁴R⁵ oder eine Gruppe der Formel (i) darstellt: 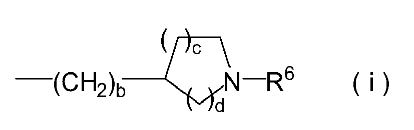 worin
a 1, 2 oder 3 darstellt;
b 0 oder 1 darstellt;
c 0, 1 oder 2 darstellt und d 0, 1, 2 oder 3 darstellt, und c und d nicht beide 0 sein können;
R⁴ Wasserstoff oder C₁₋₆-Alkyl darstellt und R⁵ und R⁶ jeweils unabhängig voneinander eine Gruppe, ausgewählt aus den Formeln (a), (b) oder (c), darstellen: 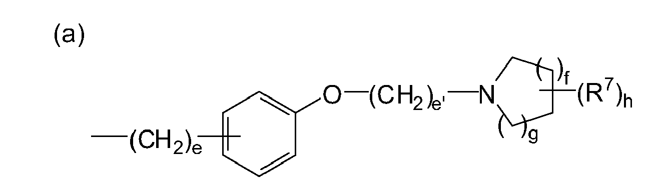 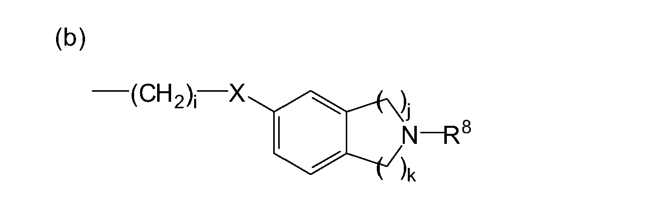 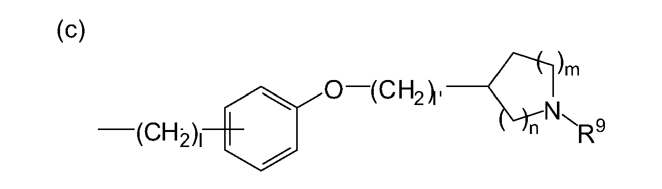 wobei in Formel (a)
e 1 bis 6 darstellt;
e' 2 bis 4 darstellt;
f 0, 1 oder 2 darstellt und g 0, 1, 2 oder 3 darstellt, und f und g nicht beide 0 sein können;
h 0, 1 oder 2 darstellt;
R⁷ C₁₋₃-Alkyl darstellt;
wobei in Formel (b)
i 1 bis 6 darstellt;
X entweder eine Bindung, O oder -N(R¹⁰)C(O)-darstellt, worin R¹⁰ Wasserstoff oder C₁₋₆-Alkyl darstellt;
j und k jeweils 1 oder 2 darstellen;
R⁸ Wasserstoff, C₃₋₆-Cycloalkyl oder C₁₋₆-Alkyl darstellt;
wobei in Formel (c)
l 1 bis 6 darstellt;
l' 0 bis 3 darstellt;
m 0, 1 oder 2 darstellt und n 0, 1, 2 oder 3 darstellt, und m und n nicht beide 0 sein können und
l' plus n 1, 2 oder 3 darstellen müssen;
R⁹ Wasserstoff, C₃₋₆-Cycloalkyl oder C₁₋₆-Alkyl darstellt;
oder ein Salz davon.

2. Verbindung gemäss Anspruch 1, worin A CH darstellt.

3. Verbindung gemäss Anspruch 1 oder Anspruch 2, worin z 1 darstellt und R² in der 4-Position (para) substituiert ist.

4. Verbindung gemäss irgendeinem der Ansprüche 1 bis 3, worin R³ eine Gruppe der Formel (i) darstellt.

5. Verbindung gemäss Anspruch 4, worin R⁶ eine Gruppe der Formel (a) darstellt.

6. Verbindung gemäss irgendeinem der Ansprüche 1 bis 3, worin R³ die Gruppe -(CH₂)ₐNR⁴R⁵ darstellt und R⁵ eine Gruppe der Formel (b) darstellt.

7. Verbindung, die ist:
4-[(4-Chlorphenyl)methyl]-2-(2-{[4-(4-{[3-(hexahydro-1H-azepin-1-yl)propyl]oxy}phenyl)butyl]amino}ethyl)-1(2H)-phthalazinon;
4-[(4-Chlorphenyl)methyl]-2-(2-{[2-(4-{[3-(hexahydro-1H-azepin-1-yl)propyl]oxy}phenyl)ethyl]amino}ethyl)-1(2H)-phthalazinon;
4-[(4-Chlorphenyl)methyl]-2-{2-[[4-(4-{[3-(hexahydro-1H-azepin-1-yl)propyl]oxy}phenyl)butyl](methyl)-amino]ethyl}-1(2H)-phthalazinon;
4-[(4-Chlorphenyl)methyl]-2-{2-[[2-(4-{[3-(hexahydro-1H-azepin-1-yl)propyl]oxy}phenyl)ethyl](methyl)-amino]ethyl}-1(2H)-phthalazinon;
4-[(4-Chlorphenyl)methyl]-2-({(2R)-1-[5-(4-{[3-(hexahydro-1H-azepin-1-yl)propyl]oxy}phenyl)pentyl]-2-pyrrolidinyl}methyl)-1(2H)-phthalazinon;
2-({(2R)-1-[4-(4-{[3-(Hexahydro-1H-azepin-1-yl)propyl]oxy}phenyl)butyl]-2-pyrrolidinyl}methyl)-4-{[4-(methyloxy)phenyl]methyl}-1(2H)-phthalazinon;
2-({(2R)-1-[4-(4-{[3-(Hexahydro-1H-azepin-1-yl)propyl]oxy}phenyl)butyl]-2-pyrrolidinyl}methyl)-4-[(4-hydroxyphenyl)methyl]-1(2H)-phthalazinon;
4-[(4-Fluorphenyl)methyl]-2-({(2R)-1-[4-(4-{[3-(hexahydro-1H-azepin-1-yl)propyl]oxy}phenyl)butyl]-2-pyrrolidinyl}methyl)-1(2H)-phthalazinon;
4-[(4-Chlorphenyl)methyl]-2-{1-[(4-{[3-(hexahydro-1H-azepin-1-yl)propyl]oxy}phenyl)methyl]hexahydro-1H-azepin-4-yl}-1(2H)-phthalazinon;
4-[(4-Chlorphenyl)methyl]-2-{1-[2-(4-{[3-(hexahydro-1H-azepin-1-yl)propyl]oxy}phenyl)ethyl]hexahydro-1H-azepin-4-yl}-1(2H)-phthalazinon;
4-[(4-Chlorphenyl)methyl]-2-{1-[3-(4-{[3-(hexahydro-1H-azepin-1-yl)propyl]oxy}phenyl)propyl]hexahydro-1H-azepin-4-yl}-1(2H)-phthalazinon;
4-[(4-Chlorphenyl)methyl]-2-{1-[3-(4-{[3-(hexahydro-1H-azepin-1-yl)propyl]oxy}phenyl)propyl]hexahydro-1H-azepin-4-yl}-1(2H)-phthalazinon;
4-[(4-Chlorphenyl)methyl]-2-[((2S)-1-{3-[(3-cyclobutyl-2,3,4,5-tetrahydro-1H-3-benzazepin-7-yl)oxy]propyl}-2-pyrrolidinyl)methyl]-1(2H)-phthalazinon;
4-[(4-Chlorphenyl)methyl]-2-[((2R)-1-{3-[(3-cyclobutyl-2,3,4,5-tetrahydro-1H-3-benzazepin-7-yl)oxy]propyl}-2-pyrrolidinyl)methyl]-1(2H)-phthalazinon;
4-[(4-Chlorphenyl)methyl]-2-(1-{3-[(3-cyclobutyl-2,3,4,5-tetrahydro-1H-3-benzazepin-7-yl)oxy]propyl}-hexahydro-1H-azepin-4-yl)-1(2H)-phthalazinon;
4-[(4-Chlorphenyl)methyl]-2-{1-[(3-cyclobutyl-2,3,4,5-tetrahydro-1H-3-benzazepin-7-yl)methyl]hexahydro-1H-azepin-4-yl)-1(2H)-phthalazinon;
N-(2-{4-[4-[(4-Chlorphenyl)methyl]-1-oxo-2(1H)-phthalazinyl]hexahydro-1H-azepin-1-yl}ethyl)-3-cyclobutyl-2,3,4,5-tetrahydro-1H-3-benzazepin-7-carboxamid;
N-(3-{4-[4-[(4-Chlorphenyl)methyl]-1-oxo-2(1H)-phthalazinyl]hexahydro-1H-azepin-1-yl}propyl)-3-cyclobutyl-2,3,4,5-tetrahydro-1H-3-benzazepin-7-carboxamid;
N-(4-{4-[4-[(4-Chlorphenyl)methyl]-1-oxo-2(1H)-phthalazinyl]hexahydro-1H-azepin-1-yl}butyl)-3-cyclobutyl-2,3,4,5-tetrahydro-1H-3-benzazepin-7-carboxamid;
4-[(4-Chlorphenyl)methyl]-2-[1-(2-{4-[(1-cyclobutyl-4-piperidinyl)oxy]phenyl}ethyl)hexahydro-1H-azepin-4-yl]-1(2H)-phthalazinon;
4-[(4-Chlorphenyl)methyl]-2-[1-(4-{4-[(1-cyclobutyl-4-piperidinyl)oxy]phenyl}butyl)hexahydro-1H-azepin-4-yl]-1(2H)-phthalazinon;oder
4-[(4-Chlorphenyl)methyl]-2-({(2R)-1-[4-(4-{[3-(hexahydro-1H-azepin-1-yl)propyl]oxy}phenyl)butyl]-2-pyrrolidinyl}methyl)pyrido[3,4-d]pyridazin-1(2H)-on;
oder ein Salz davon.

8. Verbindung, die 4-[(4-Chlorphenyl)methyl]-2-({(2R)-1-[4-(4-{[3-(hexahydro-1H-azepin-1-yl)propyl]oxy}-phenyl)butyl]-2-pyrrolidinyl}methyl)-1(2H)-phthalazinon oder ein Salz davon ist.

9. Verbindung, die 4-[(4-Chlorphenyl)methyl]-2-({(2S)-1-[4-(4-{[3-(hexahydro-1H-azepin-1-yl)propyl]oxy}-phenyl)butyl]-2-pyrrolidinyl}methyl)-1(2H)-phthalazinon oder ein Salz davon ist.

10. Verbindung, die 4-[(4-Chlorphenyl)methyl]-2-({(2R)-1-[4-(4-{[3-(hexahydro-1H-azepin-1-yl)propyl]oxy}-phenyl)butyl]-2-pyrrolidinyl}methyl)-1(2H)-phthalazinon ist, in Form ihrer freien Base.

11. Verbindung, die 4-[(4-Chlorphenyl)methyl]-2-({(2R)-1-[4-(4-{[3-(hexahydro-1H-azepin-1-yl)propyl]oxy}-phenyl)butyl]-2-pyrrolidinyl}methyl)-1(2H)-phthalazinon in Form eines 1,5-Naphthalindisulfonatsalzes ist.

12. Verbindung, die 4-[(4-Chlorphenyl)methyl]-2-({(2R)-1-[4-(4-{[3-(hexahydro-1H-azepin-1-yl)propyl]oxy}-phenyl)butyl]-2-pyrrolidinyl}methyl)-1(2H)-phthalazinon in Form eines 1,5-Naphthalindisulfonatmonohydratsalzes ist.

13. Verbindung gemäss irgendeinem der Ansprüche 1 bis 9 oder ein pharmazeutisch annehmbares Salz davon.

14. Verbindung, wie in irgendeinem der Ansprüche 1 bis 9 definiert, oder ein pharmazeutisch annehmbares Salz davon zur Verwendung in der Therapie.

15. Verbindung, die 4-[(4-Chlorphenyl)methyl]-2-({(2R)-1-[4-(4-{[3-(hexahydro-1H-azepin-1-yl)propyl]oxy}-phenyl)butyl]-2-pyrrolidinyl}methyl)-1(2H)-phthalazinon oder ein 1,5-Naphthalindisulfonatmonohydratsalzes davon ist, zur Verwendung in der Therapie.

16. Verbindung gemäss Anspruch 14 oder Anspruch 15 zur Verwendung bei der Behandlung einer entzündlichen und/oder allergischen Erkrankung der Atemwege.

17. Verbindung gemäss Anspruch 16 zur Verwendung bei der Behandlung von allergischer Rhinitis.

18. Verbindung gemäss Anspruch 16 zur Verwendung bei der Behandlung von nicht-allergischer Rhinitis.

19. Zusammensetzung, die eine Verbindung der Formel (I), wie in irgendeinem der Ansprüche 1 bis 9 definiert, oder ein pharmazeutisch annehmbares Salz davon und einen oder mehrere pharmazeutisch annehmbare(n) Träger und/oder Exzipienten umfasst.

20. Zusammensetzung, die eine Verbindung, die 4-[(4-Chlorphenyl)methyl]-2-({(2R)-1-[4-(4-{[3-(hexahydro-1H-azepin-1-yl)propyl]oxy}-phenyl)butyl]-2-pyrrolidinyl}methyl)-1(2H)-phthalazinon oder ein 1,5-Naphthalindisulfonatmonohydratsalzes davon ist, und einen oder mehrere pharmazeutisch annehmbare(n) Träger und/oder Exzipienten umfasst.

21. Zusammensetzung gemäss Anspruch 19 oder Anspruch 20, wobei die Zusammensetzung zur intranasalen Abgabe angepasst ist.

22. Kombination, umfassend eine Verbindung, wie in irgendeinem der Ansprüche 1 bis 9 definiert, oder ein pharmazeutisch annehmbares Salz davon und ein oder mehrere andere(s) therapeutische(s) Mittel.

23. Kombination, umfassend eine Verbindung, die 4-[(4-Chlorphenyl)methyl]-2-({(2R)-1-[4-(4-{[3-(hexahydro-1H-azepin-1-yl)propyl]oxy}-phenyl)butyl]-2-pyrrolidinyl}methyl)-1(2H)-phthalazinon oder ein 1,5-Naphthalindisulfonatmonohydratsalzes davon ist, und ein oder mehrere andere(s) therapeutische(s) Mittel.

24. Kombination gemäss Anspruch 22 oder Anspruch 23, wobei das/die ein(e) oder mehreren andere(n) therapeutische(n) Mittel 6α,9α-Difluor-17α-[(2-furanylcarbonyl)oxy]-11β-hydroxy-16α-methyl-3-oxo-androsta-1,4-dien-17β-carbothionsäure-S-fluormethylester (Fluticasonfuroat) umfasst.

25. Kombination gemäss irgendeinem der Ansprüche 22 bis 24, wobei die Kombination zur intranasalen Abgabe angepasst ist.

26. Verwendung einer Verbindung, wie in irgendeinem der Ansprüche 1 bis 9 definiert, oder eines pharmazeutisch annehmbaren Salzes davon bei der Herstellung eines Medikaments zur Behandlung einer entzündlichen und/oder allergischen Erkrankung der Atemwege.

27. Verwendung einer Verbindung, die 4-[(4-Chlorphenyl)methyl]-2-({(2R)-1-[4-(4-{[3-(hexahydro-1H-azepin-1-yl)propyl]oxy}-phenyl)butyl]-2-pyrrolidinyl}methyl)-1(2H)-phthalazinon ist, oder eines 1,5-Naphthalindisulfonatmonohydratsalzes davon bei der Herstellung eines Medikaments zur Behandlung einer entzündlichen und/oder allergischen Erkrankung der Atemwege.

28. Verwendung gemäss Anspruch 26 oder Anspruch 27, wobei die Erkrankung allergische Rhinitis ist.

29. Verwendung gemäss Anspruch 26 oder Anspruch 27, wobei die Erkrankung nicht-allergische Rhinitis ist.

## Revendications

1. Composé de formule (I) 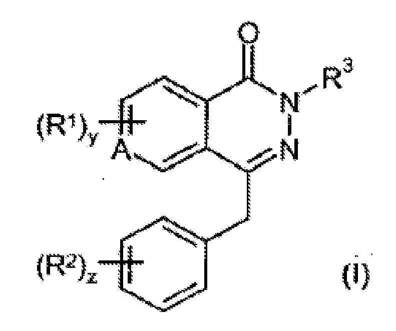 dans laquelle
A représente N ou CH ;
R¹ et R² représentent chacun indépendamment un halogène, un alkyle en C₁ à C₆, un alcoxy en C₁ à C₆, un hydroxyle ou un trifluorométhyle ;
y et z représentent chacun indépendamment 0, 1 ou 2 ;
R³ représente le groupe -(CH₂)ₐNR⁴R⁵ ou un groupe de formule (i) 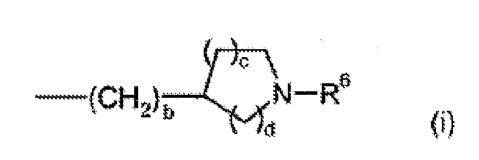 dans laquelle
a représente 1, 2 ou 3 ;
b représente 0 ou 1 ;
c représente 0, 1 ou 2 et d représente 0, 1, 2 ou 3, de telle sorte que c et d ne peuvent pas être tous les deux 0 ;
R⁴ représente un hydrogène ou un alkyle en C₁ à C₆, et R⁵ et R⁶ représentent chacun indépendamment un groupe choisi parmi les formules (a), (b) ou (c) 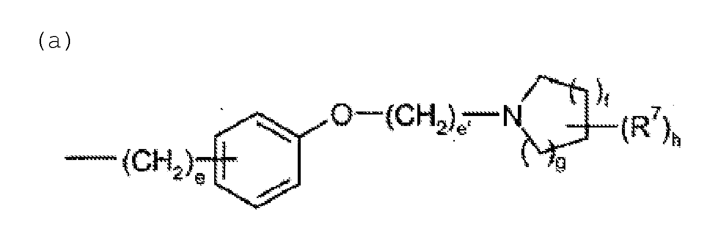 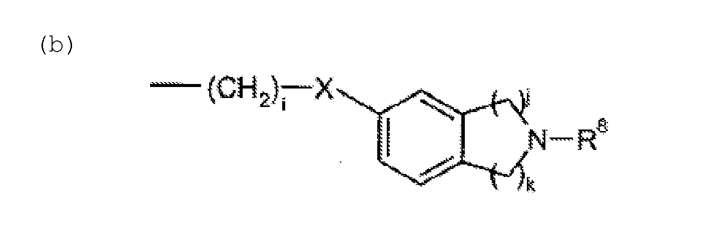 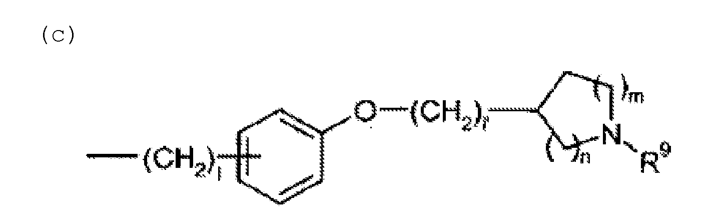 dans laquelle, pour la formule (a)
e représente 1 à 6 ;
e' représente 2 à 4 ;
f représente 0, 1 ou 2 et g représente 0, 1, 2 ou 3, de telle sorte que f et g ne peuvent pas être tous les deux 0 ;
h représente 0, 1 ou 2 ;
R⁷ représente un alkyle en C₁ à C₃ ;
dans laquelle, pour la formule (b) i représente 1 à 6 ;
X représente une liaison, O ou -N(R¹⁰)C(O)-, où R¹⁰ représente un hydrogène ou un alkyle en C₁ à C₆ ;
j et k représentent chacun 1 ou représentent chacun 2 ;
R⁸ représente un hydrogène, un cycloalkyle en C₃ à C₆ ou un alkyle en C₁ à C₆ ;
dans laquelle, pour la formule (c)
I représente 1 à 6 ;
I' représente 0 à 3 ;
m représente 0, 1 ou 2 et n représente 0, 1, 2 ou 3, de telle sorte que m et n ne peuvent pas être tous les deux 0, et de telle sorte que I' plus n doivent représenter 1, 2 ou 3 ;
R⁹ représente un hydrogène, un cycloalkyle en C₃ à C₆ ou un alkyle en C₁ à C₆ ;
ou l'un de ses sels.

2. Composé selon la revendication 1, dans lequel A représente CH.

3. Composé selon la revendication 1 ou la revendication 2, dans lequel z représente 1, et R² est substitué en position 4 (para).

4. Composé selon l'une quelconque des revendications 1 à 3, dans lequel R³ représente un groupe de formule (i).

5. Composé selon la revendication 4, dans lequel R⁶ représente un groupe de formule (a).

6. Composé selon l'une quelconque des revendications 1 à 3, dans lequel R³ représente le groupe -(CH₂)ₐNR⁴R⁵ et R⁵ représente un groupe de formule (b).

7. Composé qui est :
la 4-[(4-chlorophényl)méthyl]-2-(2-{[4-(4-{[3-(hexahydro-1*H*-azépin-1-yl)propyl]oxy}phényl)butyl]-amino}éthyl)-1(2*H*)-phtalazinone ;
la 4-[(4-chlorophényl)méthyl]-2-(2-{[2-(4-{[3-(hexahydro-1*H*-azépin-1-yl)propyl]oxy}phényl)éthyl]-amino}éthyl)-1(2*H*)-phtalazinone ;
la 4-[(4-chlorophényl)méthyl]-2-{2-[[4-(4-{[3-(hexahydro-1*H*-azépin-1-yl)propyl]oxy}phényl)butyl]-(méthyl)amino]éthyl}-1(2*H*)-phtalazinone;
la 4-[(4-chlorophényl)méthyl]-2-{2-[[2-(4-{[3-(hexahydro-1*H*-azépin-1-yl)propyl]oxy}phényl)éthyl]-(méthyl)amino]éthyl}-1(2*H*)-phtalazinone;
la 4-[(4-chlorophényl)méthyl]-2-({(2*R*)-1-[5-(4-{[3-(hexahydro-1*H*-azépin-1-yl)propyl]oxy}phényl)-pentyl]-2-pyrrolidinyl}méthyl)-1(2*H*)-phtalazinone ;
la 2-({(2*R*)-1-[4-(4-{[3-(hexahydro-1*H*-azépin-1-yl)propyl]oxy}phényl)butyl]-2-pyrrolidinyl}méthyl)-4-{[4-(méthyloxy)phényl]méthyl}-1(2*H*)-phtalazinone;
la 2-({(2*R*)-1-[4-(4-{[3-(hexahydro-1*H*-azépin-1-yl)propyl]oxy}phényl)butyl]-2-pyrrolidinyl}méthyl)-4-[(4-hydroxyphényl)méthyl]-1(2*H*)-phtalazinone ;
la 4-[(4-fluorophényl)méthyl]-2-({(2*R*)-1-[4-(4-{[3-(hexahydro-1*H*-azépin-1-yl)propyl]oxy}phényl)butyl]-2-pyrrolidinyl}méthyl)-1(2*H*)-phtalazinone ;
la 4-[(4-chlorophényl)méthyl]-2-{1-[(4-{[3-(hexahydro-1*H*-azépin-1-yl)propyl]oxy}phényl)méthyl]hexahydro-1*H*-azépin-4-yl}-1(2*H*)-phtalazinone ;
la 4-[(4-chlorophényl)méthyl]-2-{1-[2-(4-{[3-(hexahydro-1*H*-azépin-1-yl)propyl]oxy}phényl)éthyl]hexahydro-1*H*-azépin-4-yl}-1(2*H*)-phtalazinone ;
la 4-[(4-chlorophényl)méthyl]-2-{1-[3-(4-{[3-(hexahydro-1*H*-azépin-1-yl)propyl]oxy}phényl)propyl]-hexahydro-1*H*-azépin-4-yl}-1(2*H*)-phtalazinone ;
la 4-[(4-chlorophényl)méthyl]-2-{1-[3-(4-{[3-(hexahydro-1*H*-azépin-1-yl)propyl]oxy}phényl)propyl]-hexahydro-1*H*-azépin-4-yl}-1(2*H*)-phtalazinone ;
la 4-[(4-chlorophényl)méthyl]-2-[((2*S*)-1-{3-[(3-cyclobutyl-2,3,4,5-tétrahydro-1*H*-3-benzazépin-7-yl)-oxy]propyl}-2-pyrrolidinyl)méthyl]-1(2*H*)-phtalazinone ;
la 4-[(4-chlorophényl)méthyl]-2-[((2*R*)-1-{3-[(3-cyclobutyl-2,3,4,5-tétrahydro-1*H*-3-benzazépin-7-yl)-oxy]propyl}-2-pyrrolidinyl)méthyl]-1(2*H*)-phtalazinone ;
la 4-[(4-chlorophényl)méthyl]-2-(1-{3-[(3-cyclobutyl-2,3,4,5-tétrahydro-1*H*-3-benzazépin-7-yl)oxy]-propyl}hexahydro-1*H*-azépin-4-yl)-1(2*H*)-phtalazinone ;
la 4-[(4-chlorophényl)méthyl]-2-{1-[(3-cyclobutyl-2,3,4,5-tétrahydro-1*H*-3-benzazépin-7-yl)méthyl]hexahydro-1*H*-azépin-4-yl}-1(2*H*)-phtalazinone ;
le *N*-(2-{4-[4-[(4-chlorophényl)méthyl]-1-oxo-2(1*H*)-phtalazinyl]hexahydro-1*H*-azépin-1-yl}éthyl)-3-cyclobutyl-2,3,4,5-tétrahydro-1H-3-benzazépine-7-carboxamide ;
le *N*-(3-{4-[4-[(4-chlorophényl)méthyl]-1-oxo-2(1*H*)-phtalazinyl]hexahydro-1*H*-azépin-1-yl}propyl)-3-cyclobutyl-2,3,4,5-tétrahydro-1*H*-3-benzazépine-7-carboxamide ;
le *N*-(4-{4-[4-[(4-chlorophényl)méthyl]-1-oxo-2(1*H*)-phtalazinyl]hexahydro-1*H*-azépin-1-yl}butyl)-3-cyclobutyl-2,3,4,5-tétrahydro-1*H*-3-benzazépine-7-carboxamide ;
la 4-[(4-chlorophényl)méthyl]-2-[1-(2-{4-[(1-cyclobutyl-4-pipéridinyl)oxy]phényl}éthyl)hexahydro-1*H-*azépin-4-yl]-1(2*H*)-phtalazinone ;
la 4-[(4-chlorophényl)méthyl]-2-[1-(4-{4-[(1-cyclobutyl-4-pipéridinyl)oxy]phényl}butyl)hexahydro-1*H-*azépin-4-yl]-1(2*H*)-phtalazinone ; ou
la 4-[(4-chlorophényl)méthyl]-2-({(2*R*)-1-[4-(4-{[3-(hexahydro-1*H*-azépin-1-yl)propyl]oxy}phényl)butyl]-2-pyrrolidinyl}méthyl)pyrido[3,4-*d*]pyridazin-1-(2*H*)-one ;
ou l'un de leurs sels.

8. Composé qui est : la 4-[(4-chlorophényl)-méthyl]-2-({(2R)-1-[4-(4-{[3-(hexahydro-1*H*-azépin-1-yl)propyl]oxy}phényl)butyl]-2-pyrrolidinyl}méthyl)-1(2*H*)-phtalazinone ou l'un de ses sels.

9. Composé qui est : la 4-[(4-chlorophényl)-méthyl]-2-({(2*S*)-1-[4-(4-{[3-(hexahydro-1*H*-azépin-1-yl)propyl]oxy}phényl)butyl]-2-pyrrolidinyl}méthyl)-1(2*H*)-phtalazinone ou l'un de ses sels.

10. Composé qui est : la 4-[(4-chlorophényl)-méthyl]-2-({(2*R*)-1-[4-(4-{[3-(hexahydro-1*H*-azépin-1-yl)propyl]oxy}phényl)butyl]-2-pyrrolidinyl}méthyl)-1(2*H*)-phtalazinone sous la forme de sa base libre.

11. Composé qui est : la 4-[(4-chlorophényl)-méthyl]-2-({(2*R*)-1-[4-(4-{[3-(hexahydro-1*H*-azépin-1-yl) propyl]oxy}phényl)butyl]-2-pyrrolidinyl}méthyl)-1(2*H*)-phtalazinone, sous la forme d'un sel de 1,5-naphtalène disulfonate.

12. Composé qui est : la 4-[(4-chlorophényl)-méthyl]-2-({(2*R*)-1-[4-(4-{[3-(hexahydro-1*H*-azépin-1-yl) propyl]oxy}phényl)butyl]-2-pyrrolidinyl}méthyl)-1(2*H*)-phtalazinone, sous la forme d'un sel de 1,5-naphtalène disulfonate monohydraté.

13. Composé selon l'une quelconque des revendications 1 à 9, ou l'un de ses sels pharmaceutiquement acceptables.

14. Composé selon l'une quelconque des revendications 1 à 9, ou l'un de ses sels pharmaceutiquement acceptables pour un usage en thérapie.

15. Composé qui est : la 4-[(4-chlorophényl)-méthyl]-2-({(2*R*)-1-[4-(4-{[3-(hexahydro-1*H*-azépin-1-yl) propyl]oxy}phényl)butyl]-2-pyrrolidinyl}méthyl)-1(2*H*) -phtalazinone, ou son sel de 1,5-naphtalène disulfonate monohydraté, pour un usage en thérapie.

16. Composé selon la revendication 14 ou la revendication 15, pour un usage dans le traitement des maladies inflammatoires et/ou allergiques des voies respiratoires.

17. Composé selon la revendication 16, pour un usage dans le traitement d'une rhinite allergique.

18. Composé selon la revendication 16, pour un usage dans le traitement d'une rhinite non allergique.

19. Composition comprenant un composé de formule (I) selon l'une quelconque des revendications 1 à 9, ou l'un de ses sels pharmaceutiquement acceptables, et un ou plusieurs supports et/ou excipients pharmaceutiquement acceptables.

20. Composition comprenant un composé qui est : la 4-[(4-chlorophényl)-méthyl]-2-({(2*R*)-1-[4-(4-{[3-(hexahydro-1*H*-azépin-1-yl)propyl]oxy}phényl)butyl]-2-pyrrolidinyl}méthyl)-1(2*H*)-phtalazinone, ou son sel de 1,5-naphtalène disulfonate monohydraté, et un ou plusieurs supports et/ou excipients pharmaceutiquement acceptables.

21. Composition selon la revendication 19 ou la revendication 20, dans laquelle ladite composition est adaptée pour une administration par voie intranasale.

22. Combinaison comprenant un composé selon l'une quelconque des revendications 1 à 9, ou l'un de ses sels pharmaceutiquement acceptables, et un ou plusieurs autres agents thérapeutiques.

23. Combinaison comprenant un composé qui est : la 4-[(4-chlorophényl)méthyl]-2-({(2*R*)-1-[4-(4-{[3-(hexahydro-1*H*-azépin-1-yl)propyl]oxy}phényl)butyl]-2-pyrrolidinyl}méthyl)-1(2*H*)-phtalazinone, ou son sel de 1,5-naphtalène disulfonate monohydraté, et un ou plusieurs autres agents thérapeutiques.

24. Combinaison selon la revendication 22 ou la revendication 23, dans laquelle l'autre ou les autres agent(s) thérapeutique(s) comprend(comprennent) l'ester de *S*-fluorométhyle de l'acide 6α,9α-difluoro-17α-[(2-furanylcarbonyl)oxy]-11β-hydroxy-16α-méthyl-3-oxo-androsta-1,4-diène-17β-carbothioïque (furoate de fluticasone).

25. Combinaison selon l'une quelconque des revendications 22 à 24, dans laquelle ladite combinaison est adaptée pour une administration par voie intranasale.

26. Utilisation d'un composé selon l'une quelconque de la revendication 1 à 9, ou de l'un de ses sels pharmaceutiquement acceptables, dans la fabrication d'un médicament destiné au traitement des maladies inflammatoires et/ou allergiques des voies respiratoires.

27. Utilisation d'un composé qui est : la 4-[(4-chlorophényl)méthyl]-2-({(2*R*)-1-[4-(4-{[3-(hexahydro-1*H*-azépin-1-yl)propyl]oxy}phényl)butyl]-2-pyrrolidinyl} méthyl)-1(2*H*)-phtalazinone, ou de son sel de 1,5-naphtalène disulfonate monohydraté, dans la fabrication d'un médicament destiné au traitement des maladies inflammatoires et/ou allergiques des voies respiratoires.

28. Utilisation selon la revendication 26 ou la revendication 27, dans laquelle la maladie est une rhinite allergique.

29. Utilisation selon la revendication 26 ou la revendication 27, dans laquelle la maladie est une rhinite non allergique.
